(19) Europäisches Patentamt
European Patent Office
Office européen des brevets

(11) Publication number: **0 550 493 B1**

# (12) EUROPEAN PATENT SPECIFICATION

(45) Date of publication of patent specification: **09.08.95**

(51) Int. Cl.6: **A61K 31/35**, C07D 311/46, C07D 311/54, C07D 405/12, C07D 417/12, C07D 417/14, C07F 9/655

(21) Application number: **91916189.3**

(22) Date of filing: **24.09.91**

(86) International application number: **PCT/US91/06750**

(87) International publication number: **WO 92/06083 (16.04.92 92/09)**

## (54) ANTHELMINTIC AND ANTICOCCIDAL 3-CARBAMOYL-4-HYDROXYCOUMARINS, METHOD OF USE AND COMPOSITIONS.

(30) Priority: **28.09.90 US 589934**

(43) Date of publication of application: **14.07.93 Bulletin 93/28**

(45) Publication of the grant of the patent: **09.08.95 Bulletin 95/32**

(84) Designated Contracting States: **AT BE CH DE DK ES FR GB GR IT LI LU NL SE**

(56) References cited:
**EP-A- 0 038 427** **EP-A- 0 241 834**
**DE-A- 2 643 428** **DE-A- 2 643 476**
**DE-B- 1 214 696** **US-A- 3 991 204**

**CHIMIE THERAPEUTIOUE, vol. 2, no. 6, November 1967, (Paris, FR), L. FONTAINE et al.: "Etude expérimentale des propriétés cholérétiques de coumarines, indane diones et acyl indane diones apparentées aux anticoagulants oraux", pages 430-440, see table 7, compound 41 (cited in the application)**

(73) Proprietor: **THE UPJOHN COMPANY**
**301 Henrietta Street**
**Kalamazoo,**
**Michigan 49001 (US)**

(72) Inventor: **CLOTHIER, Michael, F.**
**5427 East O Avenue**
**Kalamazoo, MI 49001 (US)**
Inventor: **LEE, Byung, Hyun**
**7695 South 12th Street**
**Kalamazoo, MI 49002 (US)**

(74) Representative: **Perry, Robert Edward**
**GILL JENNINGS & EVERY**
**Broadgate House**
**7 Eldon Street**
**London EC2M 7LH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**CHEMICAL ABSTRACTS**, vol. 67, no. 19, 6 November 1967, page 8532, abstract no. 90676k, (Columbus, Ohio, US), & JP,A,42004667 (TANABE SEIYAKU) 25 February 1967, see abstract (cited in the application)

## Description

### SUMMARY OF THE INVENTION

This invention pertains to a new method for killing and controlling worms (Helminths), and new formulations for killing and controlling worms in animals, and new chemical compounds. The invention is more particularly directed to a new method for killing and controlling parasitic worms in animals with novel 3-carbamoyl-4-hydroxycoumarins, and to new anthelmintic formulations comprising the same.

In addition, various 3-carbamoyl-4-hydroxycoumarins demonstrate anticoccidial activity. The anthelmintic 3-carbamoyl-4-hydroxycoumarins have the general structural Formula I. The anticoccidial 3-carbamoyl-4-hydroxycoumarins have the general structural Formula IW.

### BACKGROUND OF THE INVENTION

The diseases or groups of diseases described generally as helminthiasis are due to infection of the animal with parasitic worms known as helminths. Helminthiasis and helminthosis are prevalent and may lead to serious economic problems in valuable domestic warm-blooded animals such as sheep, swine, cattle, goats, dogs, cats, horses, poultry and man. Among the helminths, the groups of worms known as nematodes, trematodes and cestodes cause widespread and often-times serious infections in various species of animals including man. The most common genera of nematodes, trematodes and cestodes infecting the animals referred to above are Dictyocaulus, Haemonchus, Trichostrongylus, Ostertagia, Nematodirus, Cooperia, Bunostomum, Oesophagostomum, Chabertia, Strongyloides, Trichuris, Fasciola, Dicrocoelium, Enterobius, Ascaris, Toxascaris, Toxocara, Ascaridia, Capillaria, Heterakis, Ancylostoma, Uncinaria, Dirofilaria, Onchocerca, Taenia, Moniezia, Dipylidium, Metastrongylus, Triodontophorus, Macracanthorhynchus, Hyostrongylus, and Strongylus. Some of these genera attack primarily the intestinal tract while others inhabit the stomach, lungs, liver and subcutaneous tissues. The parasitic infections causing helminthiasis and helminthosis lead to anemia, malnutrition, weakness, weight loss, unthriftiness, severe damage to the gastrointestinal tract wall and, if left to run their course, may result in death of the infected animals.

The anthelmintic activity of various 3-carbamoyl-4-hydroxycoumarins has been disclosed. However, their action is not always satisfactory, especially when low concentrations are applied.

U.S. Patent 3,991,204 discloses various 3-carbamoyl-4-hydroxycoumarins, including 3',4'-dichlo-4-hydroxy-2-oxo-2H-1-benzopyran-3-carboxanilide, 3'-chloro-4-hydroxy-2-oxo-2H-1-benzopyran-3-carboxanilide, 3'-fluoro-4-hydroxy-2-oxo-2H-1-benzopyran-3-carboxanilide, 4'-fluor-4-hydroxy-2-oxo-2H-1-benzopyran-3-carboxanilide and 2',4'-difluoro-4-hydroxy-2-oxo-2H-1-benzopyran-3-carboxanilide, for controlling helminths.

DE 2643476 and 2643428 disclose various 3-carbamoyl-4-hydroxycoumarins, including 3-N-(4-bromophenyl)-carbamoyl-4-hydroxycoumarin, 3-N-(4-chlorophenyl)-carbamoyl-4-hydroxycoumarin, and 3-N-(4-fluorophenyl)-carbamoyl-4-hydroxycoumarin as pesticides. EP 14372 discloses quatenary aluminum salts of 4-hydroxy-3-(4-trifluoromethylphenyl)-coumarin.

DE 3012642 A1 discloses various 3-carbamoyl-4-hydroxycoumarins, including 3-N-(4-trifluoromethoxy)-carbamoyl-4-hydroxycoumarin and 3-N-(4-chloro-difluoromethoxy)-carbamoyl-4-hydroxycoumarin, as pesticides.

U.S. Patent 4,766,144 discloses various 3-carbamoyl-4-hydroxycoumarins, including 3-N-(4-trifluoromethoxyphenyl)-carbamoyl-4-hydroxycoumarin, 3-N-(3-chloro-4-trifluoromethoxyphenyl)-carbamoyl-4-hydroxycoumarin, 3-N-(3-trifluoromethyl-4-methoxyphenyl)-carbamoyl-4-hydroxycoumarin and 3-N-(3-chloro-4-trifluorothiophenyl)-carbamoyl-4-hydroxycoumarin,for controlling helminths.

U.S. Patent 4,078,075 discloses 3-N-(4-trifluoromethylphenyl)-carbamoyl-4-hydroxycoumarin as useful for combating insect pests.

U.S. Patent 3,511,856 discloses 3-(alkoxyphenylcarbamoyl)-4-hydroxycoumarins useful as bactericides and pesticides.

3-N-(3-chlorophenyl)-8-methyl-carbamoyl-4-hydroxycoumarin and other 3-carbamoyl-4-hydroxycoumarins are disclosed in Pharmazie, 39(2), 86-91 (1984).

3-N-(2-naphthyl)-6-phenyl-carbamoyl-4-hydroxycoumarin, 3-N-(2-naphthyl)-carbamoyl-4-hydroxycoumarin and other 3-carbamoyl-4-hydroxycoumarins are disclosed in Chem. Chron. 1 (3.4), 199-202 (1972).

3-N-(2-hydroxyphenyl)-carbamoyl-4-hydroxycoumarin, 3-N-(4-hydroxyphenyl)-6-halo-carbamoyl-4-hydroxycoumarin and other 3-carbamoyl-4-hydroxycoumarins are disclosed in Chem. Ther. 2(6), 430-440

(1967).

Japanese Patent 4667 (February 25, 1967) describes the preparation of various 3-carbamoyl-4,7-dihydroxycoumarins, including 3-(5-methyl-1,3,4-thiadiazol-2-yl-carbamoyl)-4,7-dihydroxycoumarin, 3-(1,3-thiazole-2-yl-carbamoyl)-4,7-dihydroxycoumarin, 3-(4-chloroanilinocarbamoyl-4,7-dihydroxycoumarin and 3-(3-trifluoromethylanilinocarbamoyl)-4,7-dihydroxycoumarin.

Japanese Patent 4668 (February 25, 1967) describes the preparation of various 3-carbamoyl-4-hydroxy-7-nitrocoumarins and 3-carbamoyl-4-hydroxy-7-acetamidecoumarins, including 3-(5-methyl-1,3,4-thiadiazol-2-yl-carbamoyl)-4-hydroxy-7-nitrocoumarin and 3-(4-trifluoromethylanilinocarbamoyl)-4-hydroxy-7-acetamidecoumarin.

French Patent 1,216,966 discloses various 3-(substituted)-carbamoyl-4-hydroxycoumarins, including 3-(pyridin-3-yl)-carbamoyl-4-hydroxycoumarins and 3-(pyridin-2-yl)-carbamoyl-4-hydroxycoumarins having bactericidal and antifungicidal. See also CA 55:17556f, CA 60:507g, and U.S. Patent 3,122,577.

US-A-3293255 discloses 3-(alkyl-substituted)-carbamoyl-4-hydrocoumarins.

CA 72(19):100433y discloses 4-hydroxy-6-phenyl-3-(phenyl-carbamoyl)-coumarin.

CA 78(13):84191j discloses 4-hydroxy-N-1-naphthyl-2-oxo-6-phenyl-2H-1-benzopyran-3-carboxamide.

## SUMMARY OF THE INVENTION

According to the invention, compounds of formula I, all of which except 3-[N-(4-trifluoromethylphenyl)-carbamoyl]-4'-coumarinare novel, or of formula IW, or a hydrate or pharmaceutically-acceptable salt thereof, are useful for the manufacture of a medicament for use in killing parasitic worms in humans and valuable warm-blooded domestic animals,

wherein X is selected from

(1) 4-halophenyl optionally-substituted at the 2-position by Cl, Br, F or I;

(2) (5-$R'_5$)-1,3,4-thiadiazol-2-yl, wherein $R'_5$ is Cl, Br, F, I, cyano, $-CF_3$, $-CF_2Cl$, $-CF_2CF_3$ or optionally-substituted benzyl, pyridin-2-yl, pyridin-3-yl or pyridin-4-yl and any substituents are one, two or three members selected from Cl, Br, F, I, CN, $CF_3$, $C_1$-$C_5$ alkyl and $C_1$-$C_5$ alkoxy;

(3) (2-$SR''_5$)-1,3,4-thiadiazol-5-yl, wherein $R''_5$ is hydrogen, $C_1$-$C_5$ alkyl, Cl, Br, F, I, cyano, $-CF_3$, $-CF_2Cl$, $-CF_2CF_3$ or optionally-substituted phenyl, benzyl, pyridin-2-yl, pyridin-3-yl, pyridin-4-yl, pyridin-2-yl($CH_2$)-, pyridin-3-yl($CH_2$)- or pyridin-4-yl($CH_2$)- and any substituents are one, two or three members as defined above;

(4) optionally-substituted pyridin-2-yl, pyridin-3-yl, pyridin-4-yl, pyrimidin-5-yl, pyrazin-2-yl, pyrimidin-2-yl or -NH-phenyl and any substituents are one, two or three members as defined above;

(5) 4-[di($C_1$-$C_5$)alkoxyphosphonomethyl]phenyl; or

(6) 4-(pyridin-4-yl-methyl)phenyl optionally-substituted in the pyridinyl moiety with one or two members as defined above;

R is hydrogen, phenyl, Cl, Br, F, CN, $CF_3$, $C_1$-$C_5$ alkyl or $C_1$-$C_5$ alkoxy;

$R'_2$ is hydrogen, $CF_3$, Cl, Br, F or I;

$R'_4$ is Br, F, Cl or $CF_3$; and

R' is Br, F or $CF_3$;

with the provisos that, when X is 4-halophenyl, R is Cl, F, Br, $CF_3$ or phenyl, that the compound of formula IW is not 3-[N-(4-chlorophenyl)carbamoyl]-7-trifluoromethyl-4-hydroxycoumarin, and that the compound of formula I is other than:

3-[N-(5-benzyl-1,3,4-thiadiazol-2-yl)carbamoyl]-7-chloro-4-hydroxycoumarin,
7-chloro-4-hydroxy-3-[N-[5-(2-pyridinylmethyl)thio-1,3,4-thiadiazol-5-yl]carbamoylcoumarin,
7-chloro-4-hydroxy-3-[N-(5-phenyl-1,3,4-thiadiazol-2-yl]-carbamoylcoumarin,
3-[N-(2-benzylthio-1,3,4-thiadiazol-5-yl)carbamoyl]-7-chloro-4-hydroxycoumarin,
4-hydroxy-3-[N-(2-pyridinylcarbamoyl)]coumarin,
4-hydroxy-3-[N-(3-pyridinylcarbamoyl)]coumarin,
7-triflouromethyl-3-[N-(3-chloro-5-trifluoromethylpyridin-2-yl)carbamoyl]-4-hydroxycoumarin.
3-[N-(6-chloropyridin-3-yl)carbamoyl]-4-hydroxycoumarin,
3-[N-(6-methylpyridin-2-yl)carbamoyl]-4-hydroxycoumarin,
3-[N-(5-bromophyridin-2-yl)carbamoyl]-7-methoxy-4-hydroxycoumarin, or
3-[N-(2,6-chloropyridin-4-yl)carbamoyl]-4-hydroxycoumarin.

Halogen refers to a bromo, chloro, iodo or fluoro atom.

Halo refers to a chloro, bromo or fluoro atom.

4-Halophenyl (Formula A) refers to 4-chorophenyl, 4-fluorophenyl and 4-bromophenyl, preferably 4-bromophenyl.

R is preferably selected from the group consisting of halo (Cl, Br, or F) or $CF_3$.

Examples of 4-[di($C_1$-$C_5$)alkoxyphosphonomethyl]phenyl include 4-(diethoxyphosphonomethyl)phenyl, 4-(dimethoxyphosphonomethyl)phenyl and 4-(ethoxymethoxyphosphonomethyl)phenyl.

Examples of $C_1$-$C_5$ alkyl are methyl, ethyl, propyl, butyl, pentyl and isomeric forms thereof. Examples of $C_1$-$C_5$ alkoxy are methoxy, ethoxy, propoxy, butoxy, pentoxy and isomeric forms thereof.

Pharmaceutically acceptable refers to those properties and/or substances which are acceptable to the patient from a pharmacologically-toxicological point of view and to the manufacturing pharmaceutical chemist from a physical-chemical point of view regarding composition, formulation, stability, patient acceptance and bioavailability.

C__-C__ means the carbon content of various hydrocarbon-containing moieties is indicated by a prefix designating the minimum and maximum number of carbon atoms in the moiety. Thus ($C_1$-$C_3$) alkyl refers to alkyl of one to 3 carbon atoms, inclusive or methyl, ethyl, propyl, and isopropyl.

Preferred 3-carbamoyl-4-hydroxycoumarins of Formula I are where X is 4-halophenyl, i.e. a 3-N-(4-halophenyl)-carbamoyl-4-hydroxycoumarin (Formula IA) or 1,3,4-thiadiazol-2-yl, i.e. a 3-N-(1,3,4-thiadiazol-2-yl)-carbamoyl-4-hydroxycoumarin(Formula IB)substituted at the 5-position with a halogen (Cl, Br, F, or I) atom, cyano (-CN), $CF_3$, $-CF_2Cl$, $-CF_2CF_3$, -CN, $C_1$-$C_5$ alkyl, benzyl optionally substituted with [one member selected from the group consisting of halogen, -CN or trifluoromethyl], or pyridinyl optionally substituted with [one member selected from the group consisting of halogen, -CN or trifluoromethyl].

More preferred 3-carbamoyl-4-hydroxycoumarins of Formula I are where X is 1,3,4-thiadiazol-2-yl, i.e. a 3-N-(1,3,4-thiadiazol-2-yl)-carbamoyl-4-hydroxycoumarin (Formula IB) substituted at the 5-position with a halogen (Cl, Br, F, or I) atom, cyano, $-CF_3$, $-CF_2Cl$ or $-CF_2CF_3$.

Particularly preferred anthelmintic 3-carbamoyl-4-hydroxycoumarins of Formula I are: 7-chloro-4-hydroxy-3-[N-[5-(trifluoromethyl)-1,3,4-thiadiazol-2-yl]carbamoyl]coumarin (Cpd. #11), 7-chloro-4-hydroxy-3-[N-[5-(chlorodifluoromethyl)-1,3,4-thiadiazol-2-yl]carbamoyl]coumarin (Cpd. #17), 7-chloro-4-hydroxy-3-[N-[5-(trifluoromethyl-difluoromethyl)-1,3,4-thiadizol-2-yl]carbamoyl]coumarin (Cpd. #18), 3-[N-(4-chlorophenyl)-carbamoyl]-7-fluoro-4-hydroxycoumarin (Cpd. #6), 3-[N-(4-fluorophenyl)carbamoyl]-7-trifluoromethyl-4-hydroxycoumarin (Cpd. #8).

Particularly preferred anticoccidial 3-carbamoyl-4-hydroxycoumarins of Formula IW are: 3-[N-2,4-difluorophenyl)carbamoyl]-7-bromo-4-hydroxycoumarin (Cpd. #53) and 3[N-(4-chlorophenyl)carbamoyl]-7-bromo-4-hydroxycoumarin (Cpd. #54).

A is 4-halophenyl (preferably 4-bromophenyl) optionally subsituted at the 2 position with halogen (Cl, Br, F or I, preferably F).

B is 1,3,4-thiadizol-2-yl substituted at the 5-position with halogen (Cl, Br, F, or I), cyano, $-CF_3$, $-CF_2Cl$, $-CF_2CF_3$, benzyl, benzyl substituted with [one, two or three members selected from the group consisting of halogen (Cl, Br, F or I), CN, $CF_3$, $C_1$-$C_5$ alkyl or $C_1$-$C_5$ alkoxy], pyridin-2-yl, pyridin-2-yl substituted with [one, two or three members selected from the group consisting of halogen (Cl, Br, F or I), CN, $CF_3$, $C_1$-$C_5$ alkyl or $C_1$-$C_5$ alkoxy], pyridin-3-yl, pyridin-3-yl substituted with [one, two or three members selected from the group consisting of halogen (Cl, Br, F or I), CN, $CF_3$, $C_1$-$C_5$ alkyl or $C_1$-$C_5$ alkoxy], pyridin-4-yl, pyridin-4-yl substituted with [one, two or three members selected from the group consisting of halogen (Cl, Br, F or I), CN, $CF_3$, $C_1$-$C_5$ alkyl or $C_1$-$C_5$ alkoxy].

C is 5-(SR''$_5$)-1,3,4-thiadizol-2-yl wherein R''$_5$ is selected from hydrogen, halogen (Cl, Br, F, or I), cyano, $-CF_3$, $-CF_2Cl$, $-CF_2CF_3$, phenyl, phenyl substituted with [one, two or three members selected from the group consisting of halogen (Cl, Br, F or I), CN, $CF_3$, $C_1$-$C_5$ alkyl or $C_1$-$C_5$ alkoxy], benzyl, benzyl substituted with [one, two or three members selected from the group consisting of halogen (Cl, Br, F or I), CN, $CF_3$, $C_1$-$C_5$ alkyl or $C_1$-$C_5$ alkoxy], pyridin-2-yl, pyridin-2-yl substituted with [one, two or three members selected from the group consisting of halogen (Cl, Br, F or I), CN, $CF_3$, $C_1$-$C_5$ alkyl or $C_1$-$C_5$ alkoxy], pyridin-3-yl, pyridin-3-yl substituted with [one, two or three members selected from the group consisting of halogen (Cl, Br, F or I), CN, $CF_3$, $C_1$-$C_5$ alkyl or $C_1$-$C_5$ alkoxy], pyridin-4-yl, pyridin-4-yl substituted with [one, two or three members selected from the group consisting of halogen (Cl, Br, F or I), CN, $CF_3$, $C_1$-$C_5$ alkyl or $C_1$-$C_5$ alkoxy].

F is pyrimidin-5-yl optionally substituted with one, two or three members selected from the group consisting of halogen (Cl, Br, F or I), CN, $CF_3$, $C_1$-$C_5$ alkyl or $C_1$-$C_5$ alkoxy.

G is pyrazin-2-yl optionally substituted with one, two or three members selected from the group consisting of halogen (Cl, Br, F or I), CN, $CF_3$, $C_1$-$C_5$ alkyl or $C_1$-$C_5$ alkoxy.

H is pyrimidin-2-yl optionally substituted with one, two or three members selected from the group consisting of halogen (Cl, Br, F or I), CN, $CF_3$, $C_1$-$C_5$ alkyl or $C_1$-$C_5$ alkoxy.

I is -NH-phenyl optionally substituted with one, two or three members selected from the group consisting of halogen (Cl, Br, F or I), CN, $CF_3$, $C_1$-$C_5$ alkyl or $C_1$-$C_5$ alkoxy.

J is 4-[di($C_1$-$C_5$)alkoxyphosphonomethyl]phenyl.

K is 4-(pyridin-4-ylmethyl)phenyl wherein the pyridinyl moiety is optionally substituted with $C_1$-$C_5$ alkyl.

X is pyridin-2-yl optionally substituted with one, two or three members selected from the group consisting of halogen (Cl, Br, F or I), CN, $CF_3$, $C_1$-$C_5$ alkyl or $C_1$-$C_5$ alkoxy.

Y is pyridin-2-yl optionally substituted with one, two or three members selected from the group consisting of halogen (Cl, Br, F or I), CN, $CF_3$, $C_1$-$C_5$ alkyl or $C_1$-$C_5$ alkoxy.

Z is pyridin-2-yl optionally substituted with one, two or three members selected from the group consisting of halogen (Cl, Br, F or I), CN, $CF_3$, $C_1$-$C_5$ alkyl or $C_1$-$C_5$ alkoxy.

The 3-carbamoyl-4-hydroxycoumarins of the invention (Formula I) are readily prepared by refluxing the appropriate 4-hydroxycoumarin esters (1) with the appropriate arylamines (2) (CHART A. Scheme A) or by reacting the appropriate 4-hydroxycoumarins (4) with the appropriate arylisocyanates (5) (CHART A, Scheme B).

The reaction of Scheme A is carried out in a nitrogen atmosphere and refluxed in the presence of a suitable solvent or solvent mixture in order to completely dissolve the starting materials; for example, xylene, xylene/DMF, DMSO or DMF. After the reaction is complete, the mixture is cooled to room temperature to precipitate the desired product. The precipitate is filtered and washed with a suitable solvent(s) such as methanol, xylene, ether, ethyl acetate or chloroform to give the product in a yield of about 30-90%. The product can be recrystallized from DMF, DMSO or preferably dioxane.

The acylation reaction of Scheme B is carried out in the presence of a suitable base such as a tertiary amine, for example, N-methyl morpholine, DBU (1,8-diazabicyclo[5.4.0]undec-7-ene), DBN (1,5-diazabicyclo[4.3.0]non-5-ene), DABCO (1,4-diazabicyclo[2.2.2)octane), pyridine, or triethylamine.

Preferably, the coumarin esters (1) are obtained from the corresponding salicylates as outlined in CHART B. Acetylation of the salicylic acid 6 followed by treatment of the resultant acetate 7 with refluxing thionyl chloride provides the acid chloride 8. Addition of the acid chloride 8 to a solution of sodium methoxide and diethyl malonate in toluene, followed by heating at reflux provides the desired coumarin ester 1. Improved yields are obtained by the addition of the acid chloride 8 to the malonate anion (1.2 eq) generated with sodium hydride, followed by cyclization of the resultant acylated malonate 9 with sodium methoxide in refluxing tetrahydrofuran (THF).

The starting amines (2) and isocyanates (5) are commercially available or prepared by known methods.

The following detailed examples/procedures describe how to prepare various 3-carbamoyl-4-hydroxycoumarins of the invention and are to be construed as merely illustrative, and not limitations of the preceding disclosure in any way whatsoever. Those skilled in the art will promptly recognize appropriate variations from the procedures both as to reactants as well as to reaction conditions and techniques.

Example 1: Preparation of 3-[N-(4-bromophenyl)carbamoyl]-7-chloro-4-hydroxycoumarin, Compound No. 1.

A mixture of 3-ethoxycarbonyl-4-hydroxy-7-chlorocoumarin (8.0 g) and 4-bromoaniline (5.1 g) is heated to reflux in xylene (60 ml) and dimethyl formamide (5 ml) for one hour. Upon cooling, the desired product crystallites. The crude product is filtered, washed with diethyl ether, and dried to give 10.4 gm. of the title compound having a melting point of 228.4° C.

Following the general procedure of Example 1, except using the appropriate coumarin esters (1) and amines (2) and making non-critical variations, the following compounds are prepared.

| Example No./Compound No. | Chemical Name | Melting Point °C |
|---|---|---|
| 2 | 3-[N-(4-bromophenyl)carbamoyl]-7-trifluoromethyl-4-hydroxycoumarin | 234 |
| 3 | 3-[N-(4-bromophenyl)carbamoyl]-7-fluoro-4-hydroxycoumarin | 212.5 |
| 4 | 3-[N-(4-chlorophenyl)carbamoyl]-7-chloro-4-hydroxycoumarin | 213.5 |
| 5 | 3-[N-(4-chlorophenyl)carbamoyl]-7-trifluoromethyl-4-hydroxycoumarin | 219.9 |
| 6 | 3-[N-(4-chlorophenyl)carbamoyl]-7-fluoro-4-hydroxycoumarin | 212.2 |
| 7 | 3-[N-(4-fluorophenyl)carbamoyl]-7-chloro-4-hydroxycoumarin | 212.8 |
| 8 | 3-[N-(4-fluorophenyl)carbamoyl]-7-trifluoromethyl-4-hydroxycoumarin | 194.1 |
| 9 | 3-[N-(4-fluorophenyl)carbamoyl]-7-fluoro-4-hydroxycoumarin | 235.6 |

Example 10: Preparation of 3-[N-(4-bromophenyl)carbamoyl]-7-fluoro-4-hydroxycoumarin, triethylamine salt.

A mixture of 7-fluoro-4-hydroxycoumarin (180 mg), p-bromophenyl isocyanate (198 mg), and triethylamine (101 mg) is heated to reflux in tetrahydrofuran (THF, 6 ml) for one hour. After the reaction is complete, the solvent is evaporated and the resulting solid is triturated with methanol to give 200 mg of the title compound having a melting point of 187-189° C.

Example 11: Preparation of 7-chloro-4-hydroxy-3-[N-[5-(trifluoromethyl)-1,3,4-thiadiazol-2-yl]carbamoyl]-coumarin, Compound No. 11.

A mixture of 3-ethoxycarbonyl-4-hydroxy-7-chlorocoumarin (6.0 gm) and 2-amino-5-trifluromethyl-1,3,4-thiadiazole (3.7 gm) is heated to reflux in xylene (170 ml) for one hour. After 70 ml of the xylene has distilled off over about 2 hours, the resulting mixture is heated to reflux in xylene for 17 hours. Upon cooling, the desired product crystallizes. The solid is washed with 20 ml of acetonitrile and dried *in vacuo* to give 8 gm of the title compound having a melting point of 246° C.

Following the general procedure of Example 11, except using the appropriate coumarin esters and amines and making non-critical variations, the following compounds are prepared.

| Example No./ Compound No. | Chemical Name | Melting Point °C |
|---|---|---|
| 12 | 4-hydroxy-3-[N-[5-(trifluoromethyl)-1,3,4-thiadiazol-2-yl]carbamoyl]coumarin | 249.3 |
| 13 | 4-hydroxy-7-methyl-3-[N-5-(trifluoromethyl)-1,3,4-thiadiazol-2-yl]carbamoyl]coumarin | 273.9 |
| 14 | 4-hydroxy-7-methoxy-3-[N-[5-(trifluoromethyl)-1,3,4-thiadiazol-2-yl]carbamoyl]coumarin | 258-259.5 |
| 15 | 7-fluoro-4-hydroxy-3-[N-[5-(trifluoromethyl)-1,3,4-thiadiazol-2-yl]carbamoyl]coumarin | 240.5 |
| 16 | 7-trifluoromethyl-4-hydroxy-3-[N-[5-(trifluoromethyl)-1,3,4-thiadiazol-2-yl]carbamoyl]coumarin | 264 (dec) |
| 17 | 7-chloro-4-hydroxy-3-[N-[5-(chlorodifluoromethyl)-1,3,4-thiadiazol-2-yl]carbamoyl]coumarin | 234-238 |
| 18 | 7-chloro-4-hydroxy-3-[N-[5-(trifluoromethyl-difluoromethyl)-1,3,4-thiadiazol-2-yl]carbamoyl]coumarin | 214-218 |
| 19 | 4-hydroxy-3-[N-[5-(trifluoromethyldifluoromethyl)-1,3,4-thiadiazol-2-yl]carbamoyl]coumarin | 225-227 |
| 20 | 7-fluoro-4-hydroxy-3-[N-[5-(trifluoromethyl-difluoromethyl)-1,3,4-thiadiazol-2-yl]carbamoyl]coumarin | 205-207 |

| | | |
|---|---|---|
| 21 | 4-hydroxy-3-[N-(5-phenyl-1,3,4-thiadiazol-2-yl)-carbamoyl]coumarin | 227.2 |
| 23 | 3-[N-(5-benzyl-1,3,4-thiadiazol-2-yl)carbamoyl]-4-hydroxycoumarin | 241.7 |
| 24 | 3-[N-(5-benzyl-1,3,4-thiadiazol-2-yl)carbamoyl]-4-hydroxy-7-methylcoumarin | 275.1 (dec) |
| 25 | 3-[N-[5-(4-chlorophenyl)methyl-1,3,4-thiadiazol-2-yl]carbamoyl]-4-hydroxycoumarin | 226.5-230.5 |
| 26 | 7-chloro-3-[N-[5-(4-chlorophenyl)methyl-1,3,4-thiadiazol-2-yl]carbamoyl]-4-hydroxycoumarin | 221-223 |
| 27 | 3-[N-[2-[(4-fluorophenyl)methyl]thio-1,3,4-thiadiazol-5-yl]carbamoyl]-4-hydroxycoumarin | 224.0-226.5 (dec) |
| 28 | 4-hydroxy-3-[N-(2-mercapto-1,3,4-thiadiazol-5-yl)-carbamoyl]coumarin | 304-306 (dec) |
| 29 | 4-hydroxy-3-[N-[2-(methylthio)-1,3,4-thiadiazol-5-yl]-carbamoyl]coumarin | 239.3 |
| 30 | 4-hydroxy-3-[N-[5-(3,5-dichloropyridin-2-yl)thio-1,3,4-thiadiazol-2-yl]carbamoyl]coumarin | 233-239 |
| 31 | 4-hydroxy-3-[N-[2-(2-pyridinylmethyl)thio-1,3,4-thiadiazol-5-yl]carbamoyl]coumarin | 217.4 |
| 32 | 4-hydroxy-7-methyl-3-[N-[2-(2-pyridinylmethyl)thio-1,3,4-thiadiazol-5-yl]carbamoyl]coumarin | 237-239 (dec) |
| 33 | 3-N-[2-(4-pyridinylmethyl)thio-1,3,4-thiadiazol-5-yl]-carbamoyl-4-hydroxycoumarin | 226-228.5 |
| 34 | 3-[N-[2-(benzylthio)-1,3,4-thiadiazol-5-yl]carbamoyl]-4-hydroxycoumarin | 215.1 |
| 35 | 3-[N-(5-bromopyrimidin-2-yl)carbamoyl]4-hydroxy-coumarin | 266.5 |
| 36 | 4-hydroxy-3-[N-(phenylamino)carbamoyl]coumarin | 215.6 |
| 37 | 4-hydroxy-3-[N-[(4-bromophenyl)amino]carbamoyl]-coumarin | 237.2 |
| 38 | 4-hydroxy-3-[N-(2-bromo-1,3,4-thiadiazol-5-yl)-carbamoyl]coumarin | 252.5 (dec) |

| | | |
|---|---|---|
| 39 | 4-hydroxy-3-[N-(2-chloro-1,3,4-thiadiazol-5-yl)-carbamoyl]coumarin | 245.8 (dec) |
| 40 | 3-[N-(4-chloro-6-methylpyrimidin-2-yl)carbamoyl]4-hydroxycoumarin | 207.7 |
| 41 | 3-[N-(6-chloropyrazin-2-yl)carbamoyl]-4-hydroxycoumarin | 260.4 |
| 42 | 3-[N-(4,6-dichloropyrimidin-5-yl)carbamoyl]-4-hydroxy-coumarin | 201.1 |
| 43 | 3-[N-(3,6-dichloropyridazin-4-yl)carbamoyl]-4-hydroxy-coumarin | 214.5 |
| 44 | 4-hydroxy-3-[N-[5-(2-fluorophenyl)-1,3,4-thiadiazol-2-yl]carbamoyl]coumarin | 250.8 |
| 45 | 7-phenyl-4-hydroxy-3-[N-[5-(trifluoromethyl)-1,3,4-thiadiazol-2-yl]caramoyl]coumarin | 214.5 (dec) |
| 46 | 3-[N-(4-bromophenyl)carbamoyl]-7-phenyl-4-hydroxy-coumarin | 218.9 |
| 47 | 3-[N-(5-bromopyridin-2-yl)carbamoyl]-4-hydroxycoumarin | 228.1 |
| 48 | 3-[N-(2-pyridinyl)carbamoyl]-4-hydroxycoumarin | 236.2 |
| 49 | 3-[N-(5-chloropyridin-2-yl)carbamoyl]-4-hydroxycoumarin | 242.8 |
| 50 | 3-[N-(2-chloropyridin-3-yl)carbamoyl]-4-hydroxycoumarin | 222.1 |
| 51 | 3-[N-(5-methylpyridin-2-yl)carbamoyl]-4-hydroxycoumarin | 246.5 |
| 52 | 3-[N-(6-methoxypyridin-3-yl)carbamoyl]-4-hydroxy-coumarin | 195.2 |
| 53 | 7-trifluoromethyl-3-[N-[4-(2,4-difluoro)phenyl]]-4-hydroxycoumarin | 202.4 |
| 54 | 7-bromo-3-[N-[4-chlorophenyl)carbamoyl]-4-hydroxy-coumarin | 233.6 |
| 55 | 3-[N-(3,5-dichloropyridin-2-yl)carbamoyl]-4-hydroxy-coumarin | 242.1 |
| 56 | 3-[N-(5-bromopyridin-2-yl)carbamoyl]-7-chloro-4-hydroxycoumarin | 240.9 |
| 58 | 7-bromo-3-[N-[4-(2,4-difluoro)phenyl]carbamoyl]-4-hydroxycoumarin | 223.2 (dec) |

| | | |
|---|---|---|
| 60 | 3-[N-[5-(1,1-dimethylethyl)-1,3,4-thiadiazol-2-yl)carbamoyl]-4-hydroxycoumarin | 241.2 |
| 61 | 3-[N-(5-bromopyridin-2-yl)carbamoyl]-7-methyl-4-hydroxycoumarin | 240.8 |
| 62 | 7-bromo-3-[N-(4-fluorophenyl)carbamoyl]-4-hydroxy-coumarin | 218.1 |
| 63 | 3-[N-(2,3,5,6-tetrafluoropyridin-4-yl)carbamoyl]-6-methoxy-4-hydroxycoumarin | 186.9 |
| 64 | 7-chloro-3-[N-(5-chloropyridin-2-yl)carbamoyl]-4-hydroxycoumarin | 241.5 |
| 65 | 7-chloro-3-[N-(5-chloropyridin-2-yl)carbamoyl]-4-hydroxycoumarin, triethylamine salt | 210.9 |
| 67 | 7-fluoro-3-[N-(5-bromopyridin-2-yl)carbamoyl]-4-hydroxy-coumarin | 205.4 |
| 68 | 7-chloro-3-[N-(3-chloro-5-trifluoromethylpyridin-2-yl)-carbamoyl]-4-hydroxycoumarin | 252-254 |
| 69 | 3-[N-(3-chloro-5-trifluoromethylpyridin-2-yl)carbamoyl]-4-hydroxycoumarin | 221.6 |
| 70 | 3-[N-(3,5-ditrifluoromethylpyridin-2-yl)carbamoyl]-4-hydroxycoumarin | 173-174 |
| 71 | 7-trifluoromethyl-3-[N-(5-bromopyridin-2-yl)carbamoyl]-4-hydroxycoumarin | 236.6 |
| 72 | 7-trifluoromethyl-3-[N-(5-chloropyridin-2-yl)carbamoyl]-4-hydroxycoumarin | 228.6 |
| 73 | 7-trifluoromethyl-3-[N-(2-chloropyridin-3-yl)carbamoyl]-4-hydroxycoumarin | 231.5 |
| 74 | 7-trifluoromethyl-3-[N-(6-chloropyridin-3-yl)carbamoyl]-4-hydroxycoumarin | 269.3 |
| 75 | 7-chloro-3-[N-(6-methoxypyridin-3-yl)carbamoyl]-4-hydroxycoumarin | 237.0 |
| 76 | 7-trifluoromethyl-3-[N-(6-methoxypyridin-3-yl)carbamoyl]-4-hydroxycoumarin | 263.4 (dec) |

| | | |
|---|---|---|
| 77 | 7-chloro-3-[N-(2-chloropyridin-3-yl)carbamoyl]-4-hydroxy-coumarin | 255.7 |
| 78 | 7-chloro-3-[N-(6-chloropyridin-3-yl)carbamoyl]-4-hydroxy-coumarin | 269.8 |
| 79 | 7-chloro-3-[N-(3,5-ditrifluoromethylpyridin-2-yl)carbamoyl]-4-hydroxycoumarin | 206-209 |
| 80 | 7-trifluoromethyl-3-[N-2,3,5,6-tetrafluoropyridin-4-yl)-carbamoyl]-4-hydroxycoumarin | 166.8 |
| 81 | 7-chloro-4-hydroxy-3-[N-[5-(1,1,2,2,3,3,3-heptafluoropropyl)-1,2,4-thiadiazol-2-yl]carbamoyl]coumarin | 218.7 |
| 91 | 7-bromo-3-[N-[4-bromophenyl)carbamoyl]-4-hydroxy-coumarin | 240.5 |

The 3-carbamoyl-4-hydroxycoumarins of this invention (Formula I) are effective against parasitic worms, particularly those of valuable domestic warm-blooded animals and more particularly helminth parasites in ovines (sheep) and bovines (cattle).

Although a significant number of the 3-carbamoyl-4-hydroxycoumarins have failed to demonstrate significant activity against Caenorhabditis elegans in vitro, observations in Jirds, Meriones unguiculatus, infected with Haemonchus contortus in accordance with the procedure described in J. Parasitol. 76(2), 1990, p. 168-170, generally confirm anthelmintic activity as set forth in Table I.

In addition, various 3-carbamoyl-4-hydroxycoumarins have been tested for in vivo activity against the hookworm, Nematospiroides dubius, of mice in accordance with Procedure I and the results set forth in Table II.

Further observations in sheep experimentally infected with Haemonchus contortus in accordance with Procedure II, confirm anthelmintic activity as set forth in Table III.

Procedure I

Mice are infected with N. dubius. Each mouse is given ~ 100 infective larvae of N. dubius per os. Two weeks later mice from each community cage are sacrificed and examined for the presence of hookworms. Following sampling, the remaining animals are randomly allotted to treated groups of five mice/group. Throughout the experiments mice receive food and water ad libitum.

Method A (Oral Administration)

Test compounds are evaluated orally in five mouse groups at the following dosage:

| Dose mg/mse/day | Number Doses | mg/kg Mouse |
|---|---|---|
| 7.5 | 4 | 1500 |
| 2.75 | 4 | 550 |
| 1.0 | 4 | 200 |
| 0.36 | 4 | 72 |
| 0.13 | 4 | 26.4 |

Oral treatments are administered with a 1 cc tuberculin syringe fitted with a cannula. Each mouse receives 1/4 of the test dose/day on four consecutive days in 0.1 ml of vehicle (40% glycerol formal, 5% polyvinylpyrrolidone C-15 in propylene glycol). In each separate experiment ten mice are used as non-treated controls.

Method B (Drug in Feed)

The feed containing test compound is weighed and randomly allotted to the cages that will house each group of mice for the duration of the trial (6 days). The food is contained in a controlled access feeder. Food and water are available to the mice ad libitum.

The following doses are used for test compound:

| % Drug in Diet | mg Drug/120 g Food | Estimated Dose mg/Mouse | Estimated Dose mg/kg- Mouse |
|---|---|---|---|
| 0.15 | 180 | 30 | 1500 |
| 0.10 | 120 | 20 | 1000 |
| 0.075 | 90 | 15 | 750 |
| 0.05 | 60 | 10 | 500 |
| 0.0375 | 45 | 7.5 | 375 |
| 0.025 | 30 | 5 | 250 |

All mice in each trial are sacrificed three days after the last treatment and examined for N. dubius. The clearance rate is calculated according to the following formula:

$$\text{Clearance rate} = \frac{\text{number of mice cleared}}{\text{number of mice treated}} \times 100$$

Procedure II

In individual experiments all sheep are treated identically, however non-critical variations occur between experiments. All of the sheep used in this procedure are treated twice with levamisole hydrochloride orally at 8 mg/kg or once each with ivermectin parenterally at 200 µg/kg and levamisole hydrochloride orally at 8 mg/kg. The second treatment in each case is administered 4-7 days after the first treatment. Two weeks after the second treatment all sheep are inoculated per os with —3,500 to —7,500 infective larvae of H. contortus. Rectal fecal samples are taken from each sheep 26-41 days post-inoculation (PI), and these samples are examined for eggs of H. contortus using the McMaster counting chamber technique. All sheep harboring good infections of H. contortus are randomly allocated to a treatment group; those which do not exhibit suitable infections are dropped from the study. One-three days later on days 27-42 PI each sheep remaining in the study (excluding the non-treated controls) is treated with a test compound (orally at 12.5 mg/kg unless indicated otherwise) or a standard (levamisole hydrochloride orally at 8 mg/kg) or is used as an untreated control. All sheep receive food and water ad lib. throughout the experiment.

Prior to administration, all solid compounds are finely ground using a mortar and pestle. The compounds are suspended in 20-30 ml of sterile vehicle #98 (each ml contains: carboxymethylcellulose - 10 mg, polysorbate 80-4 mg, propylparaben - 0.42 mg) using a sonicator and administered along with a tap water wash via a stomach tube. All test compounds are given to a single sheep/route of administration. Two or more sheep are treated with levamisole hydrochloride and five are used as nontreated controls. All animals are monitored for signs of toxicity following treatment.

The sheep are sacrificed 7-12 days after treatment (days 35-49 PI), and the abomasum is ligated and removed from each sheep. Each abomasum is longitudinally sectioned and rinsed into an 80 mesh sieve. Sieve contents are collected in individual containers and fixed in formol-alcohol. Later each sample is transferred to a 1000 or 2000 ml beaker and the volume brought to 400-1000 ml with tap water. The total number of worms in a 40-100 ml aliquot (10%) is determined. When no worms are found in the 10% aliquot, the entire sample is examined. Total worm number/sheep and percentage clearance for each treatment are calculated. Percentage clearance for a particular test compound in a given trial is determined according to the following formula:

Percentage Clearance (Test Compound) = [(Mean number of worms recovered from nontreated control sheep - Number of worms recovered from treated sheep)/Mean number of worms recovered from nontreated control sheep] x 100.

Sheep which die within 24 hr following treatment are not examined for worms, while any that die between 24 hr post-treatment and necropsy are examined in an identical manner as that described above. The results of various trials are combined and reported in Table III as percentage clearance.

In addition, various 3-carbamoyl-4-hydroxycoumarins have been tested for *in vivo* anticoccidial activity against the coccidian parasite, *Eimeria tenella*, in chickens in accordance with Procedure III and the results set forth in Table IV.

Procedure III

Procedures for Handling Birds:

One-day-old Leghorn or Leghorn-Broiler cross cockerals are placed in Petersime brood-units (25 birds per section) where they are reared under continuous light and at approximately 75 °F for two weeks. Feed (diet P-607) and water are available *ad libitum*.

When birds are two weeks old, the number required for a trial are moved to the screening rooms which have continuous light and are maintained at 75 °F. A separate feed supply is available in each cage; water is available behind each row of cages. Birds are checked daily and the floors are washed. When a trial is terminated, cages and waterers are washed with a high-pressure sprayer.

Preparation of Test Samples:

Test compounds are initially evaluated at 400-500 ppm technical material in the feed. To prepare a sample for testing, a small amount of test sample is mixed in a few gm of fine feed (60 mesh screen) and then ground with a mortar and pestle to make a premix. This sample is mixed with additional feed on a batch mixer.

Coban (Eli Lilly) containing 45 gm monensin as monensin sodium per lb is the currently used standard. It is included in the feed at a technical drug concentration of 121 ppm.

Test Procedures:

Each experimental unit consists of three birds in a single cage. A completely random experimental design with one replicate is used. There are four groups of birds. Group 1 is an environmental control; these birds receive non-medicated feed (diet P-607) and are not infected. Group 2 is a positive control; these birds are artificially infected with sporulated oocysts and are fed a diet containing a commercial anticoccidial. Group 3 is an infected control; these birds are infected with sporulated oocysts and receive non-medicated fee. Group 4 represents test birds; these birds are infected with sporulated oocysts and provided feed containing test samples.

Birds are weighed and placed in cages and provided the appropriately medicated feed on Monday morning. Forty-eight hours later, birds are intubated with the inoculum containing sporulated oocysts of mixed populations of *Eimeria acervulina, E. maxima, E. tenella.*

Evaluation Variables:

Birds are observed throughout the test period (8 days) for mortalities, and on Day 8 scored for blood in the droppings. The scale for dropping scores is as follows: 0 = normal, 1 = few loose droppings, 2 = loose droppings with blood, and 3 = many bloody droppings. On Day 8 of the test period (following Tuesday), birds are sacrified by cervical dislocation, weighed and the intestines scored for hemorrhagic lesions (upper intestine: *E. acervulina*, middle intestine: *E. maxima*, cecum: *E. tenella*) by a modified method of Johnson and Reid (1970). The modified scale for lesion scores is as follows: 0 = no lesions, 1 = few lesions, 2 = moderate lesions and 3 = severe lesions. Birds that die during the experiment ware weighed and scored for lesions at or near the time of death. A compound is considered active against a particular species of coccidian if the lesion scores for that species is ≦2.0.

Diet:

| DIET P-607 FOR BIRDS | |
|---|---|
| Ingredients | Percent |
| Corn, coarse ground | 70.5 |
| Soybean Oil Meal, 48.5 | 25.0 |
| Yellow Tallow, stabilized | 1.0 |
| Limestone | 0.7 |
| Dicalcium Phosphate (18.5%) | 2.0 |
| Salt | 0.4 |
| DL-methionione (98%) | 0.1 |
| Vitamin D, 15,000 ICU/gm | 0.006 |
| Vitamin A, 30,000 IU/gm | 0.005 |
| Dawes 3-6-9-90 (B-vits) | 0.05 |
| Vitamin $B_{12}$ Suppl., 10 mg/lb | 0.04 |
| Trace Minerals (CCC 10% Zn) | 0.05 |
| Nutrigard - 25 (25% ethoxyguin) | 0.05 |

DETAILED DESCRIPTION (cont'd)

The 3-carbamoyl-4-hydroxycoumarins of Formula I (including IA, IB, IC, IF, IG, IH, II, IJ, IK, IW, IX, IY, and IZ) can be used as the pure compounds or as mixtures of pure compounds but for practical reasons the compounds are preferably formulated as anthelmintic compositions and administered as a single or multiple dose, alone or in combination with other anthelmintics (e.g. avermectins, benzimidazoles, levamisole, praziquantel, etc.). For example, aqueous or oil suspensions can be administered orally, or the compounds can be formulated with a solid carrier for feeding. Furthermore, an oil suspension can be converted into an aqueous emulsion by mixing with water and injecting the emulsion intramuscularly, subcutaneously or into the peritoneal cavity. In addition, the active compound(s) can be administered topically to the animal in a conventional pour-on formulation.

Pure compounds, mixtures of the active compounds, or combinations thereof with a solid carrier can be administered in the animal's food, or administered in the form of tablets, pills, boluses, wafers, pastes, and other conventional unit dosage forms, as well as sustained release dosage forms which deliver the active compound over an extended period of days, weeks or months. All of these various forms of the active compounds of this invention can be prepared using physiologically acceptable carriers and known methods of formulation and manufacture.

Representative solid carriers conveniently available and satisfactory for physiologically acceptable, unit dosage formulations include corn starch, powdered lactose, powdered sucrose, talc, stearic acid, magnesium stearate, finely divided bentonite, and the like. The active agent can be mixed with a carrier in varying proportions from, for example, about 0.001 percent by weight in animal feed to about 90 or 95 percent or more in a pill or capsule. In the latter form, one might use no more carrier than sufficient to bind the particles of active compound.

In general, the compounds can be formulated in stable powders or granules for mixing in an amount of feed for a single feeding or enough feed for one day and thus obtain therapeutic efficacy without complication. It is the prepared and stored feeds or feed premixes that require care. A recommended practice is to coat a granular formulation to protect and preserve the active ingredient. A prepared sheep-feed containing about 0.2 percent of the active compound will provide a dosage of about 100 mg per kg body weight for each 100 lb sheep in its daily ration.

A solid diluent carrier need not be a homogeneous entity, but mixtures of different diluent carriers can include small proportions of adjuvants such as water; alcohols; protein solutions and suspensions like skimmed milk; edible oils; solutions, e.g., syrups; and organic adjuvants such as propylene glycols, sorbitol, glycerol, diethyl carbonate, and the like.

The solid carrier formulations of the inventions are conveniently prepared in unit dosage forms, to facilitate administration to animals. Accordingly, several large boluses (about 20 g weight) amounting to about 54 g of active compound would be required for a single dosage to a 900 lb horse at a dosage rate of

50 mg/kg of body weight. Similarly, a 60 lb lamb at a dosage rate of 100 mg/kg of body weight would require a pill, capsule, or bolus containing about 2.7 g of active compound. A small dog, on the other hand, weighing about 20 lbs. would require a total dosage of about 225 mg at a dosage rate of 25 mg/kg of body weight. The solid, unit dosage forms can be conveniently prepared in various sizes and concentrations of active ingredient, to accommodate treatment of the various sizes of animals that are parasitized by worms.

Liquid formulations can also be used. Representative liquid formulations include aqueous (including isotonic saline) suspensions, oil solutions and suspensions, and oil in water emulsions. Aqueous suspensions are obtained by dispersing the active compound in water, preferably including a suitable surface-active dispersing agent such as cationic, anionic, or non-ionic surface-active agents. Representative suitable ones are polyoxyalkylene derivatives of fatty alcohols and of sorbitan esters, and glycerol and sorbitan esters of fatty acids. Various dispersing or suspending agents can be included and representative ones are synthetic and natural gums, tragacanth, acacia, alginate, dextran, gelatin, sodium carboxymethylcellulose, methylcellulose, sodium polyvinylpyrrolidone, and the like. The proportion of the active compound in the aqueous suspensions of the invention can vary from about 1 percent to about 20 percent or more.

Oil solutions are prepared by mixing the active compound and an oil, e.g. an edible oil such as cottonseed oil, peanut oil, coconut oil, modified soybean oil, and sesame oil. Usually, solubility in oil will be limited and oil suspensions can be prepared by mixing additional finely divided compound in the oil.

Oil in water emulsions are prepared by mixing and dispersing an oil solution or suspension of the active compound in water preferably aided by surface-active agents and dispersing or suspending agents as indicated above.

In general, the formulations of this invention are administered to animals so as to achieve therapeutic or prophylactic levels of the active compound. Effective dosages of various compounds are contemplated, e.g., in the range of 1 to 75 mg/kg of body weight.

In other animals, and for other kinds of parasitic worms, definitive dosages can be proposed. Contemplated are dosage rates of about 1 mg to about 800 mg/kg of body weight. A preferred, contemplated range of dosage rates is from about 1 mg to about 50 mg/kg of body weight. In this regard, it should be noted that the concentration of active compound in the formulation selected for administration is in many situations not critical. One can administer a larger quantity of a formulation having a relatively low concentration and achieve the same therapeutic or prophylactic dosage as a relatively small quantity of a relatively more concentrated formulation. More frequent small dosages will likewise give results comparable to one large dose. One can also administer a sustained release dosage system (protracted delivery formulation) so as to provide therapeutic and/or prophylactic dosage amounts over an extended period. Unit dosage forms in accordance with this invention can have anywhere from less than 1 mg to 500 g of active compound per unit.

Although the anthelmintic agents of this invention will find their primary use in the treatment and/or prevention of helminth parasitism in valuable warm-blooded domesticated animals such as sheep, cattle, horses, dogs, swine, goats and poultry, they are also effective in treatment that occurs in other warm blooded animals including man. The optimum amount to be employed for best results will, of course, depend upon the particular compound employed, species of animal to be treated, the regimen of treatment and the type and severity of helminth infection. Generally good results are obtained with compounds of Formula I by the oral or parenteral route of administration of about 1 to 300 mg/kg of animal bodyweight (such total dose being given at one time, in a protracted manner or in divided doses over a short period of time such as 1-4 days). The technique for administering these materials to animals are known to those skilled in the veterinary and medical fields.

It is contemplated that the compounds of Formula I can be used to treat various helminth diseases in humans, including those caused by Ascaris, Enterobius, Ancylostoma, Trichuris, Strongyloides, Fasciola, Taenia, and/or Onchocerca or other filariae at a dose of from 1 mg/kg to 300 mg/kg of body weight upon oral and/or parenteral administration.

The anticoccidial compositions of this invention containing as the active ingredient a 3-carbamoyl-4-hydroxycoumarin of Formula IW [wherein $R'_2$ is hydrogen, $CF_3$ or halogen (Cl, Br, F or I), $R'_4$ is Br, F, Cl or $CF_3$ and R is Br, F or $CF_3$] (preferably 4-chlorophenyl, more preferably 2,4-difluorophenyl), hydrates or pharmaceutically acceptable salts are prepared by mixing the active ingredient with an inert ingredient. The inert ingredient can comprise a feedstuff, extender materials and the like. By the term "inert ingredient" is meant a material which does not function as an antiparasitic agent, e.g., an anticoccidial, is in active with respect to the active ingredient and which may be safely ingested by the animals to be treated, and thus, such inert material is one which is inactive for the purpose of the present invention.

The active ingredient, when orally administered to coccidiosis susceptible domestic animals, particularly fowl such as turkeys and chickens, as a component of feed, effectively controls the disease by preventing

it. (Furthermore, the treated fowl either maintain their weight or actually gain weight when compared to controls. Thus, the compositions of this invention not only control coccidiosis, but also aids in improving the efficiency of conversion of feed to weight gains.)

The actual concentration of the active ingredient in animal feed can, of course, be adjusted to the individual needs and may vary over a wide range. The limiting criteria of the concentration are that the minimum concentration is such that a sufficient amount of active ingredient is provided to effect the desired control of coccidiosis and the maximum concentration is such that the amount of composition ingested does not result in any untoward or undesirable side effects.

Thus, for example, a feed premix or complete feed contains sufficient active ingredient to provide from about 1 to 250 ppm in feed.

The optimum dose level will, of course, vary with the size of the animal. When using the antibiotics in accordance with the invention for treating or preventing coccidiosis, it can be first compounded or blended with a feed ingredient or carrier to become a feed additive premix, a feed concentrate, or a feed additive supplement. A feed additive, concentrate or premix is an article intended to be diluted to produce a complete feed, i.e., an article intended to be administered as a sole ration. A feed additive supplement is an article intended for consumption by an animal directly or which can be further diluted to produce a complete feed or can be ingested and used as a supplement to other rations. Feed additive supplements, concentrates and premixes contain the active ingredient to a suitable carrier and mixing in a manner to give substantially uniform dispersion of the anticoccidial in the carrier. Suitable carriers are solids that are inert with respect to the active ingredient and which may safely be ingested by the animals to be treated. Typical of such carriers are commercial poultry feeds, ground cereal grains, grain by-products, plant protein concentrates (soy, peanuts, et.), fermentation by-products, salts, limestone, inorganic compounds, and the like or admixtures thereof. Liquid dispersions can be prepared by using water or vegetable oil preferably including a surface active agent, emulsifying agent, and the like, in the liquid dispersion such a ethylenediaminetetraacetic acid, etc., and solubilizers. Any suitable carrier or extender material can function a the inert ingredient in the solid form of the antiparasitic agent provided that it is inert to the active material and is non-toxic insofar as the animal to which it is to be administered is concerned.

The active ingredient may be blended into a mesh, pellet, or any desired configuration with the inert carrier or extender solid material by any convenient technique. For example, compositions can be found by finely grinding or pulverizing the active ingredient and the inert ingredient using any commercially available grinder or pulverizer with or without the feed material being present. If the feed material is not present when the grinding or pulverizing is effected, the resultant material can be distributed, in accordance with the present invention, in any conveniently available feed material. Typically, poultry feeds which can be medicated with the active ingredient of this invention can contain several ingredients; for example, they can contain high energy grain products such a corn, wheat, wheat red dog flour, milo, oatmeal, or the like; medium and low energy grain products, such as oats, barley, wheat flour, middlings, standard middlings or the like; stabilized fats; vegetable protein such as soybean meal, corn gluten meal, peanut meal, or the like; animal protein such as fish meal, fish solubles, meat scraps or the like; UGF (Unidentified Growth Factor) sources and other B-vitamin carriers such as dried milk products, dried brewers yeast, distillers dried solubles, fermentation solubles, or the like; dehydrated alfalfa meal; and various special additives such as additional riboflavin, vitamin $B_{12}$, calcium pantothenate, niacin, choline, vitamin K and vitamin E or the like, as well as stabilized vitamin A, vitamin $D_3$ (D-activated animal sterols); calcium and phosphorus supplements such as dicalcium phosphate, steamed bone meal, defluorinated phosphate, limestone, or the like; iodized salt, manganese sulfate, zinc carbonate, an antibiotic feed supplement; methionine or its hydroxy analog, and the antioxidant.

As is evident from the above, the anticoccidial compositions are intended for oral ingestion. They can be added to the normal feed supply of the treated animal or can be administered by other procedures, such as incorporating the same in a tablet, pill or bolus and supplying it forcibly to the animal. The administration of the active ingredient must be considered in terms of the specific animal under the husbandry practices encountered.

FORMULAE

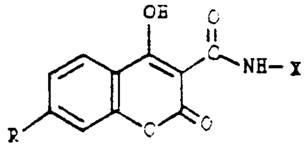

I

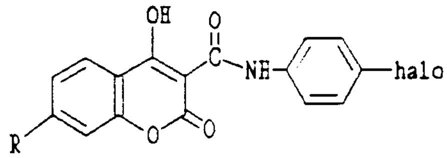

IA

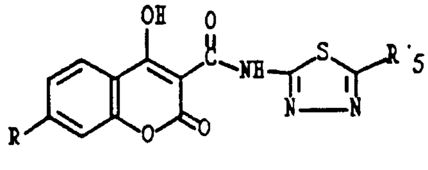

IB

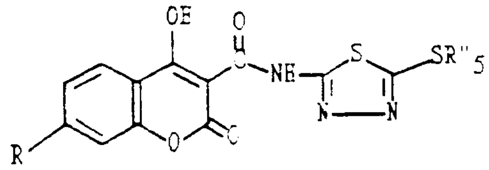

IC

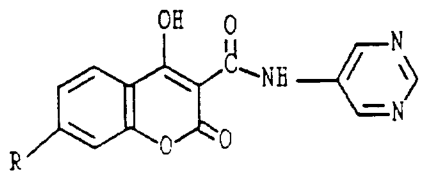

IF

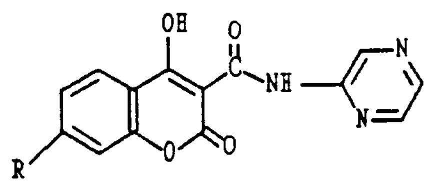

IG

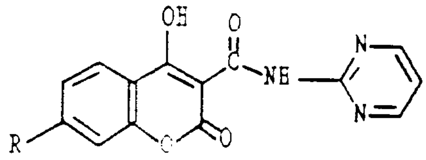

IH

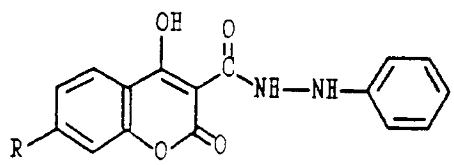

II

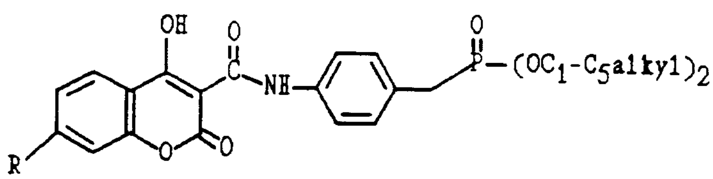

IJ

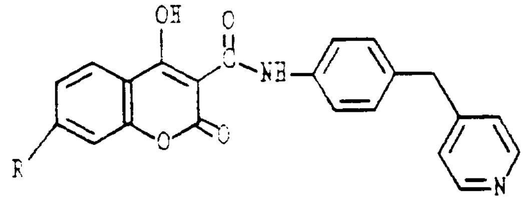

IK

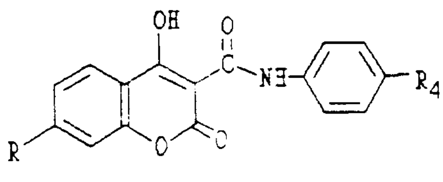

IL

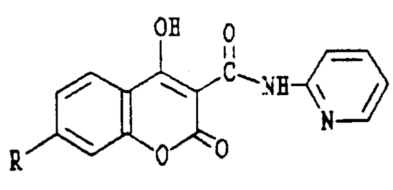

IX

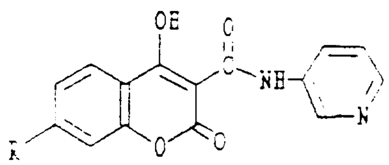

IY

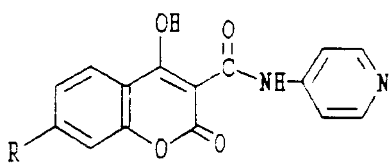

IZ

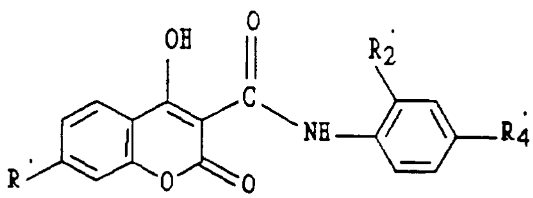

**IW**

SPECIFIC FORMULAE

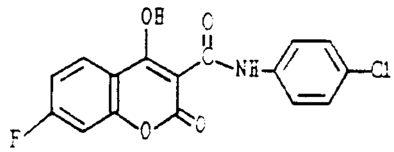

Cpd 6

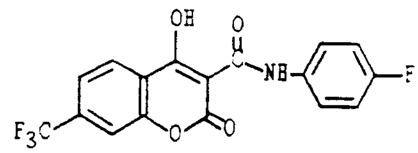

Cpd 8

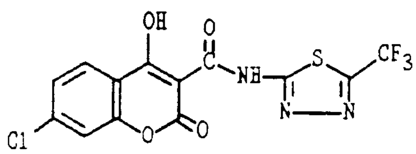

Cpd 11

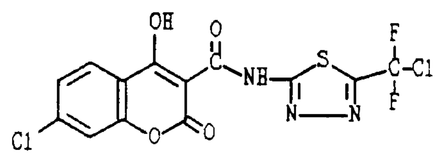

Cpd 17

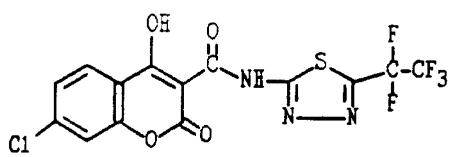

Cpd 18

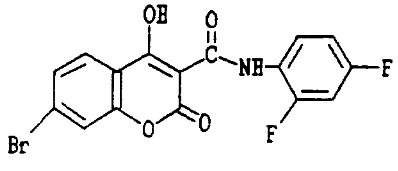

Cpd 53

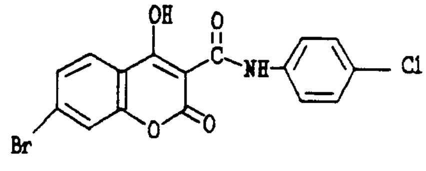

Cpd 54

## CHART A

Scheme A.

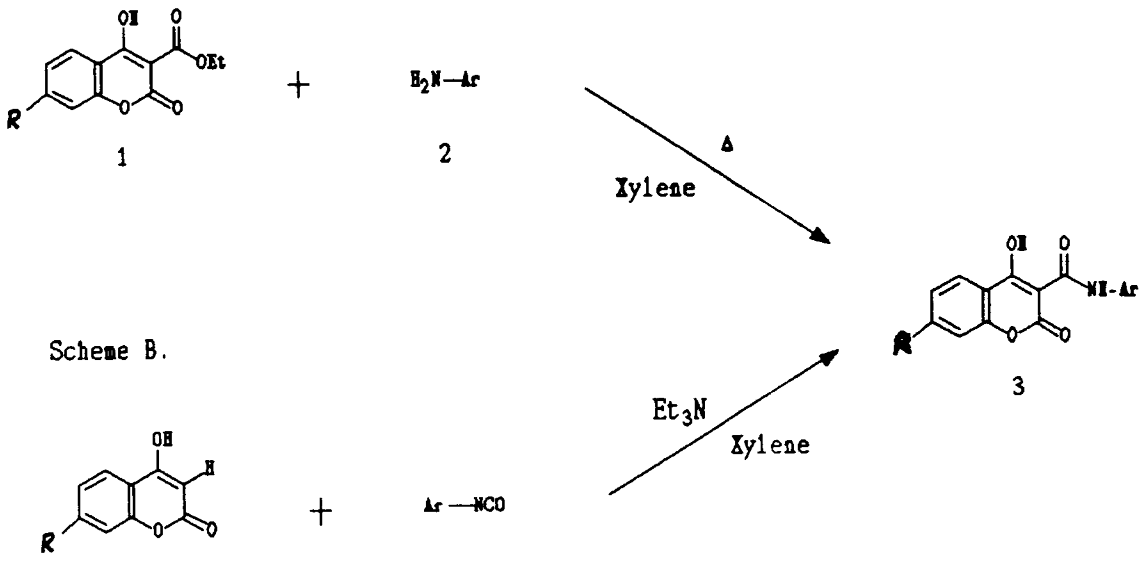


1 2 3

Scheme B.

4 5

## CHART B

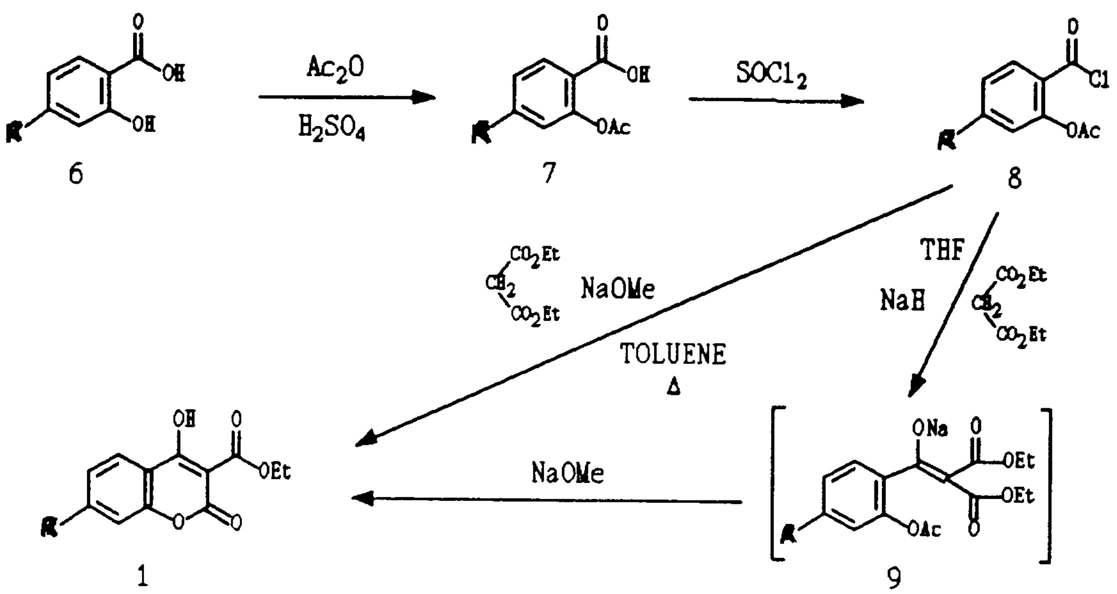


6 7 8 9 1

TABLE I

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #1 | 50 PPM | 4.0 | 2.7500 MG/JIRD | 100 | |
| | 50 PPM | 5.0 | 2.7500 MG/JIRD | 100 | 0 |
| #2 | 50 PPM | 5.0 | 2.7500 MG/JIRD | 100 | 56 |
| | 50 PPM | 5.0 | 2.7500 MG/JIRD | 99 | 72 |
| #3 | 50 PPM | 5.0 | 2.7500 MG/JIRD | 100 | 22 |
| | 50 PPM | 5.0 | | | |
| #4 | 50 PPM | 5.0 | 2.7500 MG/JIRD | 100 | 32 |
| | 50 PPM | 5.0 | | | |
| #5 | 50 PPM | 5.0 | 2.7500 MG/JIRD | 100 | |
| | 50 PPM | 5.0 | 2.7500 MG/JIRD | 99 | 78 |
| #6 | 50 PPM | 5.0 | 2.7500 MG/JIRD | 100 | 88 |
| | 50 PPM | 5.0 | 2.7500 MG/JIRD | 100 | 77 |
| #7 | 50 PPM | 5.0 | 2.7500 MG/JIRD | 100 | |
| | 50 PPM | 5.0 | 2.7500 MG/JIRD | 96 | 0 |
| #8 | 50 PPM | 5.0 | 0.9200 MG/JIRD | 99 | 73 |
| | | | 0.9200 MG/JIRD | 100 | 69 |
| #9 | 50 PPM | 1.0 | 2.7500 MG/JIRD | 100 | 31 |
| | 50 PPM | 1.0 | | | |
| #10 | 50 PPM | 5.0 | 2.7500 MG/JIRD | 100 | |
| | 50 PPM | 5.0 | 2.7500 MG/JIRD | 100 | 20 |
| #11 | 50 PPM | 5.0 | 2.7500 MG/JIRD | 98 | |
| | 50 PPM | 5.0 | 2.7500 MG/JIRD | 95 | 100 |
| #12 | 50 PPM | 2.0 | 2.7500 MG/JIRD | 100 | 87 |
| | 50 PPM | 0.5 | 2.7500 MG/JIRD | 99 | 67 |
| #13 | 50 PPM | 0.0 | 2.7500 MG/JIRD | 99 | |
| | | | 2.7500 MG/JIRD | 100 | 0 |

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #14 | 50 PPM | 0.0 | 2.7500 MG/JIRD | 99 | |
| | | | 2.7500 MG/JIRD | 100 | 6 |
| #15 | 50 PPM | 0.0 | 2.7500 MG/JIRD | 98 | 97 |
| | 50 PPM | 3.0 | 2.7500 MG/JIRD | 67 | 52 |
| #16 | 50 PPM | 5.0 | 2.7500 MG/JIRD | 75 | |
| | 50 PPM | 5.0 | 2.7500 MG/JIRD | 87 | 76 |
| #17 | 50 PPM | 5.0 | 2.7500 MG/JIRD | 96 | 92 |
| | 50 PPM | 5.0 | 2.7500 MG/JIRD | 96 | 96 |
| #18 | 50 PPM | 5.0 | 2.7500 MG/JIRD | 100 | 100 |
| | 50 PPM | 5.0 | 2.7500 MG/JIRD | 98 | 96 |
| #19 | 50 PPM | 4.5 | 2.7500 MG/JIRD | 100 | 46 |
| | 50 PPM | 5.0 | 2.7500 MG/JIRD | 88 | 70 |
| #20 | 50 PPM | 5.0 | 2.7500 MG/JIRD | 98 | 75 |
| | 50 PPM | 4.0 | 2.7500 MG/JIRD | 100 | 82 |
| #23 | 50 PPM | 0.0 | 2.7500 MG/JIRD | 99 | |
| | 50 PPM | 0.5 | 2.7500 MG/JIRD | 97 | 29 |
| #24 | 50 PPM | 0.0 | 2.7500 MG/JIRD | 65 | 0 |
| #25 | 50 PPM | 1.0 | 2.7500 MG/JIRD | 100 | |
| | 50 PPM | 0.0 | 2.7500 MG/JIRD | 99 | 19 |
| #26 | 50 PPM | 0.0 | 2.7500 MG/JIRD | 88 | 0 |
| | 50 PPM | 0.0 | | | |
| #27 | 50 PPM | 0.0 | 2.7500 MG/JIRD | 100 | |
| | | | 2.7500 MG/JIRD | 100 | 0 |
| #28 | 50 PPM | 0.0 | 2.7500 MG/JIRD | 51 | |
| | | | 2.7500 MG/JIRD | 77 | 0 |

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #29 | 50 PPM | 0.0 | 2.7500 MG/JIRD | 71 | |
| | | | 2.7500 MG/JIRD | 98 | 25 |
| #30 | 50 PPM | 4.0 | 2.7500 MG/JIRD | 100 | 0 |
| | 50 PPM | 4.5 | | | |
| #31 | 50 PPM | 0.0 | 2.7500 MG/JIRD | 100 | |
| | 50 PPM | 0.0 | 2.7500 MG/JIRD | 100 | 61 |
| #32 | 50 PPM | 0.0 | 2.7500 MG/JIRD | 86 | 9 |
| | 50 PPM | 0.0 | 2.7500 MG/JIRD | 87 | |
| #33 | 50 PPM | 0.0 | 2.7500 MG/JIRD | 81 | 19 |
| #34 | 50 PPM | 0.0 | 2.7500 MG/JIRD | 100 | |
| | 50 PPM | 0.5 | 2.7500 MG/JIRD | 100 | 13 |
| #35 | 50 PPM | 0.0 | 2.7500 MG/JIRD | 94 | |
| | 50 PPM | 0.0 | 2.7500 MG/JIRD | 94 | 0 |
| #36 | 50 PPM | 0.0 | 2.1700 MG/JIRD | 84 | |
| | | | 2.1700 MG/JIRD | 95 | 3 |
| #37 | 50 PPM | 1.0 | 2.7500 MG/JIRD | 79 | |
| | 50 PPM | 2.5 | 2.7500 MG/JIRD | 76 | 1 |
| #38 | 50 PPM | 4.0 | 2.7500 MG/JIRD | 63 | 0 |
| | 50 PPM | 0.0 | | | |
| #39 | 50 PPM | 4.0 | 2.7500 MG/JIRD | 98 | 21 |
| | 50 PPM | 3.5 | | | |
| #40 | 50 PPM | 2.5 | 2.7500 MG/JIRD | 68 | |
| | 50 PPM | 3.0 | 2.7500 MG/JIRD | 30 | 11 |
| #41 | 50 PPM | 0.0 | 2.7500 MG/JIRD | 57 | |
| | | | 2.7500 MG/JIRD | 48 | 7 |
| #42 | 50 PPM | 0.0 | 2.7500 MG/JIRD | 75 | |
| #43 | 50 PPM | 2.0 | 2.7500 MG/JIRD | 74 | |

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #44 | 50 PPM | 1.0 | 2.7500 MG/JIRD | 69 | |
| | 50 PPM | 1.5 | 2.7500 MG/JIRD | 76 | 19 |
| #45 | 50 PPM | 5.0 | 1.4200 MG/JIRD | 97 | 91 |
| | 50 PPM | 5.0 | 1.4200 MG/JIRD | 86 | 88 |
| | 5 PPM | 4.0 | 1.0000 MG/JIRD | 77 | 47 |
| #46 | 50 PPM | 5.0 | 2.7500 MG/JIRD | 100 | |
| | 50 PPM | 5.0 | 2.75 MG/JIRD | 99 | 16 |
| #47 | 50 PPM | 5.0 | 2.7500 MG/JIRD | 99 | |
| | 50 PPM | 5.0 | 2.7500 MG/JIRD | 96 | 0 |
| | 5 PPM | 5.0 | 7.500 MG/JIRD | 100 | 28 |
| #48 | 50 PPM | 0.0 | 2.7500 MG/JIRD | 61 | |
| #49 | 50 PPM | 5.0 | 2.7500 MG/JIRD | 100 | 0 |
| | 50 PPM | 4.5 | 2.7500 MG/JIRD | 91 | 39 |
| #50 | 50 PPM | 5.0 | 2.7500 MG/JIRD | 87 | |
| | 50 PPM | 5.0 | 2.7500 MG/JIRD | 88 | |
| #51 | 50 PPM | | 2.7500 MG/JIRD | 62 | |
| #52 | 50 PPM | 0.5 | 2.7500 MG/JIRD | 87 | |
| | 50 PPM | | 2.7500 MG/JIRD | 96 | 0 |
| #53 | 50 PPM | 4.0 | 2.7500 MG/JIRD | 100 | 78 |
| | 50 PPM | 3.0 | 2.7500 MG/JIRD | 99 | 51 |
| #54 | 50 PPM | 5.0 | 2.7500 MG/JIRD | 100 | 2 |
| | 50 PPM | 5.0 | | | |
| #55 | 50 PPM | 0.0 | 2.7500 MG/JIRD | 60 | |
| #56 | 50 PPM | 5.0 | 2.7500 MG/JIRD | 100 | |
| | 50 PPM | 4.5 | 2.7500 MG/JIRD | 100 | 0 |
| #58 | | | 1.0000 MG/JIRD | 99 | 11 |

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #60 | 50 PPM | 0 | 2.7500 MG/JIRD | 91 | |
| | | | 2.7500 MG/JIRD | 97 | 40 |
| #61 | 50 PPM | 5.0 | 2.7500 MG/JIRD | 93 | |
| | 50 PPM | 5.0 | 2.7500 MG/JIRD | toxic | |
| #62 | 50 PPM | 5.0 | 2.7500 MG/JIRD | 100 | 40 |
| | 50 PPM | 5.0 | | | |
| #63 | 50 PPM | 2.0 | 2.7500 MG/JIRD | 92 | |
| | 50 PPM | 0.0 | 2.7500 MG/JIRD | 97 | 7 |
| #64 | 50 PPM | 4.5 | 2.7500 MG/JIRD | 99 | |
| | 50 PPM | 5.0 | 2.7500 MG/JIRD | 99 | 12 |
| #65 | 50 PPM | 5.0 | 2.7500 MG/JIRD | 98 | |
| | 50 PPM | 4.5 | 2.7500 MG/JIRD | 100 | 36 |
| #67 | 50 PPM | 5.0 | 2.7500 MG/JIRD | 97 | 45 |
| | 50 PPM | 4.5 | | | |
| #68 | 50 PPM | 1.5 | 2.7500 MG/JIRD | 80 | 70 |
| | 50 PPM | 2.0 | | | |
| #69 | 50 PPM | 0.0 | 2.7500 MG/JIRD | 85 | 6 |
| #70 | 50 PPM | 1.0 | 1.7500 MG/JIRD | 82 | 41 |
| | 50 PPM | 0.0 | 1.7500 MG/JIRD | 88 | 0 |
| #71 | 50 PPM | 0.0 | 2.7500 MG/JIRD | 100 | |
| | 50 PPM | 0.0 | 2.7500 MG/JIRD | 100 | 0 |
| #72 | 50 PPM | 5.0 | 2.7500 MG/JIRD | 97 | |
| | 50 PPM | 3.0 | 2.7500 MG/JIRD | 99 | 0 |

| Compound No. | Anthelmintic C-elegans Phase I (EGG Screen) | | Anthelmintic Jird Phase 2 Screen | | |
|---|---|---|---|---|---|
| | Conc | Score Day 7 | Dose | % Red HC | % Red TC |
| #73 | 50 PPM | 4.0 | 2.7500 MG/JIRD | 100 | |
| | 50 PPM | 4.5 | 2.7500 MG/JIRD | 100 | 38 |
| | | | 2.7500 MG/JIRD | 100 | 55 |
| | | | 2.7500 MG/JIRD | 100 | 18 |
| | | | 2.7500 MG/JIRD | 96 | 0 |
| #74 | 50 PPM | 1.0 | 2.7500 MG/JIRD | 92 | |
| | 50 PPM | 0.0 | 2.7500 MG/JIRD | 100 | 0 |
| #75 | 50 PPM | 0.0 | 2.7500 MG/JIRD | 99 | 34 |
| #76 | 50 PPM | 0.0 | 2.7500 MG/JIRD | 72 | 31 |
| #77 | 50 PPM | 1.0 | 2.7500 MG/JIRD | 97 | 29 |
| | 50 PPM | 0.0 | | | |
| | 50 PPM | 0.0 | | | |
| #78 | 50 PPM | 5.0 | 2.7500 MG/JIRD | 89 | 23 |
| | 50 PPM | 0.0 | | | |
| #79 | 50 PPM | 1.0 | 2.7500 MG/JIRD | 95 | 37 |
| | 50 PPM | 0.0 | | | |
| | 50 PPM | 0.0 | | | |
| #80 | 50 PPM | 5.0 | 2.7500 MG/JIRD | toxic | |
| | 50 PPM | 5.0 | 0.92 MG/JIRD | 99 | |
| | | | 0.92 MG/JIRD | 100 | 0 |
| #81 | 50 PPM | 5.0 | 2.7500 MG/JIRD | 100 | 44 |
| | 50 PPM | 5.0 | 2.7500 MG/JIRD | 98 | 0 |
| #91 | 50 PPM | 5.0 | 2.7500 MG/JIRD | 100 | 1 |
| | 50 PPM | 5.0 | | | |

TABLE II

| Compound No. | Anthelmintic Mouse Phase 2 Screen | | |
|---|---|---|---|
| | Dose | Num-Dose | PR_ND |
| #1 | .0500000%DRUG FOOD | 1 | 100.0 |
| | 2.75 MG/MSE/DAY | 4 | 100.0 |
| #2 | 2.75 MG/MSE/DAY | 4 | 26.0 |
| | 1 MG/MSE/DAY | 4 | 7.0 |
| #6 | 2.75 MG/MSE/DAY | 4 | 99.0 |
| #8 | 2.75 MG/MSE/DAY | 3 | 73.0 |
| #10 | .1500000%DRUG FOOD | 1 | 100.0 |
| | 2.75 MG/MSE/DAY | 3 | 100.0 |
| | 1 MG/MSE/DAY | 4 | 100.0 |
| #11 | .0500000%DRUG FOOD | 1 | 100.0 |
| | 2.75 MG/MSE/DAY | 4 | 100.0 |
| | 1 MG/MSE/DAY | 4 | 100.0 |
| | 1 MG/MSE/DAY | 4 | 99.0 |
| #12 | .1000000%DRUG FOOD | 1 | 41.0 |
| | .1500000%DRUG FOOD | 1 | 0.0 |
| | .2000000%DRUG FOOD | 1 | 28.0 |
| | 2.71 MG/MSE/DAY | 1 | 36.0 |
| #13 | .1500000%DRUG FOOD | 1 | 11.0 |
| #14 | 7.5 MG/MSE/DAY | 4 | 96.0 |
| #15 | 1 MG/MSE/DAY | 4 | 52.0 |
| #16 | 2.75 MG/MSE/DAY | 4 | 100.0 |
| #17 | 2.75 MG/MSE/DAY | 4 | 100.0 |
| | 1 MG/MSE/DAY | 4 | 71.0 |
| | 1 MG/MSE/DAY | 4 | 100.0 |
| #18 | 2.75 MG/MSE/DAY | 4 | 100.0 |
| | 1 MG/MSE/DAY | 4 | 76.0 |
| | 1 MG/MSE/DAY | 4 | 99.0 |
| #19 | 2.75 MG/MSE/DAY | 4 | 39.0 |

| Compound No. | Anthelmintic Mouse Phase 2 Screen | | |
|---|---|---|---|
| | Dose | Num-Dose | PR_ND |
| #20 | 2.75 MG/MSE/DAY | 3 | |
| #21 | .1000000%DRUG FOOD | 1 | 56.0 |
| #28 | .1500000%DRUG FOOD | 1 | 40.0 |
| #31 | .1500000%DRUG FOOD | 1 | 0.0 |
| #32 | .1500000%DRUG FOOD | 1 | 58.0 |
| #35 | .1500000%DRUG FOOD | 1 | 2.0 |
| #40 | .1500000%DRUG FOOD | 1 | 15.0 |
| #42 | .1500000%DRUG FOOD | 1 | 0.0 |
| #47 | 2.75 MG/MSE/DAY | 4 | 23.0 |
| | .1000000 DRUG FOOD | 1 | 68.0 |
| | .1500000 DRUG FOOD | 1 | 76.0 |
| | .2000000 DRUG FOOD | 1 | 75.0 |
| #48 | .1500000 DRUG FOOD | 1 | 2.0 |
| #49 | .1500000 DRUG FOOD | 1 | 44.0 |
| | .2000000 DRUG FOOD | 1 | 95.0 |
| #50 | .1500000 DRUG FOOD | 1 | 0.0 |
| #51 | .1500000 DRUG FOOD | 1 | 22.0 |
| #52 | .1500000 DRUG FOOD | 1 | 0.0 |
| #55 | .0600000 DRUG FOOD | 1 | 0.0 |
| #56 | .1500000 DRUG FOOD | 1 | 44.0 |
| #60 | .1000000 DRUG FOOD | 1 | 15.0 |
| #61 | .1500000 DRUG FOOD | 1 | 8.0 |
| #64 | .2000000 DRUG FOOD | 1 | 39.0 |
| | .1500000 DRUG FOOD | 1 | 54.0 |
| #65 | .2000000 DRUG FOOD | 1 | 49.0 |
| | .1500000 DRUG FOOD | 1 | 0.0 |
| #68 | 2.75 MG/MSE/DAY | 4 | 48.0 |

TABLE III

| Compound No. | Anthelmintic New Sheep Phase 3 | | |
|---|---|---|---|
| | Parasite | Treatment Dose | Pct Clr |
| #1 | HC | 12.5 MG/KG | 98.3 |
| | HC | 12.5 MG/KG | 32.9 |
| #2 | HC | 12.5 MG/KG | 99.9 |
| #5 | HC | 12.5 MG/KG | 93.0 |
| | HC | 12.5 MG/KG | 66.0 |
| #6 | HC | 12.5 MG/KG | 100.0 |
| | HC | 12.5 MG/KG | 99.9 |
| #8 | HC | 12.5 MG/KG | 99.4 |
| | HC | MG/KG | 80.5 |
| #12 | HC | 12.5 MG/KG | 0.0 |
| | HC | 12.5 MG/KG | 86.4 |
| | HC | 12.5 MG/KG | 100.0 |
| | HC | 12.5 MG/KG | 75.2 |
| #11 | HC | 12.5 MG/KG | 99.8 |
| | HC | 12.5 MG/KG | 99.8 |
| | HC | 12.5 MG/KG | 94.5 |
| | HC | 12.5 MG/KG | 100.0 |
| | HC | 4.2 MG/KG | 25.3 |
| #15 | HC | 12.5 MG/KG | 93.1 |
| | HC | 12.5 MG/KG | 95.0 |
| #16 | HC | 12.5 MG/KG | 91.0 |
| | HC | 12.5 MG/KG | 20.7 |
| | HC | 12.5 MG/KG | 23.7 |
| | HC | 12.5 MG/KG | 58.6 |
| #17 | HC | 12.5 MG/KG | 100.0 |
| | HC | 12.5 MG/KG | 100.0 |
| #18 | HC | 12.5 MG/KG | 100.0 |
| | HC | 12.5 MG/KG | 100.0 |

| | | | |
|---|---|---|---|
| #19 | HC | 12.5 MG/KG | 99.5 |
| #20 | HC | 12.5 MG/KG | 90.4 |
| #31 | HC | 12.5 MG/KG | 0.0 |
| | HC | 12.5 MG/KG | 0.0 |
| | HC | 12.5 MG/KG | 43.6 |
| | HC | 12.5 MG/KG | 46.4 |
| #47 | HC | 12.5 MG/KG | 100.0 |
| | HC | 4.20 MG/KG | 99.7 |
| | HC | 12.5 MG/KG | 100.0 |
| | HC | 4.20 MG/KG | 99.7 |
| #49 | HC | 12.5 MG/KG | 99.9 |
| #50 | HC | 12.5 MG/KG | 99.9 |
| #53 | HV | 12.5 MG/KG | 99.8 |
| #56 | HC | 12.5 MG/KG | 99.8 |
| #60 | HC | 12.5 MG/KG | 4.7 |
| #73 | HC | 12.5 MG/KG | 99.9 |

TABLE IV

| Compd No. | Anticoccidial *in vivo* Chick | | | | | | |
|---|---|---|---|---|---|---|---|
| | Dose | WtGain | PctSur | DrpScr | Les_U | Les_M | Les_C |
| #5 | 93 PPM | -24.00 | 0.0 | 3.0 | 3.0 | 3.0 | 3.0 |
| #6 | 500 PPM | -30.70 | 0.0 | 0.0 | | | |
| | 250 PPM | -34.00 | 66.7 | 0.0 | 3.0 | 2.5 | 0.5 |
| | 127 PPM | -34.00 | 100.0 | 0.0 | 3.0 | 3.0 | 0.0 |
| | 63 PPM | -16.60 | 100.0 | 0.0 | 3.0 | 2.0 | 1.0 |
| | 32 PPM | -16.60 | 33.3 | 3.0 | 3.0 | 2.0 | 2.0 |
| #8 | 367 PPM | -36.00 | 66.7 | 0.0 | 2.0 | 2.0 | 0.7 |
| | 183 PPM | -27.33 | 33.3 | 0.0 | 3.0 | 3.0 | 0.7 |
| | 46 PPM | -16.67 | 0.0 | 3.0 | 3.0 | 3.0 | 3.0 |
| #53 | 167 PPM | 10.67 | 100.0 | 0.0 | 3.0 | 2.7 | 0.7 |
| #58 | 200 PPM | | 0.0 | | | | |
| | 50 PPM | 23.00 | 100.0 | 2.0 | 3.0 | 3.0 | 2.3 |
| | 76 PPM | 24.40 | 100.0 | 1.0 | 2.0 | 1.0 | 1.3 |
| | 100 PPM | -14.70 | 100.0 | 0.0 | 1.3 | 1.3 | 0.0 |
| | 50 PPM | -1.70 | 100.0 | 0.0 | 2.3 | 2.3 | 0.7 |
| | 64 PPM | 21.00 | 100.0 | 0.0 | 1.7 | 1.3 | 0.0 |
| | 76 PPM | -6.00 | 100.0 | 0.0 | 2.0 | 2.0 | 1.3 |
| | 100 PPM | 2.00 | 100.0 | 0.0 | 2.7 | 2.0 | 1.3 |
| #60 | 333 PPM | | 100.0 | 0.0 | 3.0 | 2.0 | 1.7 |
| #62 | 400 PPM | | 0.0 | | | | |
| | 100 PPM | | 0.0 | | | | |
| | 200 PPM | | 0.0 | | | | |
| | 50 PPM | | 50.0 | | | | |
| | 36 PPM | 5.70 | 100.0 | 2.0 | 3.0 | 2.3 | 2.7 |
| | 50 PPM | 22.30 | 66.7 | 3.0 | 3.0 | 3.0 | 3.0 |
| | 76 PPM | -37.60 | 33.3 | | | | |

| Compd No. | Anticoccidial *in vivo* Chick | | | | | | |
|---|---|---|---|---|---|---|---|
| | Dose | WtGain | PctSur | DrpScr | Les_U | Les_M | Les_C |
| | .0130000 G/3KG | 19.70 | 100.0 | 2.0 | 2.3 | 2.3 | 2.0 |
| #65 | 500 PPM | | 100.0 | 2.0 | 3.0 | 2.7 | 0.7 |
| #72 | 448 PPM | -10.00 | 66.7 | 2.0 | 3.0 | 3.0 | 2.0 |
| | 230 PPM | 2.00 | 0.0 | 3.0 | 3.0 | 3.0 | 3.0 |
| | 213 PPM | -2.00 | 0.0 | 3.0 | 3.0 | 3.0 | 3.0 |
| #91 | 500 PPM | 3.30 | 66.7 | 0.0 | 2.0 | 2.0 | 0.7 |
| | 200 PPM | -1.30 | 66.7 | 3.0. | 2.7. | 2.7. | 2.7. |
| | 400 PPM | | 0.0 | | | | |
| | 100 PPM | 10.30 | 100.0 | 0.0 | 2.3 | 2.3 | 0.0 |
| | 200 PPM | | 33.3 | | | | |
| | 100 PPM | 33.30 | 100.0 | 3.0 | 3.0 | 3.0 | 2.7 |
| | 50 PPM | 28.70 | 33.3 | 3.0. | 3.0 | 2.7 | 2.7 |
| | 76 PPM | 12.60 | 33.3 | 3.0 | 3.0 | 2.7 | 3.0 |
| | 100 PPM | 30.00 | 66.7 | 2.0 | 3.0 | 2.3 | 1.7 |
| #92 | 300 PPM | -1.00 | 66.7 | 0.0 | 1.0 | 0.0 | 0.0 |
| | 50 PPM | 28.70 | 100.0 | 0.0 | 3.0 | 2.3 | 1.3 |
| | 76 PPM | -8.40 | 66.7 | 0.0 | 1.3 | 1.7 | 0.3 |
| | 100 PPM | -30.00 | 0.0 | 0.0 | | | |
| | 150 PPM | -27.70 | 33.3 | 0.0 | 1.0 | 0.7 | 0.3 |
| | 200 PPM | | 33.3 | | | | |
| | 36 PPM | 45.00 | 100.0 | 2.0 | 3.0 | 2.3 | 2.0 |

| Compd No. | Anticoccidial *in vivo* Chick | | | | | | |
|---|---|---|---|---|---|---|---|
| | Dose | WtGain | PctSur | DrpScr | Les_U | Les_M | Les_C |
| #92 | 50 PPM | 11.00 | 66.7 | 1.0 | 3.0 | 2.7 | 2.0 |
| (cont) | 64 PPM | -32.00 | 66.7 | 2.0 | 3.0 | 3.0 | 2.3 |
| | 76 PPM | -25.00 | 66.7 | 0.0 | 3.0 | 3.0 | 1.0 |
| | 76 PPM | 5.00 | 66.7 | 2.0 | 2.3 | 2.3 | 2.3 |

## Claims

**Claims for the following Contracting States : AT, BE, CH, DE, DK, FR, GB, GR, IT, LU, NL, SE**

1. Use of a compound of formula I, or a hydrate or pharmaceutically-acceptable salt thereof, for the manufacture of a medicament for use in killing parasitic worms in humans and valuable warm-blooded domestic animals, wherein formula I is

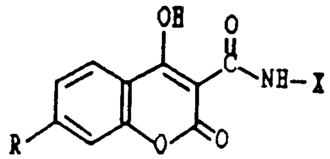

I

wherein X is selected from

(1) 4-halophenyl optionally-substituted at the 2-position by Cl, Br, F or I;

(2) (5-$R'_5$)-1,3,4-thiadiazol-2-yl, wherein $R'_5$ is Cl, Br, F, I, cyano, $-CF_3$, $-CF_2Cl$, $-CF_2CF_3$ or optionally-substituted benzyl, pyridin-2-yl, pyridin-3-yl or pyridin-4-yl and any substituents are one, two or three members selected from Cl, Br, F, I, CN, $CF_3$, $C_1$-$C_5$ alkyl and $C_1$-$C_5$ alkoxy;

(3) (2-$SR''_5$)-1,3,4-thiadiazol-5-yl, wherein $R''_5$ is hydrogen, $C_1$-$C_5$ alkyl, Cl, Br, F, I, cyano, $-CF_3$, $-CF_2Cl$, $-CF_2CF_3$ or optionally-substituted phenyl, benzyl, pyridin-2-yl, pyridin-3-yl, pyridin-4-yl, pyridin-2-yl($CH_2$)-, pyridin-3-yl($CH_2$)- or pyridin-4-yl($CH_2$)- and any substituents are one, two or three members as defined above;

(4) optionally-substituted pyridin-2-yl, pyridin-3-yl, pyridin-4-yl, pyrimidin-5-yl, pyrazin-2-yl, pyrimidin-2-yl or -NH-phenyl and any substituents are one, two or three members as defined above;

(5) 4-[di($C_1$-$C_5$)alkoxyphosphonomethyl]phenyl; or

(6) 4-(pyridin-4-ylmethyl)phenyl optionally-substituted in the pyridinyl moiety with one or two members as defined above; and

R is hydrogen, phenyl, Cl, Br, F, CN, $CF_3$, $C_1$-$C_5$ alkyl or $C_1$-$C_5$ alkoxy;

with the provisos that, when X is 4-halophenyl, R is Cl, F, Br, $CF_3$ or phenyl, and that the compound of formula I is other than:

3-[N-(5-benzyl-1,3,4-thiadiazol-2-yl)carbamoyl]-7-chloro-4-hydroxycoumarin,
7-chloro-4-hydroxy-3-[N-[5-(2-pyridinylmethyl)thio-1,3,4-thiadiazol-5-yl]carbamoylcoumarin,
7-chloro-4-hydroxy-3-[N-(5-phenyl-1,3,4-thiadiazol-2-yl]carbamoylcoumarin,
3-[N-(2-benzylthio-1,3,4-thiadiazol-5-yl)carbamoyl]-7-chloro-4-hydroxycoumarin,
4-hydroxy-3-[N-(2-pyridinylcarbamoyl)]coumarin,
4-hydroxy-3-[N-(3-pyridinylcarbamoyl)]coumarin,
7-trifluoromethyl-3-[N-(3-chloro-5-trifluoromethyl-pyridin-2-yl]carbamoyl]-4-hydroxycoumarin,
3-[N-(6-chloropyridin-3-yl)carbamoyl]-4-hydroxycoumarin,
3-[N-(6-methylpyridin-2-yl)carbamoyl]-4-hydroxycoumarin,
3-[N-(5-bromopyridin-2-yl)carbamoyl]-7-methoxy-4-hydroxycoumarin, or

3-[N-(2,6-dichloropyridin-4-yl)carbamoyl]-4-hydroxycoumarin.

2. A compound of formula IW or a hydrate or pharmaceutically-acceptable salt thereof, wherein formula IW is

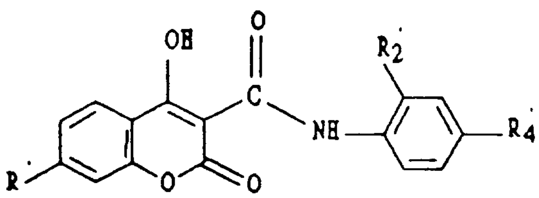

IW

wherein R'$_2$ is hydrogen, $CF_3$, Cl, Br, F or I; R'$_4$ is Br, F, Cl or $CF_3$; and R' is Br, F or $CF_3$; provided that the compound is not 3-[N-(4-chlorophenyl)carbamoyl]-7-trifluoromethyl-4-hydroxycoumarin.

3. A compound of formula I as defined in claim 1, or a hydrate or pharmaceutically-acceptable salt thereof, other than 3-[N-(4-trifluoromethylphenyl)carbamoyl]-4-hydroxycoumarin.

4. A compound according to claim 3, wherein X is 4-halophenyl.

5. A compound according to claim 4, which is:
3-[N-(4-bromophenyl)carbamoyl]-7-chloro-4-hydroxycoumarin;
3-[N-(4-bromophenyl)carbamoyl]-7-trichloromethyl-4-hydroxycoumarin;
3-[N-(4-chlorophenyl)carbamoyl]-7-chloro-4-hydroxycoumarin;
3-[N-(4-chlorophenyl)carbamoyl]-7-trifluoromethyl-4-hydroxycoumarin;
3-[N-(4-chlorophenyl)carbamoyl]-7-fluoro-4-hydroxycoumarin;
3-[N-(4-fluorophenyl)carbamoyl]-7-chloro-4-hydroxycoumarin;
3-[N-(4-fluorophenyl)carbamoyl]-7-trifluoromethyl-4-hydroxycoumarin;
3-[N-(4-fluorophenyl)carbamoyl]-7-fluoro-4-hydroxycoumarin;
or a pharmaceutically-acceptable salt of any of the foregoing compounds.

6. A compound according to claim 3, wherein X is (5-R'$_5$)-1,3,4-thiadiazol-2-yl.

7. A compound according to claim 6, wherein R'$_5$ is $CF_3$, Cl, Br, F, I, CN, $C_1$-$C_5$ alkyl, or benzyl or pyridinyl optionally substituted with one member selected from Cl, Br, F, I, -CN and $CF_3$.

8. A compound according to claim 6, wherein R is Cl, Br or $CF_3$.

9. A compound according to claim 6, which is:
3-[N-(4-bromophenyl)carbamoyl]-7-chloro-4-hydroxycoumarin (Cpd. #1),
3-[N-(4-bromophenyl)carbamoyl]-7-trifluoromethyl-4-hydroxycoumarin (Cpd. #2),
3-[N-(4-bromophenyl)carbamoyl]-7-fluoro-4-hydroxycoumarin (Cpd. #3),
7-chloro-4-hydroxy-3-[N-[5-(trifluoromethyl)-1,3,4-thiadiazol-2-yl]carbamoyl]coumarin (Cpd. #11),
7-chloro-4-hydroxy-3-[N-[5-(chlorodifluoromethyl)-1,3,4-thiadiazol-2-yl]carbamoyl]coumarin (Cpd. #17),
7-chloro-4-hydroxy-3-[N-[5-(trifluoromethyldifluoro-methyl)-1,3,4-thiadiazol-2-yl]carbamoyl]coumarin (Cpd. #18),
or a pharmaceutically-acceptable salt of any of the foregoing compounds.

10. A compound according to claim 3 which is:
3-[N-(4-bromophenyl)carbamoyl]-7-chloro-4-hydroxycoumarin (Cpd. #1),
3-[N-(4-bromophenyl)carbamoyl]-7-trifluoromethyl-4-hydroxycoumarin (Cpd. #2),
3-[N-(4-bromophenyl)carbamoyl]-7-fluoro-4-hydroxycoumarin (Cpd. #3),
3-[N-(4-chlorophenyl)carbamoyl]-7-chloro-4-hydroxycoumarin (Cpd. #4),
3-[N-(4-chlorophenyl)carbamoyl]-7-trifluoromethyl-4-hydroxycoumarin (Cpd. #5),
3-[N-(4-chlorophenyl)carbamoyl]-7-fluoro-4-hydroxycoumarin (Cpd. #6),

3-[N-(4-fluorophenyl)carbamoyl]-7-chloro-4-hydroxycoumarin (Cpd. #7),
3-[N-(4-fluorophenyl)carbamoyl]-7-trifluoromethyl-4-hydroxycoumarin (Cpd. #8),
3-[N-(4-fluorophenyl)carbamoyl]-7-fluoro-4-hydroxycoumarin (Cpd. #9),
7-chloro-4-hydroxy-3-[N-[5-(trifluoromethyl)-1,3,4-thiadiazol-2 yl]carbamoyl]coumarin (Cpd. #11),
4-hydroxy-3-[N-[5-(trifluoromethyl)-1,3,4-thiadiazol-2-yl]carbamoyl]coumarin (Cpd. #12),
4-hydroxy-7-methyl-3-[N-[5-(trifluoromethyl)-1,3,4-thiadiazol-2-yl]carbamoyl]coumarin (Cpd. #13),
4-hydroxy-7-methoxy-3-[N-[5-(trifluoromethyl)-1,3,4-thiadiazol-2-yl]carbamoyl]coumarin (Cpd. #14),
7-fluoro-4-hydroxy-3-[N-[5-(trifluoromethyl)-1,3,4-thiadiazol-2-yl]carbamoyl]coumarin (Cpd. #15),
7-trifluoromethyl-4-hydroxy-3-[N-[5-(trifluoromethyl)-1,3,4-thiadiazol-2-yl]carbamoyl]coumarin (Cpd. #16),
7-chloro-4-hydroxy-3-[N-[5-(chlorodifluoromethyl)-1,3,4-thiadiazol-2-yl]carbamoyl]coumarin (Cpd. #17),
7-chloro-4-hydroxy-3-[N-[5-(trifluoromethyldifluoromethyl)-1,3,4-thiadiazol-2-yl]carbamoyl]coumarin (Cpd. #18),
4-hydroxy-3-[N-[5-(trifluoromethyldifluoromethyl)-1,3,4-thiadiazol-2-yl]carbamoyl]coumarin (Cpd. #19),
7-fluoro-4-hydroxy-3-[N-[5-(trifluoromethyldifluoromethyl)-1,3,4-thiadiazol-2-yl]carbamoyl]coumarin (Cpd. #20),
4-hydroxy-3-[N-(5-phenyl-1,3,4-thiadiazol-2-yl)carbamoyl]coumarin (Cpd. #21),
3-[N-(5-benzyl-1,3,4-thiadiazol-2-yl)carbamoyl]-4-hydroxycoumarin (Cpd. #23),
3-[N-(5-benzyl-1,3,4-thiadiazol-2-yl)carbamoyl]-4-hydroxy-7-methylcoumarin (Cpd. #24),
3-[N-[5-(4-chlorophenyl)methyl-1,3,4-thiadiazol-2-yl]carbamoyl]-4-hydroxycoumarin (Cpd. #25),
7-chloro-3-[N-[5-(4-chlorophenyl)methyl-1,3,4-thiadiazol-2-yl]carbamoyl]-4-hydroxycoumarin (Cpd. #26),
3-[N-[2-[(4-fluorophenyl)methyl]thio-1,3,4-thiadiazol-5-yl]carbamoyl]-4-hydroxycoumarin (Cpd. #27),
4-hydroxy-3-[N-(2-mercapto-1,3,4-thiadiazol-5-yl)carbamoyl]coumarin (Cpd. #28),
4-hydrdoxy-3-[N-[2-(methylthio)-1,3,4-thiadiazol-5-yl]carbamoyl]coumarin (Cpd. #29),
4-hydroxy-3-[N-[5-(3,5-dichloropyridin-2-yl)thio-1,3,4-thiadiazol-2-yl]carbamoyl]coumarin (Cpd. #30),
4-hydroxy-3-[N-[2-(2-pyridinylmethyl)thio-1,3,4-thiadiazol-5-yl]carbamoyl]coumarin (Cpd. #31),
4-hydroxy-7-methyl-3-[N-[2-(2-pyridinylmethyl)thio-1,3,4-thiadiazol-5-yl]carbamoyl]coumarin (Cpd. #32),
3-[N-[2-(4-pyridinylmethyl)thio-1,3,4-thiadiazol-5-yl]carbamoyl]4-hydroxycoumarin (Cpd. #33),
3-[N-[2-(benzylthio)-1,3,4-thiadiazol-5-yl]carbamoyl]-4-hydroxycoumarin (Cpd. #34),
3-[N-(5-bromopyrimidin-2-yl)carbamoyl]-4-hydroxycoumarin (Cpd. #35),
4-hydroxy-3-[N-(phenylamino)carbamoyl]coumarin (Cpd. #36),
4-hydroxy-3-[N-[(4-bromophenyl)amino]carbamoyl]coumarin (Cpd. #37),
4-hydroxy-3-[N-(2-bromo-1,3,4-thiadiazol-5-yl)carbamoyl]coumarin (Cpd. #38),
4-hydroxy-3-[N-(2-chloro-1,3,4-thiadiazol-5-yl)carbamoyl]coumarin (Cpd. #39),
3-[N-(4-chloro-6-methylpyrimidin-2-yl)carbamoyl]-4-hydroxycoumarin (Cpd. #40),
3-[N-(6-chloropyrazin-2-yl)carbamoyl]-4-hydroxycoumarin (Cpd. #41),
3-[N-(4,6-dichloropyrimidin-5-yl)carbamoyl]-4-hydroxycoumarin (Cpd. #42),
4-hydroxy-3-[N-[5-(2-fluorophenyl)-1,3,4-thiadiazol-2-yl]carbamoyl]coumarin(Cpd. #44),
7-phenyl-4-hydroxy-3-[N-[5-(trifluoromethyl)-1,3,4-thiadiazol-2-yl]caramoyl]coumarin (Cpd. #45),
3-[N-(4-bromophenyl)carbamoyl]-7-phenyl-4-hydroxycoumarin (Cpd. #46),
3-[N-(5-bromopyridin-2-yl)carbamoyl]-4-hydroxycoumarin (Cpd. #47),
3-[N-(2-pyridinyl)carbamoyl]-4-hydroxycoumarin (Cpd. #48),
3-[N-(5-chloropyridin-2-yl)carbamoyl]-4-hydroxycoumarin (Cpd. #49),
3-[N-(2-chloropyridin-3-yl)carbamoyl]-4-hydroxycoumarin (Cpd. #50),
3-[N-(5-methylpyridin-2-yl)carbamoyl]-4-hydroxycoumarin (Cpd. #51),
3-[N-(6-methoxypyridin-3-yl)carbamoyl]-4-hydroxycoumarin (Cpd. #52),
7-trifluoromethyl-3-[N-[4-(2,4-difluoro)phenyl]]-4-hydroxycoumarin (Cpd. #53),
7-bromo-3-[N-[4-chlorophenyl)carbamoyl]-4-hydroxycoumarin (Cpd. #54),
3-[N-(3,5-dichloropyridin-2-yl)carbamoyl]-4-hydroxycoumarin (Cpd. #55),
3-[N-(5-bromopyridin-2-yl)carbamoyl]-7-chloro-4-hydroxycoumarin (Cpd. #56),
7-bromo-3-[N-[4-(2,4-difluoro)phenyl]carbamoyl]-4-hydroxycoumarin (Cpd. #58),
3-[N-[5-(1,1-dimethylethyl)-1,3,4-thiadiazol-2-yl)carbamoyl]-4-hydroxycoumarin (Cpd. #60),
3-[N-(5-bromopyridin-2-yl)carbamoyl]-7-methyl-4-hydroxycoumarin (Cpd. #61),
7-bromo-3-[N-(4-fluorophenyl)carbamoyl]-4-hydroxycoumarin (Cpd. #62),

3-[N-(2,3,5,6-tetrafluoropyridin-4-yl)carbamoyl]-6-methoxy-4-hydroxycoumarin (Cpd. #63),
7-chloro-3-[N-(5-chloropyridin-2-yl)carbamoyl]-4-hydroxycoumarin (Cpd. #64),
7-chloro-3-[N-(5-chloropyridin-2-yl)carbamoyl]-4-hydroxycoumarin, triethylamine salt (Cpd. #65),
7-fluoro-3-[N-(5-bromopyridin-2-yl)carbamoyl]-4-hydroxycoumarin (Cpd. #67),
7-chloro-3-[N-(3-chloro-5-trifluoromethylpyridin-2-yl)carbamoyl]-4-hydroxycoumarin (Cpd. #68),
3-[N-(3-chloro-5-trifluoromethylpyridin-2-yl)carbamoyl]-4-hydroxycoumarin (Cpd. #69),
3-[N-(3,5-ditrifluoromethylpyridin-2-yl)carbamoyl]-4-hydroxycoumarin (Cpd. #70),
7-trifluoromethyl-3-[N-(5-bromopyridin-2-yl]carbamoyl]-4-hydroxycoumarin (Cpd. #71),
7-trifluoromethyl-3-[N-(5-chloropyridin-2-yl]carbamoyl]-4-hydroxycoumarin (Cpd. #72),
7-trifluoromethyl-3-[N-(2-chloropyridin-3-yl]carbamoyl]-4-hydroxycoumarin (Cpd. #73),
7-trifluoromethyl-3-[N-(6-chloropyridin-3-yl]carbamoyl]-4-hydroxycoumarin (Cpd. #74),
7-chloro-3-[N-(6-methoxypyridin-3-yl]carbamoyl]-4-hydroxycoumarin (Cpd. #75),
7-trifluoromethyl-3-[N-(6-methoxypyridin-3-yl]carbamoyl]-4-hydroxycoumarin (Cpd. #76),
7-chloro-3-[N-(2-chloropyridin-3-yl]carbamoyl]-4-hydroxycoumarin (Cpd. #77),
7-chloro-3-[N-(6-chloropyridin-3-yl]carbamoyl]-4-hydroxycoumarin (Cpd. #78),
7-chloro-3-[N-(3,5-ditrifluoromethylpyridin-2-yl]carbamoyl]-4-hydroxycoumarin (Cpd. #79),
7-trifluoromethyl-3-[N-(2,3,5,6-tetrafluoropyridin-4-yl]carbamoyl]-4-hydroxycoumarin (Cpd. #80),
7-chloro-4-hydroxy-3-[N-(1,1,2,2,3,3,3-heptafluoropropyl)-1,2,4-thiadiazol-2-yl]carbamoyl]coumarin (Cpd. #81),
7-bromo-3-[N-[4-bromophenyl)carbamoyl]-4-hydroxycoumarin (Cpd. #91),
or a pharmaceutically-acceptable salt of any of the foregoing compounds.

**11.** A compound according to claim 3, which is Cpd. #6, 8, 11, 17 or 18 as defined in claim 10, or a pharmaceutically-acceptable salt thereof.

**12.** A compound according to claim 3, which is 7-bromo-3-[N-(4-chlorophenyl)carbamoyl]-4-hydroxycoumarin or 7-bromo-3-[N-(2,4-difluorophenyl)carbamoyl]-4-hydroxycoumarin, or a hydrate or pharmaceutically-acceptable salt of either.

**13.** Use according to claim 1, wherein the compound is as defined in any of claims 3 to 12.

**14.** Use of a compound according to claim 2, for the manufacture of a medicament for use in preventing or treating coccidiosis in fowl.

**Claims for the following Contracting State : ES**

**1.** A process for preparing a compound of formula I, or a hydrate or pharmaceutically-acceptable salt thereof, wherein formula I is

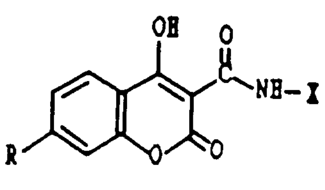

I

wherein X is selected from

(1) 4-halophenyl optionally-substituted at the 2-position by Cl, Br, F or I;

(2) (5-R'$_5$)-1,3,4-thiadiazol-2-yl, wherein R'$_5$ is Cl, Br, F, I, cyano, $-CF_3$, $-CF_2Cl$, $-CF_2CF_3$ or optionally-substituted benzyl, pyridin-2-yl, pyridin-3-yl or pyridin-4-yl and any substituents are one, two or three members selected from Cl, Br, F, I, CN, $CF_3$, $C_1$-$C_5$ alkyl and $C_1$-$C_5$ alkoxy;

(3) (2-SR''$_5$)-1,3,4-thiadiazol-5-yl, wherein R''$_5$ is hydrogen, $C_1$-$C_5$ alkyl, Cl, Br, F, I, cyano, $-CF_3$, $-CF_2Cl$, $-CF_2CF_3$ or optionally-substituted phenyl, benzyl, pyridin-2-yl, pyridin-3-yl, pyridin-4-yl, pyridin-2-yl($CH_2$)-, pyridin-3-yl($CH_2$)- or pyridin-4-yl($CH_2$)- and any substituents are one, two or three members as defined above;

(4) optionally-substituted pyridin-2-yl, pyridin-3-yl, pyridin-4-yl, pyrimidin-5-yl, pyrazin-2-yl, pyrimidin-2-yl or -NH-phenyl and any substituents are one, two or three members as defined above;

(5) 4-[di($C_1$-$C_5$)alkoxyphosphonomethyl]phenyl; or

(6) 4-(pyridin-4-ylmethyl)phenyl optionally-substituted in the pyridinyl moiety with one or two members as defined above; and

R is hydrogen, phenyl, Cl, Br, F, CN, $CF_3$, $C_1$-$C_5$ alkyl or $C_1$-$C_5$ alkoxy;

with the provisos that, when X is 4-halophenyl, R is Cl, F, Br, $CF_3$ or phenyl, and that the compound of formula I is other than:

3-[N-(5-benzyl-1,3,4-thiadiazol-2-yl)carbamoyl]-7-chloro-4-hydroxycoumarin,
7-chloro-4-hydroxy-3-[N-[5-(2-pyridinylmethyl)thio-1,3,4-thiadiazol-5-yl]carbamoylcoumarin,
7-chloro-4-hydroxy-3-[N-(5-phenyl-1,3,4-thiadiazol-2-yl]-carbamoylcoumarin,
3-[N-(2-benzylthio-1,3,4-thiadiazol-5-yl)carbamoyl]-7-chloro-4-hydroxycoumarin,
4-hydroxy-3-[N-(2-pyridinylcarbamoyl)]coumarin,
4-hydroxy-3-[N-(3-pyridinylcarbamoyl)]coumarin,
7-trifluoromethyl-3-[N-(3-chloro-5-trlfluoromethyl-pyridin-2-yl)carbamoyl]-4-hydroxycoumarin,
3-[N-(6-chloropyridin-3-yl)carbamoyl]-4-hydroxycoumarin,
3-[N-(6-methylpyridin-2-yl)carbamoyl]-4-hydroxycoumarin,
3-[N-(5-bromopyridin-2-yl)carbamoyl]-7-methoxy-4-hydroxycoumarin,
3-[N-(2,6-dichloropyridin-4-yl)carbamoyl]-4-hydroxycoumarin, or
3-[N-(4-trifluoromethylphenyl)carbamoyl]-4-hydroxycoumarin,

which comprises refluxing a corresponding compound of the formula

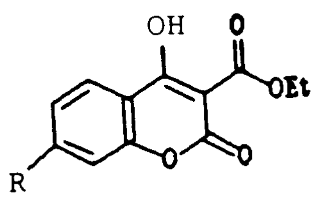

with a corresponding arylamine of the formula $XNH_2$.

2. A process for preparing a compound of formula I as defined in claim 1, or a hydrate or pharmaceutically-acceptable salt thereof, which comprises reacting a corresponding compound of the formula

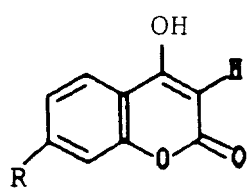

with a corresponding arylisocyanate of the formula XNCO, in the presence of a base.

3. A process according to claim 1 or claim 2, wherein the product is 7-bromo-3-[N-(4-chlorophenyl)-carbamoyl]-4-hydroxycoumarin or 7-bromo-3-[N-(2,4-diflurophenyl)carbamoyl]-4-hydroxycoumarin, or a hydrate or pharmaceutically-acceptable salt of either.

4. A process according to claim 1 or claim 2, wherein X is 4-halophenyl.

5. A process according to claim 4, wherein the product is:

3-[N-(4-bromophenyl)carbamoyl)-7-chloro-4-hydroxycoumarin;
3-[N-(4-bromophenyl)carbamoyl]-7-trichloromethyl-4-hydroxycoumarin;
3-[N-(4-chlorophenyl)carbamoyl]-7-chloro-4-hydroxycoumarin;
3-[N-(4-chlorophenyl)carbamoyl]-7-trifluoromethyl-4-hydroxycoumarin;
3-[N-(4-chlorophenyl)carbamoyl]-7-fluoro-4-hydroxycoumarin;
3-[N-(4-fluorophenyl)carbamoyl]-7-chloro-4-hydroxycoumarin;

3-[N-(4-fluorophenyl)carbamoyl]-7-trifluoromethyl-4-hydroxycoumarin;
3-[N-(4-fluorophenyl)carbamoyl]-7-fluoro-4-hydroxycoumarin;
or a pharmaceutically-acceptable salt of any of the foregoing compounds.

**6.** A process according to claim 1 or claim 2, wherein X is (5-R'$_5$)-1,3,4-thiadiazol-2-yl.

**7.** A process according to claim 6, wherein R'$_5$ is $CF_3$, Cl, Br, F, I, CN, $C_1$-$C_5$ alkyl, or benzyl or pyridinyl optionally substituted with one member selected from Cl, Br, F, I, -CN and $CF_3$.

**8.** A process according to claim 6, wherein R is Cl, Br or $CF_3$.

**9.** A process according to claim 6, which is:
3-[N-(4-bromophenyl)carbamoyl]-7-chloro-4-hydroxycoumarin (Cpd. #1),
3-[N-(4-bromophenyl)carbamoyl]-7-trifluoromethyl-4-hydroxycoumarin (Cpd. #2),
3-[N-(4-bromophenyl)carbamoyl]-7-fluoro-4-hydroxycoumarin (Cpd. #3),
7-chloro-4-hydroxy-3-[N-[5-(trifluoromethyl)-1,3,4-thiadiazol-2-yl]carbamoyl]coumarin (Cpd. #11),
7-chloro-4-hydroxy-3-[N-[5-(chlorodifluoromethyl)-1,3,4-thiadiazol-2-yl]carbamoyl]coumarin (Cpd. #17),
7-chloro-4-hydroxy-3-[N-[5-(trifluoromethyldifluoro-methyl)-1,3,4-thiadiazol-2-yl]carbamoyl]coumarin (Cpd. #18),
or a pharmaceutically-acceptable salt of any of the foregoing compounds.

**10.** A process according to claim 1 or claim 2, wherein the procuct is:
3-[N-(4-bromophenyl)carbamoyl]-7-chloro-4-hydroxycoumarin (Cpd. #1),
3-[N-(4-bromophenyl)carbamoyl]-7-trifluoromethyl-4-hydroxycoumarin (Cpd. #2),
3-[N-(4-bromophenyl)carbamoyl]-7-fluoro-4-hydroxycoumarin (Cpd. #3),
3-[N-(4-chlorophenyl)carbamoyl]-7-chloro-4-hydroxycoumarin (Cpd. #4),
3-[N-(4-chlorophenyl)carbamoyl)-7-trifluoromethyl-4-hydroxycoumarin (Cpd. #5),
3-[N-(4-chlorophenyl)carbamoyl]-7-fluoro-4-hydroxycoumarin (Cpd. #6),
3-[N-(4-fluorophenyl)carbamoyl]-7-chloro-4-hydroxycoumarin (Cpd. #7),
3-[N-(4-fluorophenyl)carbamoyl]-7-trifluoromethyl-4-hydroxycoumarin (Cpd. #8),
3-[N-(4-fluorophenyl)carbamoyl]-7-fluoro-4-hydroxycoumarin (Cpd. #9),
7-chloro-4-hydroxy-3-[N-[5-(trifluoromethyl)-1,3,4-thiadiazol-2-yl]carbamoyl]coumarin (Cpd. #11),
4-hydroxy-3-[N-[5-(trifluoromethyl)-1,3,4-thiadiazol-2-yl]carbamoyl]coumarin (Cpd. #12),
4-hydroxy-7-methyl-3-[N-[5-(trifluoromethyl)-1,3,4-thiadiazol-2-yl]carbamoyl]coumarin (Cpd. #13),
4-hydroxy-7-methoxy-3-[N-[5-(trifluoromethyl)-1,3,4-thiadiazol-2-yl]carbamoyl]coumarin (Cpd. #14),
7-fluoro-4-hydroxy-3-[N-[5-(trifluoromethyl)-1,3,4-thiadiazol-2-yl]carbamoyl]coumarin (Cpd. #15),
7-trifluoromethyl-4-hydroxy-3-[N-[5-(trifluoromethyl)-1,3,4-thiadiazol-2-yl]carbamoyl]coumarin (Cpd. #16),
7-chloro-4-hydroxy-3-[N-[5-(chlorodifluoromethyl)-1,3,4-thiadiazol-2-yl]carbamoyl]coumarin (Cpd. #17),
7-chloro-4-hydroxy-3-[N-[5-(trifluoromethyldifluoromethyl)-1,3,4-thiadiazol-2-yl]carbamoyl]coumarin (Cpd. #18),
4-hydroxy-3-[N-[5-(trifluoromethyldifluoromethyl)-1,3,4-thiadiazol-2-yl]carbamoyl]coumarin (Cpd. #19),
7-fluoro-4-hydroxy-3-[N-[5-(trifluoromethyldifluoromethyl)-1,3,4-thiadiazol-2-yl]carbamoyl]coumarin (Cpd. #20),
4-hydroxy-3-[N-(5-phenyl-1,3,4-thiadiazol-2-yl)carbamoyl]coumarin (Cpd. #21),
3-[N-(5-benzyl-1,3,4-thiadiazol-2-yl)carbamoyl]-4-hydroxycoumarin (Cpd. #23),
3-[N-(5-benzyl-1,3,4-thiadiazol-2-yl)carbamoyl]-4-hydroxy-7-methylcoumarin (Cpd. #24),
3-[N-[5-(4-chlorophenyl)methyl-1,3,4-thiadiazol-2-yl]carbamoyl]-4-hydroxycoumarin (Cpd. #25),
7-chloro-3-[N-[5-(4-chlorophenyl)methyl-1,3,4-thiadiazol-2-yl]carbamoyl]-4-hydroxycoumarin (Cpd. #26),
3-[N-[2-[(4-fluorophenyl)methyl]thio-1,3,4-thiadiazol-5-yl]carbamoyl]-4-hydroxycoumarin (Cpd. #27),
4-hydroxy-3-[N-(2-mercapto-1,3,4-thiadiazol-5-yl)carbamoyl]coumarin (Cpd. #28),
4-hydroxy-3-[N-[2-(methylthio)-1,3,4-thiadiazol-5-yl]carbamoyl]coumarin (Cpd. #29),
4-hydroxy-3-[N-[5-(3,5-dichloropyridin-2-yl)thio-1,3,4-thiadiazol-2-yl]carbamoyl]coumarin (Cpd. #30),
4-hydroxy-3-[N-[2-(2-pyridinylmethyl)thio-1,3,4-thiadiazol-5-yl]carbamoyl]coumarin (Cpd. #31),

4-hydroxy-7-methyl-3-[N-[2-(2-pyridinylmethyl)thio-1,3,4-thiadiazol-5-yl]carbamoyl]coumarin (Cpd. #32),
3-[N-[2-(4-pyridinylmethyl)thio-1,3,4-thiadiazol-5-yl]carbamoyl]4-hydroxycoumarin (Cpd. #33),
3-[N-[2-(benzylthio)-1,3,4-thiadiazol-5-yl]carbamoyl]-4-hydroxycoumarin (Cpd. #34),
3-[N-(5-bromopyrimidin-2-yl)carbamoyl]-4-hydroxycoumarin (Cpd. #35),
4-hydroxy-3-[N-(phenylamino)carbamoyl]coumarin (Cpd. #36),
4-hydroxy-3-[N-[(4-bromophenyl)amino]carbamoyl]coumarin (Cpd. #37),
4-hydroxy-3-[N-(2-bromo-1,3,4-thiadiazol-5-yl)carbamoyl]coumarin (Cpd. #38),
4-hydroxy-3-[N-(2-chloro-1,3,4-thiadiazol-5-yl)carbamoyl]coumarin (Cpd. #39),
3-[N-(4-chloro-6-methylpyrimidin-2-yl)carbamoyl]-4-hydroxycoumarin (Cpd. #40),
3-[N-(6-chloropyrazin-2-yl)carbamoyl]-4-hydroxycoumarin (Cpd. #41),
3-[N-(4,6-dichloropyrimidin-5-yl)carbamoyl]-4-hydroxycoumarin (Cpd. #42),
4-hydroxy-3-[N-[5-(2-fluorophenyl)-1,3,4-thiadiazol-2-yl]carbamoyl]coumarin (Cpd. #44),
7-phenyl-4-hydroxy-3-[N-[5-(trifluoromethyl)-1,3,4-thiadiazol-2-yl]caramoyl]coumarin (Cpd. #45),
3-[N-(4-bromophenyl)carbamoyl]-7-phenyl-4-hydroxycoumarin (Cpd. #46),
3-[N-(5-bromopyridin-2-yl)carbamoyl]-4-hydroxycoumarin (Cpd. #47),
3-[N-(2-pyridinyl)carbamoyl]-4-hydroxycoumarin (Cpd. #48),
3-[N-(5-chloropyridin-2-yl)carbamoyl]-4-hydroxycoumarin (Cpd. #49),
3-[N-(2-chloropyridin-3-yl)carbamoyl]-4-hydroxycoumarin (Cpd. #50),
3-[N-(5-methylpyridin-2-yl)carbamoyl]-4-hydroxycoumarin (Cpd. #51),
3-[N-(6-methoxypyridin-3-yl)carbamoyl]-4-hydroxycoumarin (Cpd. #52),
7-trifluoromethyl-3-[N-[4-(2,4-difluoro)phenyl]]-4-hydroxycoumarin (Cpd. #53),
7-bromo-3-[N-[4-chlorophenyl)carbamoyl]-4-hydroxycoumarin (Cpd. #54),
3-[N-(3,5-dichloropyridin-2-yl)carbamoyl]-4-hydroxycoumarin (Cpd. #55),
3-[N-(5-bromopyridin-2-yl)carbamoyl]-7-chloro-4-hydroxycoumarin (Cpd. #56),
7-bromo-3-[N-[4-(2,4-difluoro)phenyl]carbamoyl]-4-hydroxycoumarin (Cpd. #58),
3-[N-[5-(1,1-dimethylethyl)-1,3,4-thiadiazol-2-yl)carbamoyl]-4-hydroxycoumarin (Cpd. #60),
3-[N-(5-bromopyridin-2-yl)carbamoyl]-7-methyl-4-hydroxycoumarin (Cpd. #61),
7-bromo-3-[N-(4-fluorophenyl)carbamoyl]-4-hydroxycoumarin (Cpd. #62),
3-[N-(2,3,5,6-tetrafluoropyridin-4-yl)carbamoyl]-6-methoxy-4-hydroxycoumarin (Cpd. #63),
7-chloro-3-[N-(5-chloropyridin-2-yl)carbamoyl]-4-hydroxycoumarin (Cpd. #64),
7-chloro-3-[N-(5-chloropyridin-2-yl)carbamoyl]-4-hydroxycoumarin, triethylamine salt (Cpd. #65),
7-fluoro-3-[N-(5-bromopyridin-2-yl)carbamoyl]-4-hydroxycoumarin (Cpd. #67),
7-chloro-3-[N-(3-chloro-5-trifluoromethylpyridin-2-yl)carbamoyl]-4-hydroxycoumarin (Cpd. #68),
3-[N-(3-chloro-5-trifluoromethylpyridin-2-yl)carbamoyl]-4-hydroxycoumarin (Cpd. #69),
3-[N-(3,5-ditrifluoromethylpyridin-2-yl)carbamoyl]-4-hydroxycoumarin (Cpd. #70),
7-trifluoromethyl-3-[N-(5-bromopyridin-2-yl]carbamoyl]-4-hydroxycoumarin (Cpd. #71),
7-trifluoromethyl-3-[N-(5-chloropyridin-2-yl]carbamoyl]-4-hydroxycoumarin (Cpd. #72),
7-trifluoromethyl-3-[N-(2-chloropyridin-3-yl]carbamoyl]-4-hydroxycoumarin (Cpd. #73),
7-trifluoromethyl-3-[N-(6-chloropyridin-3-yl]carbamoyl]-4-hydroxycoumarin (Cpd. #74),
7-chloro-3-[N-(6-methoxypyridin-3-yl]carbamoyl]-4-hydroxycoumarin (Cpd. #75),
7-trifluoromethyl-3-[N-(6-methoxypyridin-3-yl]carbamoyl]-4-hydroxycoumarin (Cpd. #76),
7-chloro-3-[N-(2-chloropyridin-3-yl]carbamoyl]-4-hydroxycoumarin (Cpd. #77),
7-chloro-3-[N-(6-chloropyridin-3-yl]carbamoyl)-4-hydroxycoumarin (Cpd. #78),
7-chloro-3-[N-(3,5-ditrifluoromethylpyridin-2-yl]carbamoyl]-4-hydroxycoumarin (Cpd. #79),
7-trifluoromethyl-3-[N-(2,3,5,6-tetrafluoropyridin-4-yl]carbamoyl]-4-hydroxycoumarin (Cpd. #80),
7-chloro-4-hydroxy-3-[N-(1,1,2,2,3,3,3-heptafluoropropyl)-1,2,4-thiadiazol-2-yl]carbamoyl]coumarin (Cpd. #81),
7-bromo-3-[N-[4-bromophenyl)carbamoyl]-4-hydroxycoumarin (Cpd. #91),
or a pharmaceutically-acceptable salt of any of the foregoing compounds.

**11.** A process according to claim 1 or claim 2, wherein the product is Cpd. #6, 8, 11, 17 or 18 as defined in claim 10, or a pharmaceutically-acceptable salt thereof.

**12.** A method for preparing a pharmaceutical composition, which comprises compounding a compound of formula I as defined in any preceding claim, or a hydrate or pharmaceutically-acceptable salt thereof, with an inert ingredient.

13. Use of a compound of formula I as defined in any of claims 1 to 11, or a hydrate or pharmaceutically-acceptable salt thereof, for the manufacture of a medicament for use in killing parasitic worms in humans and valuable warm-blooded domestic animals.

14. Use of a compound of formula IW or a hydrate or pharmaceutically-acceptable salt thereof, for the manufacture of a medicament for use in preventing or treating coccidiosis in fowl, wherein formula IW is

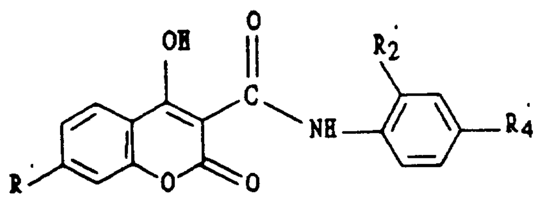

IW

wherein $R'_2$ is hydrogen, $CF_3$, Cl, Br, F or I; $R'_4$ is Br, F, Cl or $CF_3$; and R' is Br, F or $CF_3$; provided that the compound is not 3-[N-(4-chlorophenyl)carbamoyl]-7-trifluoromethyl-4-hydroxycoumarin.

## Patentansprüche

## Patentansprüche für folgende Vertragsstaaten : AT, BE, CH, DE, DK, FR, GB, GR, IT, LU, NL, SE

1. Verwendung einer Verbindung der Formel I oder eines Hydrats oder pharmazeutisch akzeptablen Salzes derselben zur Herstellung eines Medikaments zur Verwendung bei der Abtötung parasitischer Würmer bei Menschen und wertvollen, warmblütigen Haustieren, wobei die Formel I folgende ist:

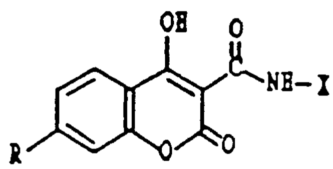

I

worin bedeuten:

X

(1) 4-Halophenyl, gegebenenfalls substituiert in 2-Stellung durch Cl, Br, F oder I;

(2) (5-$R'_5$)-1,3,4-Thiadiazol-2-yl, wobei $R'_5$ für Cl, Br, F, I, Cyano, $-CF_3$, $-CF_2Cl$, $-CF_2CF_3$ oder gegebenenfalls substituiertes Benzyl, Pyridin-2-yl, Pyridin-3-yl oder Pyridin-4-yl steht, wobei irgendwelche Substituenten aus einem, zwei oder drei Glied(ern), nämlich Cl, Br, F, I, CN, $CF_3$, $C_1$-$C_5$-Alkyl und $C_1$-$C_5$-Alkoxy, ausgewählt sind;

(3) (2-$SR''_5$)-1,3,4-Thiadiazol-5-yl, wobei $R''_5$ für Wasserstoff, $C_1$-$C_5$-Alkyl, Cl, Br, F, I, Cyano, $-CF_3$, $-CF_2Cl$, $-CF_2CF_3$ oder gegebenenfalls substituiertes Phenyl, Benzyl, Pyridin-2-yl, Pyridin-3-yl, Pyridin-4-yl, Pyridin-2-yl($CH_2$)-, Pyridin-3-yl($CH_2$)- oder Pyridin-4-yl($CH_2$)- steht, wobei irgendwelche Substituenten aus einem, zwei oder drei Glied(ern) der zuvor angegebenen Definition ausgewählt sind;

(4) gegebenenfalls substituiertes Pyridin-2-yl, Pyridin-3-yl, Pyridin-4-yl, Pyrimidin-5-yl, Pyrazin-2-yl, Pyrimidin-2-yl oder -NH-Phenyl, wobei irgendwelche Substituenten aus einem, zwei oder drei Glied(ern) der zuvor angegebenen Definition ausgewählt sind;

(5) 4-[Di($C_1$-$C_5$)alkoxyphosphonomethyl]phenyl; oder

(6) 4-(Pyridin-4-ylmethyl)phenyl, gegebenenfalls substituiert in der Pyridineinheit mit einem oder zwei Glied(ern) der zuvor angegebenen Definition und

R → Wasserstoff, Phenyl, Cl, Br, F, CN, $CF_3$, $C_1$-$C_5$-Alkyl oder $C_1$-$C_5$-Alkoxy;

wobei gilt, daß im Falle, daß X für 4-Halophenyl steht, R → Cl, F, Br, $CF_3$ oder Phenyl darstellt und daß die Verbindung der Formel I verschieden ist von:

3-[N-(5-Benzyl-1,3,4-thiadiazol-2-yl)carbamoyl]-7-chlor-4-hydroxycumarin,

7-Chlor-4-hydroxy-3-[N-[5-(2-pyridinylmethyl)thio-1,3,4-thiadiazol-5-yl]carbamoylcumarin,

7-Chlor-4-hydroxy-3-[N-(5-phenyl-1,3,4-thiadiazol-2-yl]carbamoylcumarin,
3-[N-(2-Benzylthio-1,3,4-thiadiazol-5-yl)carbamoyl]-7-chlor-4-hydroxycumarin,
4-Hydroxy-3-[N-(2-pyridinylcarbamoyl)]cumarin,
4-Hydroxy-3-[N-(3-pyridinylcarbamoyl)]cumarin,
7-Trifluormethyl-3-[N-(3-chlor-5-trifluormethyl-pyridin-2-yl)carbamoyl]-4-hydroxycumarin,
3-[N-(6-Chlorpyridin-3-yl)carbamoyl]-4-hydroxycumarin,
3-[N-(6-Methylpyridin-2-yl)carbamoyl]-4-hydroxycumarin,
3-[N-(5-Brompyridin-2-yl)carbamoyl]-7-methoxy-4-hydroxycumarin oder
3-[N-(2,6-Dichlorpyridin-4-yl)carbamoyl]-4-hydroxycumarin.

**2.** Verbindung der Formel IW oder ein Hydrat oder pharmazeutisch akzeptables Salz derselben, wobei die Formel IW folgende ist:

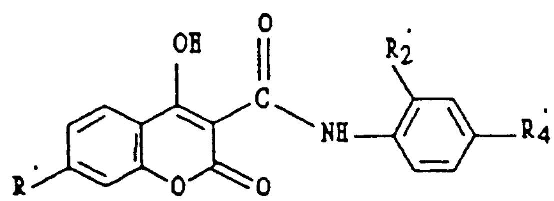

IW

worin $R'_2$ für Wasserstoff, $CF_3$, Cl, Br, F oder I steht, und $R'_4$ → Br, F, Cl oder $CF_3$ darstellt und R' Br, F oder $CF_3$ bedeutet, wobei gilt, daß es sich bei der Verbindung nicht um 3-[N-(4-Chlorphenyl)-carbamoyl]-7-trifluormethyl-4-hydroxycumarin handelt.

**3.** Verbindung der Formel I gemäß Anspruch 1, oder ein Hydrat oder pharmazeutisch akzeptables Salz derselben, die von 3-[N-(4-Trifluormethylphenyl)carbamoyl]-4-hydroxycumarin verschieden ist.

**4.** Verbindung nach Anspruch 3, wobei X für 4-Halophenyl steht.

**5.** Verbindung nach Anspruch 4, nämlich
3-[N-(4-Bromphenyl)carbamoyl]-7-chlor-4-hydroxycumarin,
3-[N-(4-Bromphenyl)carbamoyl]-7-trichlormethyl-4-hydroxycumarin,
3-[N-(4-Chlorphenyl)carbamoyl]-7-chlor-4-hydroxycumarin,
3-[N-(4-Chlorphenyl)carbamoyl]-7-trifluormethyl-4-hydroxycumarin,
3-[N-(4-Chlorphenyl)carbamoyl]-7-fluor-4-hydroxycumarin,
3-[N-(4-Fluorphenyl)carbamoyl]-7-chlor-4-hydroxycumarin,
3-[N-(4-Fluorphenyl)carbamoyl]-7-trifluormethyl-4-hydroxycumarin,
3-[N-(4-Fluorphenyl)carbamoyl]-7-fluor-4-hydroxycumarin,
oder ein pharmazeutisch akzeptables Salz einer der vorhergehenden Verbindungen.

**6.** Verbindung nach Anspruch 3, wobei X für 5(-$R'_5$)-1,3,4-Thiadiazol-2-yl steht.

**7.** Verbindung nach Anspruch 6, wobei $R'_5$ für $CF_3$, Cl, Br, F, I, CN, $C_1$-$C_5$-Alkyl oder Benzyl oder Pyridinyl, gegebenenfalls einfach substituiert durch Cl, Br, F, I, -CN und $CF_3$, steht.

**8.** Verbindung nach Anspruch 6, wobei R für Cl, Br oder $CF_3$ steht.

**9.** Verbindung nach Anspruch 6, nämlich
3-[N-(4-Bromphenyl)carbamoyl]-7-chlor-4-hydroxycumarin (Verbindung Nr. 1),
3-[N-(4-Bromphenyl)carbamoyl]-7-trifluormethyl-4-hydroxycumarin(Verbindung Nr. 2),
3-[N-(4-Bromphenyl)carbamoyl]-7-fluor-4-hydroxycumarin (Verbindung Nr. 3),
7-Chlor-4-hydroxy-3-[N-[5-(trifluormethyl)-1,3,4-thiadiazol-2-yl]carbamoyl]cumarin (Verbindung Nr. 11),
7-Chlor-4-hydroxy-3-[N-[5-(chlordifluormethyl)-1,3,4-thiadiazol-2-yl]carbamoyl]cumarin (Verbindung Nr. 17),

7-Chlor-4-hydroxy-3-[N-[5-(trifluormethyldifluormethyl)-1,3,4-thiadiazol-2-yl]carbamoyl]cumarin (Verbindung Nr. 18),
oder ein pharmazeutisch akzeptables Salz irgendeiner der vorhergehenden Verbindungen.

10. Verbindung nach Anspruch 3, nämlich
3-[N-(4-Bromphenyl)carbamoyl]-7-chlor-4-hydroxycumarin (Verbindung Nr. 1),
3-[N-(4-Bromphenyl)carbamoyl]-7-trifluormethyl-4-hydroxycumarin(Verbindung Nr. 2),
3-[N-(4-Bromphenyl)carbamoyl]-7-fluor-4-hydroxycumarin (Verbindung Nr. 3),
3-[N-(4-Chlorphenyl)carbamoyl]-7-chlor-4-hydroxycumarin (Verbindung Nr. 4),
3-[N-(4-Chlorphenyl)carbamoyl]-7-trifluormethyl-4-hydroxycumarin (Verbindung Nr. 5),
3-[N-(4-Chlorphenyl)carbamoyl]-7-fluor-4-hydroxycumarin (Verbindung Nr. 6),
3-[N-(4-Fluorphenyl)carbamoyl]-7-chlor-4-hydroxycumarin (Verbindung Nr. 7),
3-[N-(4-Fluorphenyl)carbamoyl]-7-trifluormethyl-4-hydroxycumarin (Verbindung Nr. 8),
3-[N-(4-Fluorphenyl)carbamoyl]-7-fluor-4-hydroxycumarin (Verbindung Nr. 9),
7-Chlor-4-hydroxy-3-[N-[5-(trifluormethyl)-1,3,4-thiadiazol-2-yl]carbamoyl]cumarin (Verbindung Nr. 11),
4-Hydroxy-3-[N-[5-(trifluormethyl)-1,3,4-thiadiazol-2-yl]-carbamoyl]cumarin (Verbindung Nr. 12),
4-Hydroxy-7-methyl-3-[N-[5-(trifluormethyl)-1,3,4-thiadiazol-2-yl]carbamoyl]cumarin (Verbindung Nr. 13),
4-Hydroxy-7-methoxy-3-[N-[5-(trifluormethyl)-1,3,4-thiadiazol-2-yl]carbamoyl]cumarin (Verbindung Nr. 14),
7-Fluor-4-hydroxy-3-[N-[5-(trifluormethyl)-1,3,4-thidiazol-2-yl]carbamoyl]cumarin (Verbindung Nr. 15),
7-Trifluormethyl-4-hydroxy-3-[N-[5-(trifluormethyl)-1,3,4-thiadiazol-2-yl]carbamoyl]cumarin (Verbindung Nr. 16),
7-Chlor-4-hydroxy-3-[N-[5-(chlordifluormethyl)-1,3,4-thiadiazol-2-yl]carbamoyl]cumarin (Verbindung Nr. 17),
7-Chlor-4-hydroxy-3-[N-[5-(trifluormethyldifluormethyl)-1,3,4-thiadiazol-2-yl]carbamoyl]cumarin (Verbindung Nr. 18),
4-Hydroxy-3-[N-[5-(trifluormethyldifluormethyl)-1,3,4-thiadiazol-2-yl]carbamoyl]cumarin (Verbindung Nr. 19),
7-Fluor-4-hydroxy-3-[N-[5-(trifluormethyldifluormethyl)-1,3,4-thiadiazol-2-yl]carbamoyl]cumarin (Verbindung Nr. 20),
4-Hydroxy-3-[N-(5-phenyl-1,3,4-thiadiazol-2-yl)carbamoyl]cumarin (Verbindung Nr. 21),
3-[N-(5-Benzyl-1,3,4-thiadiazol-2-yl)carbamoyl]-4-hydroxycumarin (Verbindung Nr. 23),
3-[N-(5-Benzyl-1,3,4-thiadiazol-2-yl)carbamoyl]-4-hydroxy-7-methylcumarin (Verbindung Nr. 24),
3-[N-[5-(4-Chlorphenyl)methyl-1,3,4-thiadiazol-2-yl]carbamoyl]-4-hydroxycumarin (Verbindung Nr. 25),
7-Chlor-3-[N-[5-(4-chlorphenyl)methyl-1,3,4-thiadiazol-2-yl]carbamoyl]-4-hydroxycumarin (Verbindung Nr. 26),
3-[N-[2-[(4-Fluorphenyl)methyl]thio-1,3,4-thiadiazol-5-yl]carbamoyl]-4-hydroxycumarin (Verbindung Nr. 27),
4-Hydroxy-3-[N-(2-mercapto-1,3,4-thiadiazol-5-yl)carbamoyl]cumarin (Verbindung Nr. 28),
4-Hydroxy-3-[N-[2-(methylthio)-1,3,4-thiadiazol-5-yl]carbamoyl]cumarin (Verbindung Nr. 29),
4-Hydroxy-3-[N-[5-(3,5-dichlorpyridin-2-yl)thio-1,3,4-thiadiazol-2-yl]carbamoyl]cumarin (Verbindung Nr. 30),
4-Hydroxy-3-[N-[2-(2-pyridinylmethyl)thio-1,3,4-thiadiazol-5-yl]carbamoyl]cumarin (Verbindung Nr. 31),
4-Hydroxy-7-methyl-3-[N-[2-(2-pyridinylmethyl)thio-1,3,4-thiadiazol-5-yl]carbamoyl]cumarin (Verbindung Nr. 32),
3-[N-[2-(4-Pyridinylmethyl)thio-1,3,4-thiadiazol-5-yl]carbamoyl]4-hydroxycumarin (Verbindung Nr. 33),
3-[N-[2-(Benzylthio)-1,3,4-thiadiazol-5-yl]carbamoyl]-4-hydroxycumarin (Verbindung Nr. 34),
3-[N-(5-Brompyrimidin-2-yl)carbamoyl]-4-hydroxycumarin (Verbindung Nr. 35),
4-Hydroxy-3-[N-(phenylamino)carbamoyl]cumarin (Verbindung Nr. 36),
4-Hydroxy-3-[N-[(4-bromphenyl)amino]carbamoyl]cumarin (Verbindung Nr. 37),
4-Hydroxy-3-[N-(2-brom-1,3,4-thiadiazol-5-yl)carbamoyl]cumarin (Verbindung Nr. 38)
4-Hydroxy-3-[N-(2-chlor-1,3,4-thiadiazol-5-yl)carbamoyl]cumarin (Verbindung Nr. 39),

3-[N-(4-Chlor-6-methylpyrimidin-2-yl)carbamoyl]-4-hydroxycumarin (Verbindung Nr. 40),
3-[N-(6-Chlorpyrazin-2-yl)carbamoyl]-4-hydroxycumarin (Verbindung Nr. 41),
3-[N-(4,6-Dichlorpyrimidin-5-yl)carbamoyl]-4-hydroxycumarin (Verbindung Nr. 42),
4-Hydroxy-3-[N-[5-(2-fluorphenyl)-1,3,4-thiadiazol-2-yl]carbamoyl]cumarin (Verbindung Nr. 44),
7-Phenyl-4-hydroxy-3-[N-[5-(trifluormethyl)-1,3,4-thiadiazol-2-yl]carbamoyl]cumarin (Verbindung Nr. 45),
3-[N-(4-Bromphenyl)carbamoyl]-7-phenyl-4-hydroxycumarin (Verbindung Nr. 46),
3-[N-(5-Brompyridin-2-yl)carbamoyl]-4-hydroxycumarin (Verbindung Nr. 47),
3-[N-(2-Pyridinyl)carbamoyl]-4-hydroxycumarin (Verbindung Nr. 48),
3-[N-(5-Chlorpyridin-2-yl)carbamoyl]-4-hydroxycumarin (Verbindung Nr. 49),
3-[N-(2-Chlorpyridin-3-yl)carbamoyl]-4-hydroxycumarin (Verbindung Nr. 50),
3-[N-(5-Methylpyridin-2-yl)carbamoyl]-4-hydroxycumarin (Verbindung Nr. 51),
3-[N-(6-Methoxypyridin-3-yl)carbamoyl]-4-hydroxycumarin (Verbindung Nr. 52),
7-Trifluormethyl-3-[N-[4-(2,4-difluor)phenyl]]-4-hydroxycumarin (Verbindung Nr. 53),
7-Brom-3-[N-(4-chlorphenyl)carbamoyl]-4-hydroxycumarin (Verbindung Nr. 54),
3-[N-(3,5-Dichlorpyridin-2-yl)carbamoyl]-4-hydroxycumarin (Verbindung Nr. 55),
3-[N-(5-Brompyridin-2-yl)carbamoyl]-7-chlor-4-hydroxycumarin (Verbindung Nr. 56),
7-Brom-3-[N-[4-(2,4-Difluor)phenyl]carbamoyl)-4-hydroxycumarin (Verbindung Nr. 58),
3-[N-[5-(1,1-Dimethylethyl)-1,3,4-thiadiazol-2-yl]carbamoyl]-4-hydroxycumarin (Verbindung Nr. 60),
3-[N-(5-Brompyridin-2-yl)carbamoyl]-7-methyl-4-hydroxycumarin (Verbindung Nr. 61),
7-Brom-3-[N-(4-fluorphenyl)carbamoyl]-4-hydroxycumarin (Verbindung Nr. 62),
3-[N-(2,3,5,6-Tetrafluorpyridin-4-yl)carbamoyl]-6-methoxy-4-hydroxycumarin (Verbindung Nr. 63),
7-Chlor-3-[N-(5-chlorpyridin-2-yl)carbamoyl]-4-hydroxycumarin (Verbindung Nr. 64),
7-Chlor-3-[N-(5-chlorpyridin-2-yl)carbamoyl]-4-hydroxycumarin, Triethylaminsalz (Verbindung Nr. 65),
7-Fluor-3-[N-(5-brompyridin-2-yl)carbamoyl]-4-hydroxycumarin (Verbindung Nr. 67),
7-Chlor-3-[N-(3-chlor-5-trifluormethylpyridin-2-yl)carbamoyl]-4-hydroxycumarin (Verbindung Nr. 68),
3-[N-(3-Chlor-5-trifluormethylpyridin-2-yl)carbamoyl]-4-hydroxycumarin (Verbindung Nr. 69),
3-[N-(3,5-Ditrifluormethylpyridin-2-yl)carbamoyl]-4-hydroxycumarin (Verbindung Nr. 70),
7-Trifluormethyl-3-[N-(5-brompyridin-2-yl)carbamoyl]-4-hydroxycumarin (Verbindung Nr. 71),
7-Trifluormethyl-3-[N-(5-chlorpyridin-2-yl)carbamoyl]-4-hydroxycumarin (Verbindung Nr. 72),
7-Trifluormethyl-3-[N-(2-chlorpyridin-2-yl)carbamoyl]-4-hydroxycumarin (Verbindung Nr. 73),
7-Trifluormethyl-3-[N-(6-chlorpyridin-3-yl)carbamoyl]-4-hydroxycumarin (Verbindung Nr. 74),
7-Chlor-3-[N-(6-methoxypyridin-3-yl)carbamoyl]-4-hydroxycumarin (Verbindung Nr. 75),
7-Trifluormethyl-3-[N-(6-methoxypyridin-3-yl)carbamoyl]-4-hydroxycumarin (Verbindung Nr. 76),
7-Chlor-3-[N-(2-chlorpyridin-3-yl)carbamoyl]-4-hydroxycumarin (Verbindung Nr. 77),
7-Chlor-3-[N-(6-chlorpyridin-3-yl)carbamoyl]-4-hydroxycumarin (Verbindung Nr. 78),
7-Chlor-3-[N-(3,5-ditrifluormethylpyridin-2-yl)carbamoyl]-4-hydroxycumarin (Verbindung Nr. 79),
7-Trifluormethyl-3-[N-(2,3,5,6-tetrafluorpyridin-4-yl)carbamoyl]-4-hydroxycumarin (Verbindung Nr. 80),
7-Chlor-4-hydroxy-3-[N-[(1,1,2,2,3,3,3-heptafluorpropyl)-1,2,4-thiadiazol-2-yl]carbamoyl]cumarin (Verbindung Nr. 81),
7-Brom-3-[N-(4-bromphenyl)carbamoyl]-4-hydroxycumarin (Verbindung Nr. 91),
oder ein pharmazeutisch akzeptables Salz einer der vorhergehenden Verbindungen.

**11.** Verbindung nach Anspruch 3, bei der es sich um die Verbindungen Nr. 6, 8, 11, 17 oder 18 entsprechend Anspruch 10 oder ein pharmazeutisch akzeptables Salz derselben handelt.

**12.** Verbindung nach Anspruch 3, nämlich 7-Brom-3-[N-(4-chlorphenyl)carbamoyl]-4-hydroxycumarin oder 7-Brom-3-[N-(2,4-difluorphenyl)carbamoyl]-4-hydroxycumarin oder ein Hydrat oder pharmazeutisch akzeptables Salz eines der beiden.

**13.** Verwendung nach Anspruch 1, wobei die Verbindung unter die Definition nach einem der Ansprüche 3 bis 12 fällt.

**14.** Verwendung einer Verbindung nach Anspruch 2, zur Herstellung eines Medikaments zur Verwendung bei der Verhinderung oder Behandlung von Kokzidiose bei Geflügel.

**Patentansprüche für folgenden Vertragsstaat : ES**

1. Verfahren zur Herstellung einer Verbindung der Formel I oder eines Hydrats oder pharmazeutisch akzeptablen Salzes derselben, wobei die Formel I folgende ist:

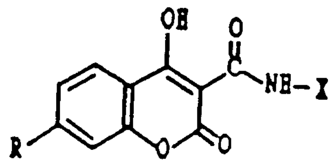

I

worin bedeuten:

X

(1) 4-Halophenyl, gegebenenfalls substituiert in 2-Stellung durch Cl, Br, F oder I;

(2) (5-$R'_5$)-1,3,4-Thiadiazol-2-yl, wobei $R'_5$ für Cl, Br, F, I, Cyano, $-CF_3$, $-CF_2Cl$, $-CF_2CF_3$ oder gegebenenfalls substituiertes Benzyl, Pyridin-2-yl, Pyridin-3-yl oder Pyridin-4-yl steht, wobei irgendwelche Substituenten aus einem, zwei oder drei Glied(ern), nämlich Cl, Br, F, I, CN, $CF_3$, $C_1$-$C_5$-Alkyl und $C_1$-$C_5$-Alkoxy, ausgewählt sind.

(3) (2-SR''$_5$)-1,3,4-Thiadiazol-5-yl, wobei $R''_5$ für Wasserstoff, $C_1$-$C_5$-Alkyl, Cl, Br, F, I, Cyano, $-CF_3$, $-CF_2Cl$, $-CF_2CF_3$ oder gegebenenfalls substituiertes Phenyl, Benzyl, Pyridin-2-yl, Pyridin-3-yl, Pyridin-4-yl, Pyridin-2-yl($CH_2$)-, Pyridin-3-yl($CH_2$)- oder Pyridin-4-yl($CH_2$)- steht, wobei irgendwelche Substituenten aus einem, zwei oder drei Glied(ern) der zuvor angegebenen Definition ausgewählt sind;

(4) gegebenenfalls substituiertes Pyridin-2-yl, Pyridin-3-yl, Pyridin-4-yl, Pyrimidin-5-yl, Pyrazin-2-yl, Pyrimidin-2-yl oder -NH-Phenyl, wobei irgendwelche Substituenten aus einem, zwei oder drei Glied(ern) der zuvor angegebenen Definition ausgewählt sind;

(5) 4-[Di($C_1$-$C_5$)alkoxyphosphonomethyl]phenyl; oder

(6) 4-Pyridin-4-ylmethyl)phenyl, gegebenenfalls substituiert in der Pyridineinheit mit einem oder zwei Glied(ern) der zuvor angegebenen Definition und

R → Wasserstoff, Phenyl, Cl, Br, F, CN, $CF_3$, $C_1$-$C_5$-Alkyl oder $C_1$-$C_5$-Alkoxy;

wobei gilt, daß im Falle, daß X für 4-Halophenyl steht, R → Cl, F, Br, $CF_3$ oder Phenyl darstellt und daß die Verbindung der Formel I verschieden ist von:

3-[N-(5-Benzyl-1,3,4-thiadiazol-2-yl)carbamoyl]-7-chlor-4-hydroxycumarin,
7-Chlor-4-hydroxy-3-[N-[5-(2-pyridinylmethyl)thio-1,3,4-thiadiazol-5-yl]carbamoylcumarin,
7-Chlor-4-hydroxy-3-[N-(5-phenyl-1,3,4-thiadiazol-2-yl]carbamoylcumarin,
3-[N-(2-Benzylthio-1,3,4-thiadiazol-5-yl)carbamoyl]-7-chlor-4-hydroxycumarin,
4-Hydroxy-3-[N-(2-pyridinylcarbamoyl)]cumarin,
4-Hydroxy-3-[N-(3-pyridinylcarbamoyl)]cumarin,
7-Trifluormethyl-3-[N-(3-chlor-5-trifluormethyl-pyridin-2-yl)carbamoyl]-4-hydroxycumarin,
3-[N-(6-Chlorpyridin-3-yl)carbamoyl]-4-hydroxycumarin,
3-[N-(6-Methylpyridin-2-yl)carbamoyl]-4-hydroxycumarin,
3-[N-(5-Brompyridin-2-yl)carbamoyl]-7-methoxy-4-hydroxycumarin oder
3-[N-(2,6-Dichlorpyridin-4-yl)carbamoyl]-4-hydroxy cumarin, durch Erwärmen einer entsprechenden Verbindung der Formel

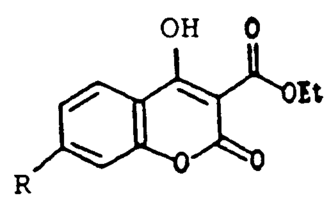

mit einem entsprechenden Arylamin der Formel $XNH_2$ auf Rückflußtemperatur.

2. Verfahren zur Herstellung einer Verbindung der Formel I nach Anspruch 1 oder eines Hydrats oder pharmazeutisch akzeptablen Salzes derselben durch Umsetzen einer entsprechenden Verbindung der Formel:

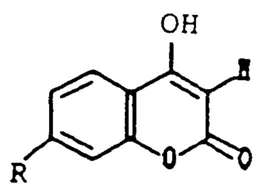

mit einem entsprechenden Arylisocyanat der Formel XNCO in Gegenwart einer Base.

3. Verfahren nach Anspruch 1 oder 2, wobei es sich bei dem Produkt um 7-Brom-3-[N-(4-chlorphenyl)-carbamoyl]-4-hydroxycumarinoder 7-Brom-3-[N-(2,4-difluorphenyl)carbamoyl]-4-hydroxycumarin oder ein Hydrat oder pharmazeutisch akzeptables Salz einer der beiden handelt.

4. Verfahren nach Anspruch 1 oder 2, wobei X für 4-Halophenyl steht.

5. Verfahren nach Anspruch 4, wobei es sich bei dem Produkt um
3-[N-(4-Bromphenyl)carbamoyl]-7-chlor-4-hydroxycumarin,
3-[N-(4-Bromphenyl)carbamoyl]-7-trichlormethyl-4-hydroxycumarin,
3-[N-(4-Chlorphenyl)carbamoyl]-7-chlor-4-hydroxycumarin,
3-[N-(4-Chlorphenyl)carbamoyl]-7-trifluormethyl-4-hydroxycumarin,
3-[N-(4-Chlorphenyl)carbamoyl]-7-fluor-4-hydroxycumarin,
3-[N-(4-Fluorphenyl)carbamoyl]-7-chlor-4-hydroxycumarin,
3-[N-(4-Fluorphenyl)carbamoyl]-7-trifluormethyl-4-hydroxycumarin,
3-[N-(4-Fluorphenyl)carbamoyl]-7-fluor-4-hydroxycumarin,
oder ein pharmazeutisch akzeptables Salz einer der vorhergehenden Verbindungen handelt.

6. Verfahren nach Anspruch 1 oder 2, wobei X für 5(-$R'_5$)-1,3,4-Thiadiazol-2-yl steht.

7. Verfahren nach Anspruch 6, wobei $R'_5$ für $CF_3$, Cl, Br, F, I, CN, $C_1$-$C_5$-Alkyl oder Benzyl oder Pyridinyl, gegebenenfalls einfach substituiert durch Cl, Br, F, I, -CN und $CF_3$, steht.

8. Verfahren nach Anspruch 6, wobei R für Cl, Br oder $CF_3$ steht.

9. Verfahren nach Anspruch 6, wobei es sich bei dem Produkt um
3-[N-(4-Bromphenyl)carbamoyl]-7-chlor-4-hydroxycumarin (Verbindung Nr. 1),
3-[N-(4-Bromphenyl)carbamoyl]-7-trifluormethyl-4-hydroxycumarin(Verbindung Nr. 2),
3-[N-(4-Bromphenyl)carbamoyl]-7-fluor-4-hydroxycumarin (Verbindung Nr. 3),
7-Chlor-4-hydroxy-3-[N-[5-(trifluormethyl)-1,3,4-thiadiazol-2-yl]carbamoyl]cumarin (Verbindung Nr. 11),
7-Chlor-4-hydroxy-3-[N-[5-(chlordifluormethyl)-1,3,4-thiadiazol-2-yl]carbamoyl]cumarin (Verbindung Nr. 17),
7-Chlor-4-hydroxy-3-[N-[5-(trifluormethyldifluormethyl)-1,3,4-thiadiazol-2-yl]carbamoyl]cumarin (Verbindung Nr. 18),
oder ein pharmazeutisch akzeptables Salz eines der vorhergehenden Verbindungen handelt.

10. Verfahren nach Anspruch 1 oder 2, wobei es sich beim Produkt um
3-[N-(4-Bromphenyl)carbamoyl]-7-chlor-4-hydroxycumarin (Verbindung Nr. 1),
3-[N-(4-Bromphenyl)carbamoyl]-7-trifluormethyl-4-hydroxycumarin(Verbindung Nr. 2),
3-[N-(4-Bromphenyl)carbamoyl]-7-fluor-4-hydroxycumarin (Verbindung Nr. 3),

3-[N-(4-Chlorphenyl)carbamoyl]-7-chlor-4-hydroxycumarin (Verbindung Nr. 4),
3-[N-(4-Chlorphenyl)carbamoyl]-7-trifluormethyl-4-hydroxycumarin (Verbindung Nr. 5),
3-[N-(4-Chlorphenyl)carbamoyl]-7-fluor-4-hydroxycumarin (Verbindung Nr. 6),
3-[N-(4-Fluorphenyl)carbamoyl]-7-chlor-4-hydroxycumarin (Verbindung Nr. 7),
3-[N-(4-Fluorphenyl)carbamoyl]-7-trifluormethyl-4-hydroxycumarin (Verbindung Nr. 8),
3-[N-(4-Fluorphenyl)carbamoyl]-7-fluor-4-hydroxycumarin (Verbindung Nr. 9),
7-Chlor-4-hydroxy-3-[N-[5-(trifluormethyl)-1,3,4-thiadiazol-2-yl]carbamoyl]cumarin (Verbindung Nr. 11),
4-Hydroxy-3-[N-[5-(trifluormethyl)-1,3,4-thiadiazol-2-yl]-carbamoyl]cumarin (Verbindung Nr. 12),
4-Hydroxy-7-methyl-3-[N-[5-(trifluormethyl)-1,3,4-thiadiazol-2-yl]carbamoyl]cumarin (Verbindung Nr. 13),
4-Hydroxy-7-methoxy-3-[N-[5-(trifluormethyl)-1,3,4-thiadiazol-2-yl]-carbamoyl]cumarin (Verbindung Nr. 14),
7-Fluor-4-hydroxy-3-[N-[5-(trifluormethyl)-1,3,4-thidiazol-2-yl]-carbamoyl]cumarin (Verbindung Nr. 15),
7-Trifluormethyl-4-hydroxy-3-[N-[5-(trifluormethyl)-1,3,4-thiadiazol-2-yl]carbamoyl]cumarin (Verbindung Nr. 16),
7-Chlor-4-hydroxy-3-[N-[5-(chlordifluormethyl)-1,3,4-thiadiazol-2-yl]carbamoyl]cumarin (Verbindung Nr. 17),
7-Chlor-4-hydroxy-3-[N-[5-(trifluormethyldifluormethyl)-1,3,4-thiadiazol-2-yl]carbamoyl]cumarin (Verbindung Nr. 18),
4-Hydroxy-3-[N-[5-(trifluormethyldifluormethyl)-1,3,4-thiadiazol-2-yl]carbamoyl]cumarin (Verbindung Nr. 19),
7-Fluor-4-hydroxy-3-[N-[5-(trifluormethyldifluormethyl)-1,3,4-thiadiazol-2-yl]carbamoyl]cumarin (Verbindung Nr. 20),
4-Hydroxy-3-[N-(5-phenyl-1,3,4-thiadiazol-2-yl)carbamoyl]cumarin (Verbindung Nr. 21),
3-[N-(5-Benzyl-1,3,4-thiadiazol-2-yl)carbamoyl]-4-hydroxycumarin (Verbindung Nr. 23),
3-[N-(5-Benzyl-1,3,4-thiadiazol-2-yl)carbamoyl]-4-hydroxy-7-methylcumarin (Verbindung Nr. 24),
3-[N-[5-(4-Chlorphenyl)methyl-1,3,4-thiadiazol-2-yl]carbamoyl]-4-hydroxycumarin (Verbindung Nr. 25),
7-Chlor-3-[N-[5-(4-chlorphenyl)methyl-1,3,4-thiadiazol-2-yl]carbamoyl]-4-hydroxycumarin (Verbindung Nr. 26),
3-[N-[2-[(4-Fluorphenyl)methyl]thio-1,3,4-thiadiazol-5-yl]carbamoyl]-4-hydroxycumarin (Verbindung Nr. 27),
4-Hydroxy-3-[N-(2-mercapto-1,3,4-thiadiazol-5-yl)carbamoyl]cumarin (Verbindung Nr. 28),
4-Hydroxy-3-[N-[2-(methylthio)-1,3,4-thiadiazol-5-yl]carbamoyl]cumarin (Verbindung Nr. 29),
4-Hydroxy-3-[N-[5-(3,5-dichlorpyridin-2-yl)thio-1,3,4-thiadiazol-2-yl]carbamoyl]cumarin (Verbindung Nr. 30),
4-Hydroxy-3-[N-[2-(2-pyridinylmethyl)thio-1,3,4-thiadiazol-5-yl]carbamoyl]cumarin (Verbindung Nr. 31),
4-Hydroxy-7-methyl-3-[N-[2-(2-pyridinylmethyl)thio-1,3,4-thiadiazol-5-yl]carbamoyl]cumarin (Verbindung Nr. 32),
3-[N-[2-(4-Pyridinylmethyl)thio-1,3,4-thiadiazol-5-yl]carbamoyl]-4-hydroxycumarin (Verbindung Nr. 33),
3-[N-[2-(Benzylthio)-1,3,4-thiadiazol-5-yl]carbamoyl]-4-hydroxycumarin (Verbindung Nr. 34),
3-[N-(5-Brompyrimidin-2-yl)carbamoyl]-4-hydroxycumarin (Verbindung Nr. 35),
4-Hydroxy-3-[N-(phenylamino)carbamoyl]cumarin (Verbindung Nr. 36),
4-Hydroxy-3-[N-[(4-bromphenyl)amino]carbamoyl]cumarin (Verbindung Nr. 37),
4-Hydroxy-3-[N-(2-brom-1,3,4-thiadiazol-5-yl)carbamoyl]cumarin (Verbindung Nr. 38)
4-Hydroxy-3-[N-(2-chlor-1,3,4-thiadiazol-5-yl)carbamoyl]cumarin (Verbindung Nr. 39),
3-[N-(4-Chlor-6-methylpyrimidin-2-yl)carbamoyl]-4-hydroxycumarin (Verbindung Nr. 40),
3-[N-(6-Chlorpyrazin-2-yl)carbamoyl]-4-hydroxycumarin (Verbindung Nr. 41),
3-[N-(4,6-Dichlorpyrimidin-5-yl)carbamoyl]-4-hydroxycumarin (Verbindung Nr. 42),
4-Hydroxy-3-[N-[5-(2-fluorphenyl)-1,3,4-thiadiazol-2-yl]carbamoyl]cumarin (Verbindung Nr. 44),
7-Phenyl-4-hydroxy-3-[N-[5-(trifluormethyl)-1,3,4-thiadiazol-2-yl]carbamoyl]cumarin (Verbindung Nr. 45),
3-[N-(4-Bromphenyl)carbamoyl]-7-phenyl-4-hydroxycumarin (Verbindung Nr. 46),
3-[N-(5-Brompyridin-2-yl)carbamoyl]-4-hydroxycumarin (Verbindung Nr. 47),

3-[N-(2-Pyridinyl)carbamoyl]-4-hydroxycumarin (Verbindung Nr. 48),
3-[N-(5-Chlorpyridin-2-yl)carbamoyl]-4-hydroxycumarin (Verbindung Nr. 49),
3-[N-(2-Chlorpyridin-3-yl)carbamoyl]-4-hydroxycumarin (Verbindung Nr. 50),
3-[N-(5-Methylpyridin-2-yl)carbamoyl]-4-hydroxycumarin (Verbindung Nr. 51),
3-[N-(6-Methoxypyridin-3-yl)carbamoyl]-4-hydroxycumarin (Verbindung Nr. 52),
7-Trifluormethyl-3-[N-[4-(2,4-difluor)phenyl]]-4-hydroxycumarin (Verbindung Nr. 53),
7-Brom-3-[N-(4-chlorphenyl)carbamoyl]-4-hydroxycumarin (Verbindung Nr. 54),
3-[N-(3,5-Dichlorpyridin-2-yl)carbamoyl]-4-hydroxycumarin (Verbindung Nr. 55),
3-[N-(5-Brompyridin-2-yl)carbamoyl]-7-chlor-hydroxycumarin (Verbindung Nr. 56),
7-Brom-3-[N-[4-(2,4-Difluor)phenyl]carbamoyl]-4-hydroxycumarin (Verbindung Nr. 58),
3-[N-[5-(1,1-Dimethylethyl)-1,3,4-thiadiazol-2-yl]carbamoyl]-4-hydroxycumarin (Verbindung Nr. 60),
3-[N-(5-Brompyridin-2-yl)carbamoyl]-7-methyl-4-hydroxycumarin (Verbindung Nr. 61),
7-Brom-3-[N-(4-fluorphenyl)carbamoyl]-4-hydroxycumarin (Verbindung Nr. 62),
3-[N-(2,3,5,6-Tetrafluorpyridin-4-yl)carbamoyl]-6-methoxy-4-hydroxycumarin (Verbindung Nr. 63),
7-Chlor-3-[N-(5-chlorpyridin-2-yl)carbamoyl]-4-hydroxycumarin (Verbindung Nr. 64),
7-Chlor-3-[N-(5-chlorpyridin-2-yl)carbamoyl]-4-hydroxycumarin, Triethylaminsalz (Verbindung Nr. 65),
7-Fluor-3-[N-(5-brompyridin-2-yl)carbamoyl]-4-hydroxycumarin (Verbindung Nr. 67),
7-Chlor-3-[N-(3-chlor-5-trifluormethylpyridin-2-yl)carbamoyl]-4-hydroxycumarin (Verbindung Nr. 68),
3-[N-(3-Chlor-5-trifluormethylpyridin-2-yl)carbamoyl]-4-hydroxycumarin (Verbindung Nr. 69),
3-[N-(3,5-Ditrifluormethylpyridin-2-yl)carbamoyl]-4-hydroxycumarin (Verbindung Nr. 70),
7-Trifluormethyl-3-[N-(5-brompyridin-2-yl)carbamoyl]-4-hydroxycumarin (Verbindung Nr. 71),
7-Trifluormethyl-3-[N-(5-chlorpyridin-2-yl)carbamoyl]-4-hydroxycumarin (Verbindung Nr. 72),
7-Trifluormethyl-3-[N-(2-chlorpyridin-2-yl)carbamoyl]-4-hydroxycumarin (Verbindung Nr. 73),
7-Trifluormethyl-3-[N-(6-chlorpyridin-3-yl)carbamoyl]-4-hydroxycumarin (Verbindung Nr. 74),
7-Chlor-3-[N-(6-methoxypyridin-3-yl)carbamoyl)-4-hydroxycumarin (Verbindung Nr. 75),
7-Trifluormethyl-3-[N-(6-methoxypyridin-3-yl)carbamoyl]-4-hydroxycumarin (Verbindung Nr. 76),
7-Chlor-3-[N-(2-chlorpyridin-3-yl)carbamoyl]-4-hydroxycumarin (Verbindung Nr. 77),
7-Chlor-3-[N-(6-chlorpyridin-3-yl)carbamoyl]-4-hydroxycumarin (Verbindung Nr. 78),
7-Chlor-3-[N-(3,5-ditrifluormethylpyridin-2-yl)carbamoyl]-4-hydroxycumarin (Verbindung Nr. 79),
7-Trifluormethyl-3-[N-(2,3,5,6-tetrafluorpyridin-4-yl)carbamoyl]-4-hydroxycumarin (Verbindung Nr. 80),
7-Chlor-4-hydroxy-3-[N-[(1,1,2,2,3,3,3-heptafluorpropyl)-1,2,4-thiadiazol-2-yl]carbamoyl]cumarin (Verbindung Nr. 81),
7-Brom-3-[N-(4-bromphenyl)carbamoyl]-4-hydroxycumarin (Verbindung Nr. 91),
oder ein pharmazeutisch akzeptables Salz einer der vorhergehenden Verbindungen handelt.

**11.** Verfahren nach Anspruch 1 oder 2, wobei es sich bei dem Produkt um die Verbindungen Nr. 6, 8, 11, 17 oder 18 gemäß der Definition von Anspruch 10 oder ein pharmazeutisch akzeptables Salz derselben handelt.

**12.** Verfahren zur Herstellung einer Arzneimittelzubereitung durch Vereinigen einer Verbindung der Formel I gemäß der Definition nach einem der vorhergehenden Ansprüche oder eines Hydrats oder pharmazeutisch akzeptablen Salzes derselben mit einem inerten Bestandteil.

**13.** Verwendung einer Verbindung der Formel I gemäß der Definition nach einem der Ansprüche 1 bis 11 oder eines Hydrats oder pharmazeutisch akzeptablen Salzes derselben zur Herstellung eines Medikaments zur Abtötung von parasitischen Würmern bei Menschen und wertvollen, warmblütigen Haustieren.

**14.** Verwendung einer Verbindung der Formel IW oder eines Hydrats oder pharmazeutisch akzeptablen Salzes desselben zur Herstellung eines Medikaments zur Verwendung bei der Verhinderung oder Behandlung von Kokzidiose bei Geflügel, wobei die Formel IW folgende ist:

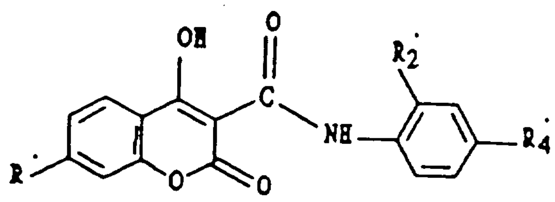

IW

worin R'$_2$ für Wasserstoff, $CF_3$, Cl, Br, F oder I steht, und R'$_4$ Br, F, Cl oder $CR_3$ darstellt und R' Br, F oder $CF_3$ bedeutet, wobei gilt, daß es sich bei der Verbindung nicht um 3-[N-(4-Chlorphenyl)-carbamoyl]-7-trifluormethyl-4-hydroxycumarin handelt.

## Revendications

## Revendications pour les Etats contractants suivants : AT, BE, CH, DE, DK, FR, GB, GR, IT, LU, NL, SE

**1.** Utilisation, pour la préparation d'un médicament destiné à être utilisé pour détruire des vers parasites chez l'homme et chez des animaux domestiques à sang chaud utiles, d'un composé de formule I ou d'un hydrate ou d'un sel pharmaceutiquement acceptable d'un composé de formule I

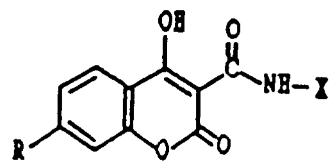

I

dans laquelle X est choisi entre :

(1) un groupe 4-halogénophényle facultativement substitué en position 2 par Cl, Br, F ou I ;

(2) un groupe (5-R'$_5$)-1,3,4-thiadiazole-2-yle dans lequel R'$_5$ représente Cl, Br, F, I, un radical cyano, $-CF_3$, $-CF_2Cl$, $-CF_2CF_3$ ou un radical, facultativement substitué, benzyle, pyridine-2-yle, pyridine-3-yle ou pyridine-4-yle et tous substituants consistent en un, deux ou trois représentants choisis dans le groupe des radicaux Cl, Br, F, I, CN, $CF_3$, alkyle en $C_1$ à $C_5$ et alkoxy en $C_1$ à $C_5$ ;

(3) un groupe (2-SR''$_5$)-1,3,4-thiadiazole-5-yle, dans lequel R''$_5$ représente l'hydrogène, un radical alkyle en $C_1$ à $C_5$, Cl, Br, F, I, cyano, $-CF_3$, $-CF_2Cl$, $-CF_2CF_3$ ou un radical, facultativement substitué, phényle, benzyle, pyridine-2-yle, pyridine-3-yle, pyridine-4-yle, pyridine-2-yl($CH_2$)-, pyridine-3-yl-($CH_2$)- ou pyridine-4-yl($CH_2$)- et tous substituants consistent en un, deux ou trois représentants des radicaux définis ci-dessus ;

(4) un groupe, facultativement substitué, pyridine-2-yle, pyridine-3-yle, pyridine-4-yle, pyrimidine-5-yle, pyrazine-2-yle, pyrimidine-2-yle ou -NH-phényle, et tous substituants consistent en un, deux ou trois représentants des radicaux indiqués ci-dessus ;

(5) un groupe 4-[di(alkoxy en $C_1$ à $C_5$)-phosphonométhyl]phényle ; ou

(6) un groupe 4-(pyridine-4-ylméthyl)phényle facultativement substitué dans le fragment pyridinyle avec un ou deux représentants des radicaux indiqués ci-dessus :

R est de l'hydrogène, un groupe phényle, Cl, Br, F, CN, $CF_3$, alkyle en $C_1$ à $C_5$ ou alkoxy en $C_1$ à $C_5$ ; sous réserve que lorsque X est un groupe 4-halogénophényle, R représente Cl, F, Br, $CF_3$ ou phényle, le composé de formule I représente autre chose que :

la 3-[N-(5-benzyl-1,3,4-thiadiazole-2-yl)carbamoyl]-7-chloro-4-hydroxycoumarine,
la 7-chloro-4-hydroxy-3-[N-5-(2-pyridinylméthyl)thio-1,3,4-thiadiazole-5-yl]carbamoylcoumarine,
la 7-chloro-4-hydroxy-3-[N-(5-phényl-1,3,4-thiadiazole-2-yl]-carbamoylcoumarine,
la 3-[N-(2-benzylthio-1,3,4-thiadiazole-5-yl)carbamoyl]-7-chloro-4-hydroxycoumarine,
la 4-hydroxy-3-[N-(2-pyridinylcarbamoyl)]coumarine,
la 4-hydroxy-3-[N-(3-pyridinylcarbamoyl)]coumarine,
la 7-trifluorométhyl-3-[N-(3-chloro-5-trifluorométhylpyridine-2-yl)carbamoyl]-4-hydroxycoumarine,

la 3-[N-(6-chloropyridine-3-yl)carbamoyl)-4-hydroxycoumarine,
la 3-[N-(6-méthylpyridine-2-yl)carbamoyl]-4-hydroxycoumarine,
la 3-[N-(5-bromopyridine-2-yl)carbamoyl-7méthoxy-4-hydroxycoumarine ou
la 3-[N-(2-6-dichloropyridine-4-yl)carbamoyl-4-hydroxycoumarine.

**2.** Composé de formule IW, hydrate ou sel pharmaceutiquement acceptable d'un composé de formule IW

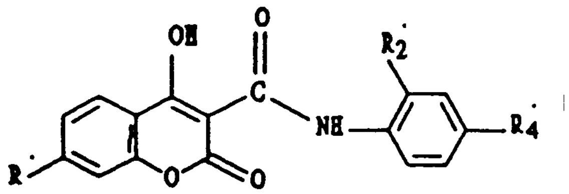

IW

formule dans laquelle $R'_2$ représente l'hydrogène, un groupe $CF_3$, Cl, Br, F ou I ; $R'_4$ représente Br, F, Cl ou $CF_3$ ; et R' représente Br, F ou $CF_3$ ; sous réserve que le composé ne soit pas la 3-[N-(4-chlorophényl)carbamoyl]-7-trifluorométhyl-4-hydroxycoumarine.

**3.** Composé de formule I suivant la revendication 1 ou hydrate ou sel pharmaceutiquement acceptable de ce composé autre que la 3-[N-(4-trifluorométhylphényl)carbamoyl]-4-hydroxycoumarine.

**4.** Composé suivant la revendication 3, dans lequel X est un groupe 4-halogénophényle.

**5.** Composé suivant la revendication 4, qui est :
la 3-[N-(4-bromophényl)carbamoyl]-7-chloro-4-hydroxycoumarine ;
la 3-(N-(4-bromophényl)carbamoyl]-7-trichlorométhyl-4-hydroxycoumarine ;
la 3-[N-(4-chlorophényl)carbamoyl]-7-chloro-4-hydroxycoumarine;
la 3-[N-(4-chlorophényl)carbamoyl]-7-trifluorométhyl-4-hydroxycoumarine ;
la 3-[N-(4-chlorophényl)carbamoyl]-7-fluoro-4-hydroxycoumarine;
la 3-[N-(4-fluorophényl)carbamoyl]-7-chloro-4-hydroxycoumarine;
la 3-[N-(4-fluorophényl)carbamoyl]-7-trifluorométhyl-4-hydroxycoumarine ;
la 3-[N-(4-fluorophényl)carbamoyl]-7-fluoro-4-hydroxycoumarine
ou un sel pharmaceutiquement acceptable de l'un quelconque des composés ci-dessus.

**6.** Composé suivant la revendication 3, dans lequel X est un groupe (5-$R'_5$)-1,3,4-thiadiazole-2-yle.

**7.** Composé suivant la revendication 6, dans lequel $R'_5$ est un groupe $CF_3$, Cl, Br, F, I, un groupe CN, alkyle en $C_1$ à $C_5$ ou un groupe benzyle ou pyridinyle facultativement substitué avec un représentant du groupe Cl, Br, F, I, -CN et $CF_3$.

**8.** Composé suivant la revendication 6, dans lequel R représente Cl, Br ou $CF_3$.

**9.** Composé suivant la revendication 6, qui est
la 3-[N-(4-bromophényl)carbamoyl]-7-chloro-4-hydroxycoumarine (composé n° 1),
la 3-[N-(4-bromophényl)carbamoyl]-7-trifluorométhyl-4-hydroxycoumarine (composé n° 2),
la 3-[N-(4-bromophényl)carbamoyl]-7-fluoro-4-hydroxycoumarine (composé n° 3),
la 7-chloro-4-hydroxy-3-[N-[5-(trifluorométhyl)-1,3,4-thiadiazole-2-yl]carbamoyl]coumarine (composé n° 11),
la 7-chloro-4-hydroxy-3-[N-[5-(chlorodifluorométhyl)-1,3,4- thiadiazole-2-yl]carbamoyl]coumarine (composé n° 17),
la 7-chloro-4-hydroxy-3-[N-[5-(trifluorométhyldifluoro-méthyl)-1,3,4-thiadiazole-2-yl]carbamoyl]-coumarine (composé n° 18),
ou un sel pharmaceutiquement acceptable de l'un quelconque des composés ci-dessus.

**10.** Composé suivant la revendication 3, qui est :
la 3-[N-(4-bromophényl)carbamoyl]-7-chloro-4-hydroxycoumarine (composé n° 1),

la 3-[N-(4-bromophényl)carbamoyl]-7-trifluorométhyl-4-hydroxycoumarine (composé n° 2),
la 3-[N-(4-bromophényl)carbamoyl]-7-fluoro-4-hydroxycoumarine (composé n° 3),
la 3-[N-(4-chlorophényl)carbamoyl]-7-chloro-4-hydroxycoumarine(composé n° 4),
la 3-[N-(4-chlorophényl)carbamoyl]-7-trifluorométhyl-4-hydroxycoumarine (composé n° 5),
la 3-[N-(4-chlorophényl)carbamoyl]-7-fluoro-4-hydroxycoumarine(composé n° 6),
la 3-[N-(4-fluorophényl)carbamoyl]-7-chloro-4-hydroxycoumarine(composé n° 7),
la 3-[N-(4-fluorophényl)carbamoyl]-7-trifluorométhyl-4-hydroxycoumarine (composé n° 8),
la 3-[N-(4-fluorophényl)carbamoyl]-7-fluoro-4-hydroxycoumarine(composé n° 9),
la 7-chloro-4-hydroxy-3-[N-[5-(trifluorométhyl)-1,3,4-thiadiazole-2yl]-carbamoyl]coumarine (composé n° 11),
la 4-hydroxy-3-[N-[5-(trifluorométhyl)-1,3,4-thiadiazole-2-yl] carbamoyl]coumarine (composé n° 12),
la 4-hydroxy-7-méthyl-3-[N-[5-(trifluorométhyl)-1,3,4-thiadiazole-2-yl]carbamoyl]coumarine (composé n° 13),
la 4-hydroxy-7-méthoxy-3-[N-[5-(trifluorométhyl)-1,3,4-thiadiazole-2 yl]carbamoyl]coumarine (composé n° 14),
la 7-fluoro-4-hydroxy-3-[N-[5-(trifluorométhyl)-1,3,4-thiadiazole-2 yl]carbamoyl]coumarine (composé n° 15),
la 7-trifluorométhyl-4-hydroxy-3-[N-[5-(trifluorométhyl)-1,3,4-thiadiazole-2-yl]carbamoyl]coumarine (composé n° 16),
la 7-chloro-4-hydroxy-3-[N-[5-(chlorodifluorométhyl)-1,3,4-thiadiazole-2-yl]carbamoyl]coumarine (composé n° 17),
la 7-chloro-4-hydroxy-3-[N-[5-(trifluorométhyldifluorométhyl)- 1,3,4-thiadiazole-2-yl]carbamoyl]-coumarine (composé n° 18),
la 4-hydroxy-3-N-[5-(trifluorométhyldifluorométhyl)-1,3,4-thiadiazole-2-yl]carbamoyl]coumarine (composé n° 19),
la 7-fluoro-4-hydroxy-3-[N-[5-(trifluorométhyldifluorométhyl)-1,3,4-thiadiazol-2-yl]carbamoyl]-coumarine (composé n° 20),
la 4-hydroxy-3-[N-(5-phényl-1,3,4-thiadiazole-2-yl)carbamoyl]coumarine (composé n° 21),
la 3-[N-(5-benzyl-1,3,4-thiadiazole-2-yl)carbamoyl]-4-hydroxycoumarine (composé n° 23),
la 3-[N-(5-benzyl-1,3,4-thiadiazole-2-yl)carbamoyl]-4-hydroxy-7-méthylcoumarine (composé n° 24),
la 3-[N-[5-(4-chlorophényl)méthyl-1,3,4-thiadiazole-2-yl]carbamoyl]4-hydroxycoumarine (composé n° 25),
la 7-chloro-3-[N-[5-(4-chlorophényl)méthyl-1,3,4-thiadiazole-2-yl]carbamoyl]-4-hydroxycoumarine (composé n° 26),
la 3-[N-[2-(4-fluorophényl)méthyl]thio-1,3,4-thiadiazole-5-yl]carbamoyl]4-hydroxycoumarine (composé n° 27),
la 4-hydroxy-3-[N-(2-mercapto- 1,3,4-thiadiazole-5-yl)carbamoyl]coumarine (composé n° 28),
la 4-hydroxy-3-[N-[2-(méthylthio)-1,3,4-thiadiazole-5-yl]carbamoyl]coumarine (composé n° 29),
la 4-hydroxy-3-[N-[5-(3,5-dichloropyridine-2-yl)thio-1,3,4-thiadiazole-2-yl]carbamoyl]coumarine (composé n° 30),
la 4-hydroxy-3-[N-[2-(2-pyridinylméthyl)thio-1,3,4-thiadiazole-5-yl]carbamoyl]coumarine (composé n° 31),
la 4-hydroxy-7-méthyl-3-[N-[2-(2-pyridinylméthyl)thio-1,3,4-thiadiazole-5-yl]carbamoyl]coumarine (composé n° 32),
la 3-[N-[2-(4-pyridinylméthyl)thio-1,3,4-thiadiazole-5-yl]carbamoyl]-4-hydroxycoumarine (composé n° 33),
la 3-[N-[2-(benzylthio)-1,3,4-thiadiazole-5-yl]carbamoyl]-4-hydroxycoumarine (composé n° 34),
la 3-[N-(5-bromopyrimidine-2-yl)carbamoyl-4-hydroxycoumarine (composé n° 35),
la 4-hydroxy-3-[N-(phénylamino)carbamoyl]coumarine (composé n° 36),
la 4-hydroxy-3-[N-[(4-bromophényl)amino]carbamoyl]coumarine (composé n° 37),
la 4-hydroxy-3-[N-(2-bromo-1,3,4-thiadiazole-5-yl)carbamoyl]coumarine (composé n° 38),
la 4-hydroxy-3-[N-(2-chloro-1,3,4-thiadiazole-5-yl)carbamoyl]coumarine (composé n° 39),
la 3-[N-(4-chloro-6-méthylpyrimidine-2-yl)carbamoyl]-4-hydroxycoumarine (composé n° 40),
la 3-[N-(6-chloropyrazine-2-yl)carbamoyl]-4-hydroxycoumarine (composé n° 41),
la 3-[N-(4,6-dichloropyrimidine-5-yl)carbamoyl]-4-hydroxycoumarine (composé n° 42),
la 4-hydroxy-3-[N-[5-(2-fluorophényl)-1,3,4-thiadiazole-2-yl]carbamoyl]coumarine (composé n° 44),
la 7-phényl-4-hydroxy-3-[N-[5-(trifluorométhyl)-1,3,4-thiadiazole-2-yl]carbamoyl]coumarine (composé n° 45),

la 3-[N-(4-bromophényl)carbamoyl]-7-phényl-4-hydroxycoumarine(composé n° 46),
la 3-[N-5-bromopyridine-2-yl)carbamoyl]4-hydroxycoumarine (composé n° 47),
la 3-[N-(2-pyridinyl)carbamoyl]-4-hydroxycoumarine (composé n° 48),
la 3-[N-(5-chloropyridine-2-yl)carbamoyl]4-hydroxycoumarine (composé n° 49),
la 3-[N-(2-chloropyridine-3-yl)carbamoyl]4-hydroxycoumarine (composé n° 50),
la 3-[N-(5-méthylpyridine-2-yl)carbamoyl]4-hydroxycoumarine (composé n° 51),
la 3-[N-(6-méthoxypyridine-3-yl)carbamoyl4-hydroxycoumarine (composé n° 52),
la 7-trifluorométhyl-3-[N-[4-(2,4-difluoro)phényl]]4-hydroxycoumarine (composé n° 53),
la 7-bromo-3-[N-[4-chlorophényl)carbamoyl]4-hydroxycoumarine (composé n° 54),
la 3-[N-(3,5-dichloropyridine-2-yl)carbamoyl]4-hydroxycoumarine(composé n° 55),
la 3-[N-(5-bromopyridine-2-yl)carbamoyl]-7chloro-4-hydroxycoumarine (composé n° 56),
la 7-bromo-3-[N-[4-(2,4-difluoro)phényl]carbamoyl]4-hydroxycoumarine (composé n° 58),
la 3-[N-[5-(1,1-diméthyléthyl)-1,3,4-thiadiazole-2-yl)carbamoyl]-4-hydroxycoumarine (composé n° 60),
la 3-[N-(5-bromopyridine-2-yl)carbamoyl]-7méthyl-4-hydroxycoumarine (composé n° 61),
la 7-bromo-3-[N-(4-fluorophényl)carbamoyl]4-hydroxycoumarine (composé n° 62),
la 3-[N-(2,3,5,6-tétrafluoropyridine-4-yl)carbamoyl]-6-méthoxy-4-hydroxycoumarine (composé n° 63),
la 7-chloro-3-[N-(5-chloropyridine-2-yl)carbamoyl]-4-hydroxycoumarine (composé n° 64),
le sel de triéthylamine de la 7-chloro-3-[N-(5-chloropyridine-2-yl)carbamoyl]4-hydroxycoumarine (composé n° 65),
la 7-fluoro-3-[N-(5-bromopyridine-2-yl)carbamoyl]4-hydroxycoumarine (composé n° 67),
la 7-chloro-3-[N-(3-chloro-5-trifluorométhylpyridine-2-yl)carbamoyl]-4-hydroxycoumarine (composé n° 68),
la 3-[N-(3-chloro-5-trifluorométhylpyridine-2-yl)carbamoyl]-4-hydroxycoumarine (composé n° 69),
la 3-[N-(3,5-ditrifluorométhylpyridine-2-yl)carbamoyl]-4-hydroxycoumarine (composé n° 70),
la 7-trifluorométhyl-3-[N-(5-bromopyridine-2-yl]carbamoyl]-4-hydroxycoumarine (composé n° 71),
la 7-trifluorométhyl-3-[N-(5-chloropyridine-2-yl]carbamoyl]-4-hydroxycoumarine (composé n° 72),
la 7-trifluorométhyl-3-[N-(2-chloropyridine-3-yl]carbamoyl]-4-hydroxycoumarine (composé n° 73),
la 7-trifluorométhyl-3-[N-(6-chloropyridine-3-yl]carbamoyl-4-hydroxycoumarine (composé n° 74),
la 7-chloro-3-[N-(6-méthoxypyridine-3-yl]carbamoyl]-4-hydroxycoumarine (composé n° 75),
la 7-trifluorométhyl-3-[N-(6-méthoxypyridine-3-yl]carbamoyl]-4- hydroxycoumarine (composé n° 76),
la 7-chloro-3-[N-(2-chloropyridine-3-yl]carbamoyl]-4-hydroxycoumarine (composé n° 77),
la 7-chloro-3- [N-(6-chloropyridine-3-yl]carbamoyl]-4-hydroxycoumarine (composé n° 78),
la 7-chloro-3-[N-(3,5-ditrifluorométhylpyridine-2-yl]carbamoyl-4-hydroxycoumarine (composé n° 79),
la 7-trifluorométhyl-3-[N-(2,3,5,6-tétrafluoropyridine-4-yl]-carbamoyl]-4-hydroxycoumarine (composé n° 80),
la 7-chloro-4-hydroxy-3-[N-1,1,2,2,3,3,3-heptafluoropropyl)-1,2,4-thiadiazole-2-yl]carbamoyl]-coumarine (composé n° 81),
la 7-bromo-3-[N-[4-bromophényl)carbamoyl]-4-hydroxycoumarine (composé n° 91),
ou un sel pharmaceutiquement acceptable de l'un quelconque des composés ci-dessus.

**11.** Composé suivant la revendication 3, qui est le composé n° 6, 8, 11, 17 ou 18 tel que défini dans la revendication 10 ou un sel pharmaceutiquement acceptable de ce compose.

**12.** Composé suivant la revendication 3, qui est la 7-bromo-3-[N-(4-chlorophényl)carbamoyl]-4-hydroxycoumarine ou la 7-bromo-3-[N-(2,4-difluorophényl)carbamoyl]-4-hydroxycoumarineou un hydrate ou un sel pharmaceutiquement acceptable de ce composé.

**13.** Utilisation suivant la revendication 1, dans laquelle le composé est tel que défini dans l'une quelconque des revendications 3 à 12.

**14.** Utilisation d'un composé suivant la revendication 2, pour la préparation d'un médicament destiné à être utilisé dans la lutte préventive ou curative contre la coccidiose chez les animaux de basse-cour.

**Revendications pour l'Etat contractant suivant : ES**

1. Procédé de production d'un composé de formule I ou d'un hydrate ou d'un sel pharmaceutiquement acceptable d'un composé de formule I

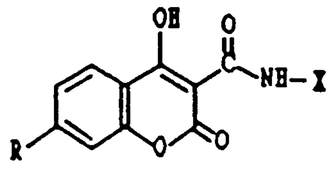

I

dans laquelle X est choisi entre :

(1) un groupe 4-halogénophényle facultativement substitué en position 2 par Cl, Br, F ou I ;

(2) un groupe (5-R'$_5$)-1,3,4-thiadiazole-2-yle dans lequel R'$_5$ représente Cl, Br, F, I, un radical cyano, $-CF_3$, $-CF_2Cl$, $-CF_2CF_3$ ou un radical, facultativement substitué, benzyle, pyridine-2-yle, pyridine-3-yle ou pyridine-4-yle et tous substituants consistent en un, deux ou trois représentants choisis dans le groupe des radicaux Cl, Br, F, I, CN, $CF_3$, alkyle en $C_1$ à $C_5$ et alkoxy en $C_1$ à $C_5$ ;

(3) un groupe (2-SR''$_5$)-1,3,4-thiadiazole-5-yle, dans lequel R''$_5$ représente l'hydrogène, un radical alkyle en $C_1$ à $C_5$, Cl, Br, F, I, cyano, $-CF_3$, $-CF_2Cl$, $-CF_2CF_3$ ou un radical, facultativement substitué, phényle, benzyle, pyridine-2-yle, pyridine-3-yle, pyridine-4-yle, pyridine-2-yl($CH_2$)-, pyridine-3-yl-($CH_2$)- ou pyridine-4-yl($CH_2$)- et tous substituants consistent en un, deux ou trois représentants des radicaux définis ci-dessus ;

(4) un groupe, facultativement substitué, pyridine-2-yle, pyridine-3-yle, pyridine-4-yle, pyrimidine-5-yle, pyrazine-2-yle, pyrimidine-2-yle ou -NH-phényle, et tous substituants consistent en un, deux ou trois représentants des radicaux indiqués ci-dessus ;

(5) un groupe 4-[di(alkoxy en $C_1$ à $C_5$)-phosphonométhyl]phényle ; ou

(6) un groupe 4-(pyridine-4-ylméthyl)phényle facultativement substitué dans le fragment pyridinyle avec un ou deux représentants des radicaux indiqués ci-dessus ;

R est de l'hydrogène, un groupe phényle, Cl, Br, F, CN, $CF_3$, alkyle en $C_1$ à $C_5$ ou alkoxy en $C_1$ à $C_5$ ; sous réserve que lorsque X est un groupe 4-halogénophényle, R représente Cl, F, Br, $CF_3$ ou phényle, le composé de formule I représente autre chose que :

la 3-[N-(5-benzyl-1,3,4-thiadiazole-2-yl)carbamoyl]-7-chloro-4-hydroxycoumarine,
la 7-chloro-4-hydroxy-3-[N-5-(2-pyridinylméthyl)thio-1,3,4-thiadiazole-5-yl]carbamoylcoumarine,
la 7-chloro-4-hydroxy-3-[N-(5-phényl-1,3,4-thiadiazole-2-yl]-carbamoylcoumarine,
la 3-[N-(2-benzylthio-1,3,4-thiadiazole-5-yl)carbamoyl]-7-chloro-4-hydroxycoumarine,
la 4-hydroxy-3-[N-(2-pyridinylcarbamoyl)]coumarine,
la 4-hydroxy-3-[N-(3-pyridinylcarbamoyl)]coumarine,
la 7-trifluorométhyl-3-[N-(3-chloro-5-trifluorométhylpyridine-2-yl)carbamoyl]-4-hydroxycoumarine,
la 3-[N-(6-chloropyridine-3-yl)carbamoyl)4-hydroxycoumarine,
la 3-[N-(6-méthylpyridine-2-yl)carbamoyl]-4-hydroxycoumarine,
la 3-[N-(5-bromopyridine-2-yl)carbamoyl-7méthoxy-4-hydroxycoumarine ou
la 3-[N-(2-6-dichloropyridine-4-yl)carbamoyl-4-hydroxycoumarine.

la 3-[N-(4-trifluorométhylphényl)carbamoyl]-4-hydroxycoumarine qui comprend le reflux d'un composé correspondant de formule

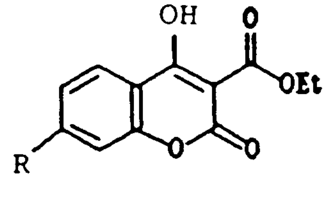

avec une arylamine correspondante de formule $XNH_2$.

2. Procédé de production d'un composé de formule I suivant la revendication 1 ou d'un hydrate ou d'un sel pharmaceutiquement acceptable de ce composé, qui comprend la réaction d'un composé correspondant de formule

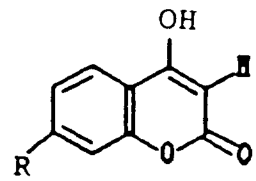

avec un arylisocyanate correspondant de formule XNCO en présence d'une base.

3. Procédé suivant la revendication 1 ou la revendication 2, dans lequel le produit est la 7-bromo-3-[N-(4-chlorophényl)carbamoyl]-4-hydroxycoumarine ou la 7-bromo-3-[N-(2,4-difluorophényl)carbamoyl]-4-hydroxycoumarineou un hydrate ou un sel pharmaceutiquement acceptable de ce composé.

4. Procédé suivant la revendication 1 ou 2, dans lequel X est un groupe 4-halogénophényle.

5. Procédé suivant la revendication 4, dans lequel le produit est
la 3-[N-(4-bromophényl)carbamoyl]-7-chloro-4-hydroxycoumarine ;
la 3-(N-(4-bromophényl)carbamoyl]-7-trichlorométhyl-4-hydroxycoumarine ;
la 3-[N-(4-chlorophényl)carbamoyl]-7-chloro-4-hydroxycoumarine;
la 3-[N-(4-chlorophényl)carbamoyl]-7-trifluorométhyl-4-hydroxy coumarine ;
la 3-[N-(4-chlorophényl)carbamoyl]-7-fluoro-4-hydroxycoumarine;
la 3-[N-(4-fluorophényl)carbamoyl]-7-chloro-4-hydroxycoumarine;
la 3-[N-(4-fluorophényl)carbamoyl]-7-trifluorométhyl-4-hydroxycoumarine ;
la 3-[N-(4-fluorophényl)carbamoyl]-7-fluoro-4-hydroxycoumarine
ou un sel pharmaceutiquement acceptable de l'un quelconque des composés ci-dessus.

6. Procédé suivant la revendication 1 ou la revendication 2, dans lequel X est un groupe (5-$R'_5$)-1,3,4-thiadiazole-2-yle.

7. Procédé suivant la revendication 6, dans lequel $R'_5$ représente $CF_3$, Cl, Br, F, I, CN, un groupe alkyle en $C_1$ à $C_5$ ou un groupe benzyle ou pyridinyle facultativement substitué avec un représentant du groupe Cl, Br, F, I, -CN et $CF_3$.

8. Procédé suivant la revendication 6, dans lequel R représente Cl, Br ou $CF_3$.

9. Procédé suivant la revendication 6, dans lequel le produit est :
la 3-[N-(4-bromophényl)carbamoyl]-7-chloro-4-hydroxycoumarine (composé n° 1),
la 3-[N-(4-bromophényl)carbamoyl]-7-trifluorométhyl-4-hydroxycoumarine (composé n° 2),
la 3-[N-(4-bromophényl)carbamoyl]-7-fluoro-4-hydroxycoumarine (composé n° 3),
la 7-chloro-4-hydroxy-3-[N-[5-(trifluorométhyl)-1,3,4-thiadiazole-2-yl]carbamoyl]coumarine (composé n° 11),
la 7-chloro-4-hydroxy-3-[N-[5-(chlorodifluorométhyl)-1,3,4-thiadiazole-2-yl]carbamoyl]coumarine (composé n° 17),
la 7-chloro-4-hydroxy-3-[N-[5-(trifluorométhyldifluoro-méthyl)-1,3,4-thiadiazole-2-yl]carbamoyl]-coumarine (composé n° 18),
ou un sel pharmaceutiquement acceptable de l'un quelconque des composés ci-dessus.

10. Procédé suivant la revendication 1 ou la revendication 2, dans lequel le produit est :
la 3-[N-(4-bromophényl)carbamoyl]-7-chloro-4-hydroxycoumarine (composé n° 1),
la 3-[N-(4-bromophényl)carbamoyl]-7-trifluorométhyl-4-hydroxycoumarine (composé n° 2),
la 3-[N-(4-bromophényl)carbamoyl]-7-fluoro-4-hydroxycoumarine (composé n° 3),
la 3-[N-(4-chlorophényl)carbamoyl]-7-chloro-4-hydroxycoumarine(composé n° 4),
la 3-[N-(4-chlorophényl)carbamoyl]-7-trifluorométhyl-4-hydroxycoumarine (composé n° 5),

la 3-[N-(4-chlorophényl)carbamoyl]-7-fluoro-4-hydroxycoumarine(composé n° 6),

la 3-[N-(4-fluorophényl)carbamoyl]-7-chloro-4-hydroxycoumarine(composé n° 7),

la 3-[N-(4-fluorophényl)carbamoyl]-7-trifluorométhyl-4-hydroxycoumarine (composé n° 8),

la 3-[N-(4-fluorophényl)carbamoyl]-7-fluoro-4-hydroxycoumarine(composé n° 9),

la 7-chloro-4-hydroxy-3-[N-[5-(trifluorométhyl)-1,3,4-thiadiazole-2 yl]-carbamoyl]coumarine (composé n° 11),

la 4-hydroxy-3-[N-[5-(trifluorométhyl)-1,3,4-thiadiazole-2-yl] carbamoyl]coumarine (composé n° 12),

la 4-hydroxy-7-méthyl-3-[N-[5-(trifluorométhyl)-1,3,4-thiadiazole-2-yl]carbamoyl]coumarine (composé n° 13),

la 4-hydroxy-7-méthoxy-3-[N-[5-(trifluorométhyl)-1,3,4-thiadiazole-2 yl]carbamoyl]coumarine (composé n° 14),

la 7-fluoro-4-hydroxy-3-[N-[5-(trifluorométhyl)-1,3,4-thiadiazole-2 yl]carbamoyl]coumarine (composé n° 15),

la 7-trifluorométhyl-4-hydroxy-3-[N-[5-(trifluorométhyl)-1,3,4-thiadiazole-2-yl]carbamoyl]coumarine (composé n° 16),

la 7-chloro-4-hydroxy-3-[N-[5-(chlorodifluorométhyl)-1,3,4-thiadiazole-2-yl]carbamoyl]coumarine (composé n° 17),

la 7-chloro-4-hydroxy-3-[N-[5-(trifluorométhyldifluorométhyl)-1,3,4-thiadiazole-2-yl]carbamoyl]-coumarine (composé n° 18),

la 4-hydroxy-3-N-[5-(trifluorométhyldifluorométhyl)-1,3,4-thiadiazole-2-yl]carbamoyl]coumarine (composé n° 19),

la 7-fluoro-4-hydroxy-3-[N-[5-(trifluorométhyldifluorométhyl)-1,3,4-thiadiazol-2-yl]carbamoyl]-coumarine (composé n° 20),

la 4-hydroxy-3-[N-(5-phényl-1,3,4-thiadiazole-2-yl)carbamoyl]coumarine (composé n° 21),

la 3-[N-(5-benzyl-1,3,4-thiadiazole-2-yl)carbamoyl]4-hydroxycoumarine (composé n° 23),

la 3-[N-(5-benzyl-1,3,4-thiadiazole-2-yl)carbamoyl]4-hydroxy-7-méthylcoumarine (composé n° 24),

la 3-[N-[5-(4-chlorophényl)méthyl-1,3,4-thiadiazole-2-yl]carbamoyl]4-hydroxycoumarine (composé n° 25),

la 7-chloro-3-[N-[5-(4-chlorophényl)méthyl-1,3,4-thiadiazole-2-yl]carbamoyl]-4-hydroxycoumarine (composé n° 26),

la 3-[N-[2-(4-fluorophényl)méthyl]thio-1,3,4-thiadiazole-5-yl]carbamoyl]4-hydroxycoumarine (composé n° 27),

la 4-hydroxy-3-[N-(2-mercapto-1,3,4-thiadiazole-5-yl)carbamoyl]coumarine (composé n° 28),

la 4-hydroxy-3-[N-[2-(méthylthio)-1,3,4-thiadiazole-5-yl]carbamoyl]coumarine (composé n° 29),

la 4-hydroxy-3-[N-[5-(3,5-dichloropyridine-2-yl)thio-1,3,4-thiadiazole-2-yl]carbamoyl]coumarine (composé n° 30),

la 4-hydroxy-3-[N-[2-(2-pyridinylméthyl)thio-1,3,4-thiadiazole-5-yl]carbamoyl]coumarine (composé n° 31),

la 4-hydroxy-7-méthyl-3-[N-[2-(2-pyridinylméthyl)thio-1,3,4-thiadiazole-5-yl]carbamoyl]coumarine (composé n° 32),

la 3-[N-[2-(4-pyridinylméthyl)thio-1,3,4-thiadiazole-5-yl]carbamoyl]4-hydroxycoumarine (composé n° 33),

la 3-[N-[2-(benzylthio)-1,3,4-thiadiazole-5-yl]carbamoyl]-4-hydroxycoumarine (composé n° 34),

la 3-[N-(5-bromopyrimidine-2-yl)carbamoyl-4-hydroxycoumarine (composé n° 35),

la 4-hydroxy-3-[N-(phénylamino)carbamoyl]coumarine (composé n° 36),

la 4-hydroxy-3-[N-[(4-bromophényl)amino]carbamoyl]coumarine (composé n° 37),

la 4-hydroxy-3-[N-(2-bromo-1,3,4-thiadiazole-5-yl)carbamoyl]coumarine (composé n° 38),

la 4-hydroxy-3-[N-(2-chloro-1,3,4-thiadiazole-5-yl)carbamoyl]coumarine (composé n° 39),

la 3-[N-(4-chloro-6-méthylpyrimidine-2-yl)carbamoyl]-4-hydroxycoumarine (composé n° 40),

la 3-[N-(6-chloropyrazine-2-yl)carbamoyl]-4-hydroxycoumarine (composé n° 41),

la 3-[N-(4,6-dichloropyrimidine-5-yl)carbamoyl]-4-hydroxycoumarine (composé n° 42),

la 4-hydroxy-3-[N-[5-(2-fluorophényl)-1,3,4-thiadiazole-2-yl]carbamoyl]coumarine (composé n° 44),

la 7-phényl-4-hydroxy-3-[N-[5-(trifluorométhyl)-1,3,4-thiadiazole-2-yl]carbamoyl]coumarine (composé n° 45),

la 3-[N-(4-bromophényl)carbamoyl]-7-phényl-4-hydroxycoumarine(composé n° 46),

la 3-[N-5-bromopyridine-2-yl)carbamoyl]4-hydroxycoumarine (composé n° 47),

la 3-[N-(2-pyridinyl)carbamoyl]-4-hydroxycoumarine (composé n° 48),

la 3-[N-(5-chloropyridine-2-yl)carbamoyl]4-hydroxycoumarine (composé n° 49),

la 3-[N-(2-chloropyridine-3-yl)carbamoyl]4-hydroxycoumarine (composé n° 50),
la 3-[N-(5-méthylpyridine-2-yl)carbamoyl]4-hydroxycoumarine (composé n° 51),
la 3-[N-(6-méthoxypyridine-3-yl)carbamoyl]4-hydroxycoumarine (composé n° 52),
la 7-trifluorométhyl-3-[N-[4-(2,4-difluoro)phényl]]4-hydroxycoumarine (composé n° 53),
la 7-bromo-3-[N-[4-chlorophényl)carbamoyl]4-hydroxycoumarine (composé n° 54),
la 3-[N-(3,5-dichloropyridine-2-yl)carbamoyl]4-hydroxycoumarine(composé n° 55),
la 3-[N-(5-bromopyridine-2-yl)carbamoyl]-7-chloro-4-hydroxycoumarine (composé n° 56),
la 7-bromo-3-[N-[4-(2,4-difluoro)phényl]carbamoyl]4-hydroxycoumarine (composé n° 58),
la 3-[N-[5-(1,1-diméthyléthyl)-1,3,4-thiadiazole-2-yl)carbamoyl]4-hydroxycoumarine (composé n° 60),
la 3-[N-(5-bromopyridine-2-yl)carbamoyl]-7-méthyl-4-hydroxycoumarine (composé n° 61),
la 7-bromo-3-[N-(4-fluorophényl)carbamoyl]4-hydroxycoumarine (composé n° 62),
la 3-[N-(2,3,5,6-tétrafluoropyridine-4-yl)carbamoyl]-6-méthoxy-4-hydroxycoumarine (composé n° 63),
la 7-chloro-3-[N-(5-chloropyridine-2-yl)carbamoyl]-4-hydroxycoumarine (composé n° 64),
le sel de triéthylamine de la 7-chloro-3-[N-(5-chloropyridine-2-yl)carbamoyl]4-hydroxycoumarine (composé n° 65),
la 7-fluoro-3-[N-(5-bromopyridine-2-yl)carbamoyl]4-hydroxycoumarine (composé n° 67),
la 7-chloro-3-[N-(3-chloro-5-trifluorométhylpyridine-2-yl)carbamoyl]-4-hydroxycoumarine (composé n° 68),
la 3-[N-(3-chloro-5-trifluorométhylpyridine-2-yl)carbamoyl]-4-hydroxycoumarine (composé n° 69),
la 3-[N-(3,5-ditrifluorométhylpyridine-2-yl)carbamoyl]-4-hydroxycoumarine (composé n° 70),
la 7-trifluorométhyl-3-[N-(5-bromopyridine-2-yl]carbamoyl]-4-hydroxycoumarine (composé n° 71),
la 7-trifluorométhyl-3-[N-(5-chloropyridine-2-yl]carbamoyl]-4-hydroxycoumarine (composé n° 72),
la 7-trifluorométhyl-3-[N-(2-chloropyridine-3-yl]carbamoyl]-4-hydroxycoumarine (composé n° 73),
la 7-trifluorométhyl-3-[N-(6-chloropyridine-3-yl]carbamoyl-4-hydroxycoumarine (composé n° 74),
la 7-chloro-3-[N-(6-méthoxypyridine-3-yl]carbamoyl]-4-hydroxycoumarine (composé n° 75),
la 7-trifluorométhyl-3-[N-(6-méthoxypyridine-3-yl]carbamoyl]-4- hydroxycoumarine (composé n° 76),
la 7-chloro-3-[N-(2-chloropyridine-3-yl]carbamoyl]-4-hydroxycoumarine (composé n° 77),
la 7-chloro-3- [N-(6-chloropyridine-3-yl]carbamoyl]-4-hydroxycoumarine (composé n° 78),
la 7-chloro-3-[N-(3,5-ditrifluorométhylpyridine-2-yl]carbamoyl-4-hydroxycoumarine (composé n° 79),
la 7-trifluorométhyl-3-[N-(2,3,5,6-tétrafluoropyridine-4-yl]-carbamoyl]-4-hydroxycoumarine (composé n° 80),
la 7-chloro-4-hydroxy-3-[N- (1,1,2,2,3,3,3-heptafluoropropyl)-1,2,4-thiadiazole-2-yl]carbamoyl]-coumarine (composé n° 81),
la 7-bromo-3-[N-[4-bromophényl)carbamoyl]-4-hydroxycoumarine (composé n° 91),
ou un sel pharmaceutiquement acceptable de l'un quelconque des composés ci-dessus.

**11.** Procédé suivant la revendication 1 ou la revendication 2, dans lequel le produit est le composé n° 6, 8, 11, 17 ou 18 tel que défini dans la revendication 10 ou un sel pharmaceutiquement acceptable de ce composé.

**12.** Procédé de préparation d'une composition pharmaceutique, qui comprend la formulation d'un composé de formule I tel que défini dans l'une quelconque des revendications précédentes ou d'un hydrate ou d'un sel pharmaceutiquement acceptable de ce composé avec un ingrédient inerte.

**13.** Utilisation d'un composé de formule I tel que défini dans l'une quelconque des revendications 1 à 11 ou d'un hydrate ou d'un sel pharmaceutiquement acceptable de ce composé pour la préparation d'un médicament destiné à être utilisé pour détruire des vers parasites chez l'homme et chez des animaux à sang chaud domestiques utiles.

**14.** Utilisation, pour la préparation d'un médicament destiné à être utilisé pour la lutte préventive ou curative contre la coccidiose chez les oiseaux de basse-cour, d'un composé de formule IW ou d'un hydrate ou d'un sel pharmaceutiquement acceptable d'un composé de formule IW

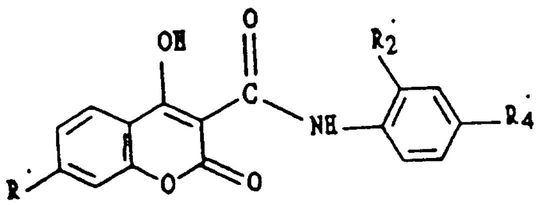

IW

dans laquelle $R'_2$ représente l'hydrogène, $CF_3$, Cl, Br, F ou I, $R'_4$ représente Br, F, Cl ou $CF_3$ ; et R' représente Br, F ou $CF_3$, sous réserve que le composé ne soit pas la 3-[N-(4-chlorophényl)carbamoyl]-7-trifluorométhyl-4-hydroxycoumarine.